(19) Europäisches Patentamt
European Patent Office
Office européen des brevets

(11) Veröffentlichungsnummer: **0 630 895 A1**

# (12) EUROPÄISCHE PATENTANMELDUNG

(21) Anmeldenummer: **94109019.3**

(22) Anmeldetag: **13.06.94**

(51) Int. Cl.[5]: **C07D 409/04**, C07D 417/04, C07D 491/04, C07D 401/04, C07D 405/04, A61K 31/445, A61K 31/47, C07D 495/04, C07D 521/00, C07D 211/90, C07D 471/04, C07D 215/14, C07D 209/10, C07D 209/44, C07D 307/79, C07D 333/54, C07D 311/58, C07D 311/18, C07D 335/06, C07D 311/32, C07D 311/76, C07D 241/42

(30) Priorität: **24.06.93 DE 4321030**

(43) Veröffentlichungstag der Anmeldung:
**28.12.94 Patentblatt 94/52**

(84) Benannte Vertragsstaaten:
**AT BE CH DE DK ES FR GB GR IE IT LI LU MC NL PT SE**

(71) Anmelder: **BAYER AG**

**D-51368 Leverkusen (DE)**

(72) Erfinder: **Straub, Alexander, Dr.**
**Moospfad 30**
**D-42113 Wuppertal (DE)**
Erfinder: **Goldmann, Siegfried, Dr.**
**Am Osterholz 91**
**D-42327 Wuppertal (DE)**
Erfinder: **Stoltefuss, Jürgen, Dipl.-Ing.**
**Parkstrasse 20**
**D-42781 Haan (DE)**
Erfinder: **Bechem, Martin, Dr.s**
**Hans-Böckler-Strasse 102**
**D-42111 Wuppertal (DE)**
Erfinder: **Dembrowsky, Klaus, Dr.**
**Bismarckstrasse 85**
**D-42115 Wuppertal (DE)**
Erfinder: **Gross, Rainer, Prof. Dr.**
**Platzhoffstrasse 23**
**D-42115 Wuppertal (DE)**
Erfinder: **Hebisch, Siegbert, Dr.**
**Johann-Breuker-Platz 8**
**D-46244 Bottrop (DE)**
Erfinder: **Hütter, Joachim, Dr.**
**Teschensudberger Strasse 13**
**D-42349 Wuppertal (DE)**
Erfinder: **Rounding, Howard-Paul, Dr.**
**Pahlkestrasse 15**
**D-42115 Wuppertal (DE)**

## (54) 4-bicyclisch substituierte Dihydropyridine, Verfahren zu ihrer Herstellung und ihre Verwendung in Arzneimitteln.

(57) Die Erfindung betrifft neue 4-bicyclisch substituierte Dihydropyridine der allgemeinen Formel (I)

EP 0 630 895 A1

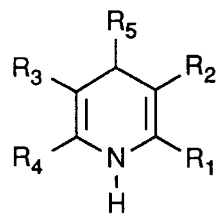

(I)

in welcher $R_1$ bis $R_5$ die in der Beschreibung angegebene Bedeutung haben, Verfahren zu ihrer Herstellung und ihre Verwendung in Arzneimitteln, insbesondere in Mitteln zur Behandlung von Herz-Kreislauferkrankungen.

Die Erfindung betrifft neue 4-bicyclisch substituierte Dihydropyridine, Verfahren zu ihrer Herstellung und ihre Verwendung in Arzneimitteln, insbesondere in Mitteln zur Behandlung von Herz-Kreislauferkrankungen.

Es ist bereits bekannt, daß 1,4-Dihydropyridine gefäßerweiternde Eigenschaften besitzen und als Coronarmittel und Antihypertensiva verwendet werden können. Weiterhin ist bekannt, daß 1,4-Dihydropyridine eine Hemmung der Kontraktionskraft von glatten und cardialen Muskeln bewirken und zur Behandlung von Coronar-und Gefäßerkrankungen eingesetzt werden können.

Ferner sind aus der US 5 100 900 schon 4-Chinolyl-dihydropyridine mit positiv inotroper Wirkung bekannt.

Die vorliegende Erfindung betrifft 4-bicyclisch substituierte Dihydropyridine der allgemeinen Formel (I)

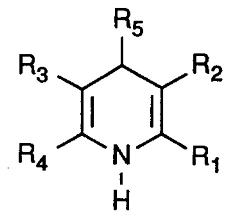

(I)

in welcher

| | |
|---|---|
| $R^1$ und $R^4$ | gleich oder verschieden sind und für Wasserstoff, Amino, Cyano, Formyl, Trifluormethyl oder für geradkettiges oder verzweigtes Alkyl mit bis zu 8 Kohlenstoffatomen stehen, das gegebenenfalls durch Hydroxy, geradkettiges oder verzweigtes Alkoxycarbonyl mit bis zu 6 Kohlenstoffatomen oder durch eine Gruppe der Formel $-NR^6R^7$, $-O-CO-R^8$, $-O-(CH_2)_a-OR^{8'}$ oder $-O-(CH_2)_b-NR^9R^{10}$ substituiert ist, worin |
| $R^6$, $R^7$, $R^9$ und $R^{10}$ | gleich oder verschieden sind und Wasserstoff, Phenyl oder geradkettiges oder verzweigtes Alkyl mit bis zu 6 Kohlenstoffatomen bedeuten, |
| $R^8$ und $R^{8'}$ | gleich oder verschieden sind und geradkettiges oder verzweigtes Alkyl mit bis zu 6 Kohlenstoffatomen bedeuten, und |
| a und b | gleich oder verschieden sind und eine Zahl 2, 3, 4 oder 5 bedeuten, |
| $R^2$ | für Cyano oder für eine Gruppe der Formel $-CO-NR^{11}R^{12}$ oder $-CO-A-R^{13}$ steht, worin |
| $R^{11}$ und $R^{12}$ | gleich oder verschieden sind und Wasserstoff, einen geradkettigen, verzweigten oder cyclischen, gesättigten oder ungesättigten Kohlenwasserstoffrest mit bis zu 8 Kohlenstoffatomen bedeuten, der gegebenenfalls durch Halogen, Hydroxy, Cyano oder durch Aryl, Aryloxy, Arylthio mit jeweils 6 bis 10 Kohlenstoffatomen oder durch einen 5- bis 7-gliedrigen, gesättigen oder ungesättigten Heterocyclus mit bis zu 3 Heteroatomen aus der Reihe S, N oder O substituiert ist, wobei die Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O bedeuten, die gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind, oder |
| $R^{11}$ und $R^{12}$ | gemeinsam unter Einbezug des Stickstoffatoms einen 3-bis 8-gliedrigen, gesättigen oder ungesättigten Heterocyclus bilden, der gegebenenfalls durch ein Sauerstoffatom oder durch einen Rest der Formel |

$S(O)_d$, -CO- oder $-NR^{15}$-unterbrochen sein kann,
worin

d eine Zahl 0, 1 oder 2 bedeutet,

$R^{15}$ Wasserstoff oder Aryl mit 6 bis 10 Kohlenstoffatomen bedeutet, das gegebenenfalls bis zu 2-fach gleich oder verschieden durch Halogen, Cyano oder durch geradkettiges oder verzweigtes Alkyl, Alkoxy, Alkylthio, Alkoxycarbonyl mit jeweils bis zu 8 Kohlenstoffatomen, Halogenalkyl, Halogenalkoxy oder Halogenalkylthio mit jeweils bis zu 4 Kohlenstoffatomen substituiert ist, oder
einen cyclischen, gesättigten oder ungesättigten, geradkettigen oder verzweigten Kohlenwasserstoffrest mit bis zu 8 Kohlenstoffatomen bedeutet, der gegebenenfalls durch Hydroxy, Halogen oder durch Aryl mit 6 bis 10 Kohlenstoffatomen oder einen 5- bis 7-gliedrigen, gesättigten oder ungesättigten Heterocyclus mit bis zu 3 Heteroatomen aus der Reihe S, N oder O substituiert ist, wobei die Cyclen ihrerseits bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,

und der Heterocyclus gegebenenfalls durch geradkettiges oder verzweigtes Alkoxy oder Alkylthio mit jeweils bis zu 4 Kohlenstoffatomen, Halogen, Aryl mit 6 bis 10 Kohlenstoffatomen, einen 5- bis 7-gliedrigen, gesättigten oder ungesättigten Heterocyclus mit bis zu 3 Heteroatomen aus der Reihe S, N oder O oder durch geradkettiges oder verzweigtes Alkyl mit bis zu 4 Kohlenstoffatomen substituiert ist, das seinerseits durch Aryl mit 6 bis 10 Kohlenstoffatomen substituiert sein kann,

A eine direkte Bindung oder ein Sauerstoffatom bedeutet,

$R^{13}$ Wasserstoff oder Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O bedeutet, wobei die Cyclen gegebenenfalls bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind, oder
einen cyclischen, geradkettigen oder verzweigten, gesättigten oder ungesättigten Kohlenwasserstoffrest mit bis zu 10 Kohlenstoffatomen bedeutet, der gegebenenfalls bis zu 3-fach gleich oder verschieden durch Sauerstoff oder durch - CO-, -CO-NH-, -O-CO-, -CO-O-, -NH-CO-, $-SO_2-NH-$, $-NH-SO_2-$, $-S(O)_e$-oder $-NR^{16}$- unterbrochen ist,
worin

e die oben angegebene Bedeutung von d hat und mit dieser gleich oder verschieden ist,

$R^{16}$ die oben angegebene Bedeutung von $R^{15}$ hat und mit dieser gleich oder verschieden ist,

oder der Kohlenwasserstoffrest gegebenenfalls bis zu 3-fach gleich oder verschieden durch Aryliden mit 6 bis 10 Kohlenstoffatomen oder heterocyclische Reste der Formeln

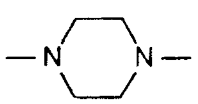 , 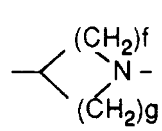 , 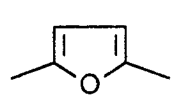 oder 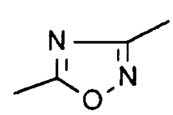

unterbrochen ist,
worin

f und g — gleich oder verschieden sind und eine Zahl 1 oder 2 bedeuten,

und wobei Aryliden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein kann,

und der Kohlenwasserstoffrest gegebenenfalls bis zu 3-fach gleich oder verschieden durch Cycloalkyl mit 3 bis 8 Kohlerstoffatomen, Halogen, Nitro, Cyano, Hydroxy, - $O-NO_2$, geradkettiges oder verzweigtes Alkylthio, Alkoxy oder Acyloxy mit jeweils bis zu 8 Kohlenstoffatomen oder durch Aryl, Aryloxy oder Arylthio mit jeweils 6 bis 10 Kohlenstoffatomen oder durch einen 5-bis 7-gliedrigen, gesättigten oder ungesättigten Heterocyclus mit bis zu 3 Heteroatomen aus der Reihe S, N oder O substituiert ist, wobei die Cyclen ihrerseits bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder

der Kohlenwasserstoffrest gegebenenfalls durch eine Gruppe der Formel $-CO_2-R^{17}$, $-CONR^{18}R^{19}$, $-NR^{20}R^{21}$ oder $-NR^{22}-CO_2R^{23}$ substituiert ist,

worin

$R^{17}$ — die oben angegebene Bedeutung von $R^{15}$ hat und mit dieser gleich oder verschieden ist

und

$R^{18}$, $R^{19}$, $R^{20}$, $R^{21}$, $R^{22}$ und $R^{23}$ — die oben angegebene Bedeutung von $R^{11}$ und $R^{12}$ haben und mit diesen gleich oder verschieden sind,

$R^3$ — für Cyano, Nitro, Formyl oder geradkettiges oder verzweigtes Alkoxycarbonyl mit bis zu 6 Kohlenstoffatomen steht, das gegebenenfalls durch geradkettiges oder verzweigtes Alkoxy mit bis zu 6 Kohlenstoffatomen substituiert ist, oder

für eine Gruppe der Formel - CO-NH-G steht,

worin

G — Cycloalkyl mit 3 bis 6 Kohlenstoffatomen bedeutet,

oder

$R^3$ und $R^4$ — gemeinsam einen Rest der Formel

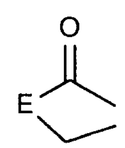

bilden,

worin

E — ein Sauerstoff-oder Schwefelatom oder die $-(CH_2)_n$-Gruppe bedeutet,

worin

n — eine Zahl 1 oder 2 bedeutet,

$R^5$ — für einen Rest der Formel

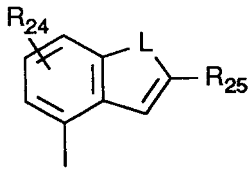, 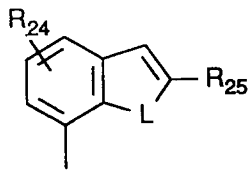, 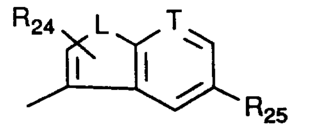,

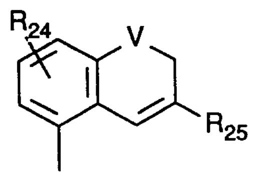, 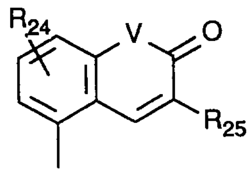, 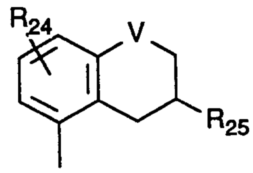,

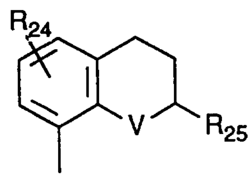, 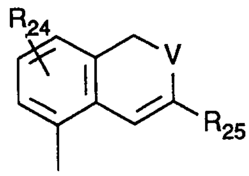, 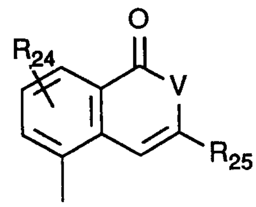,

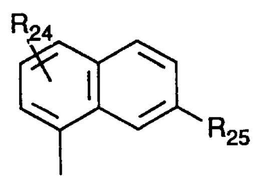, 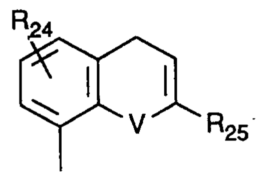, 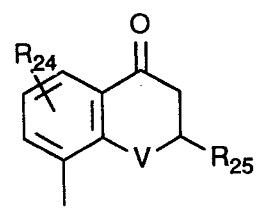,

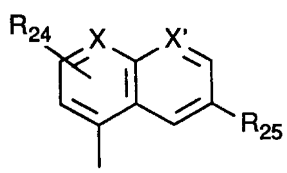, 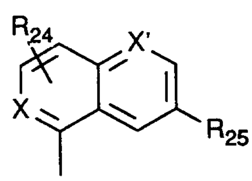, 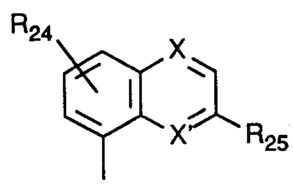,

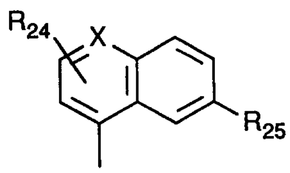, 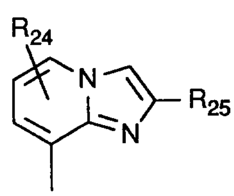 oder 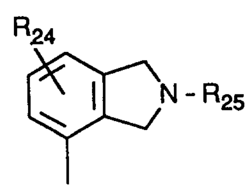

steht,
worin

$R^{24}$ Wasserstoff, Halogen oder geradkettiges oder verzweigtes Alkyl oder Alkoxy mit jeweils bis zu 8 Kohlenstoffatomen bedeutet,

$R^{25}$ Aryl mit 6 bis 10 Kohlenstoffatomen bedeutet, das gegebenenfalls bis zu 3-fach gleich oder verschieden durch Halogen, Nitro, Cyano, Trifluormethyl, Trifluormethoxy, Trifluormethylthio,
oder durch geradkettiges oder verzweigtes Alkyl mit bis zu 8 Kohlenstoffatomen substituiert ist, das seinerseits durch Aryl mit 6 bis 10 Kohlenstoffatomen substituiert sein kann,
oder durch geradkettiges oder verzweigtes Alkoxy oder Alkoxycarbonyl mit jeweils bis zu 8 Kohlenstoffatomen, Carboxy, Amino oder durch eine Gruppe der Formel - $NR^{26}R^{27}$ substituiert ist,
worin

| | |
|---|---|
| $R^{26}$ und $R^{27}$ | gleich oder verschieden sind und Wasserstoff, geradkettiges oder verzweigtes Alkyl mit bis zu 8 Kohlenstoffatomen, Phenyl oder Benzyl bedeuten, oder |
| $R^{25}$ | einen cyclischen, geradkettigen oder verzweigten, gesättigten oder ungesättigten Kohlenwasserstoffrest mit bis zu 12 Kohlenstoffatomen bedeutet, der gegebenenfalls bis zu 2-fach gleich oder verschieden durch Sauerstoff oder Schwefel unterbrochen ist, und der gegebenenfalls bis zu 3-fach gleich oder verschieden durch Cycloalkyl mit 3 bis 8 Kohlenstoffatomen, geradkettiges oder verzweigtes Acyloxy mit bis zu 4 Kohlenstoffatomen, Halogen, Nitro, Cyano, Hydroxy oder durch Aryl, Aryloxy oder Arylthio mit jeweils 6 bis 10 Kohlenstoffatomen oder durch einen 5-bis 7-gliedrigen, gesättigten oder ungesättigen gegebenenfalls kondensierten Heterocyclus mit bis zu 5 Heteroatomen aus der Reihe S, N oder O substituiert ist, wobei die Cyclen ihrerseits bis zu 3-fach gleich oder verschieden durch Halogen, Cyano, Nitro, Hydroxy oder durch geradkettiges oder verzweigtes Alkyl oder Alkoxy mit jeweils bis zu 4 Kohlenstoffatomen, Trifluormethyl, Trifluormethoxy, Trifluormethylthio oder durch eine Gruppe der Formel - $NR^{28}R^{29}$ substituiert sein können, worin |
| $R^{28}$ und $R^{29}$ | die oben angegebene Bedeutung von $R^{11}$ und $R^{12}$ haben und mit dieser gleich oder verschieden sind, oder der Kohlenwasserstoffrest gegebenenfalls durch eine Gruppe der Formel $-CO_2-R^{30}$, $-CONR^{31}R^{32}$, - $NR^{33}R^{34}$, - $NR^{35}-CO_2R^{36}$ oder $-NR^{37}-SO_2R^{38}$ substituiert ist, worin |
| $R^{30}$ | die oben angegebene Bedeutung von $R^{15}$ hat und mit dieser gleich oder verschieden ist und |
| $R^{31}$, $R^{32}$, $R^{33}$, $R^{34}$, $R^{35}$, $R^{36}$, $R^{37}$ und $R^{38}$ | die oben angegebene Bedeutung von $R^{11}$ und $R^{12}$ haben und mit diesen gleich oder verschieden sind, oder |
| $R^{25}$ | einen 5- bis 7-gliedrigen, gesättigten oder ungesättigten Heterocyclus mit bis zu 4 Heteroatomen aus der Reihe S, N oder O bedeutet, der gegebenenfalls bis zu 3-fach gleich oder verschieden durch Halogen, Amino, Cyano, Nitro oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen oder durch $C_1$-$C_4$-Mono- oder - Dialkylamino substituiert ist, oder |
| $R^{25}$ | eine Gruppe der Formel $D-R^{39}$ bedeutet, worin |
| D | die CO- oder - $S(O)_h$-Gruppe oder ein Sauerstoffatom bedeutet, worin |
| h | eine Zahl 0, 1 oder 2 bedeutet, und |
| $R^{39}$ | Aryl mit 6 bis 10 Kohlenstoffatomen oder einen 5-bis 7-gliedrigen, gesättigten oder ungesättigten Heterocyclus mit |

bis zu 3 Heteroatomen aus der Reihe S, N oder O bedeutet, der gegebenenfalls bis zu 3-fach gleich oder verschieden durch Halogen, Amino, Cyano, Nitro oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen oder durch $C_1$-$C_4$-Mono- oder -Dialkylamino substituiert ist,

oder

| | |
|---|---|
| $R^{39}$ | Wasserstoff oder einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel unterbrochen ist, und der gegebenenfalls durch Halogen, Aryl, Aryloxy oder Arylthio mit jeweils 6 bis 10 Kohlenstoffatomen oder durch einen 5- bis 7-gliedrigen, gesättigten oder ungesättigen Heterocyclus mit bis zu 3 Heteroatomen aus der Reihe S, N oder O substituiert ist, wobei die Cyclen ihrerseits durch Halogen, Trifluormethyl, Methyl, Methoxy, Nitro oder Methylthio substituiert sein können, oder durch eine Gruppe der Formel - $NR^{40}R^{41}$ substituiert ist worin |
| $R^{40}$ und $R^{41}$ | die oben angegebene Bedeutung von $R^{11}$ und $R^{12}$ haben und mit dieser gleich oder verschieden sind, |
| L | ein Schwefel-oder Sauerstoffatom oder die - NH-Gruppe bedeutet, |
| T | ein Stickstoffatom oder die N->O-Gruppe bedeutet, |
| V | ein Schwefel-oder Sauerstoffatom bedeutet, |
| X und X' | gleich oder verschieden sind und ein Stickstoffatom oder die N->O-Gruppe bedeuten, |

und deren Salze.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulforsäure, Ethansulfonsäure, Phenylsulfonsäure, Toluolsulfonsäure oder Naphthalindisulfonsäure.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Bevorzugt sind Verbindungen der allgemeinen Formel (I),
in welcher

| | |
|---|---|
| $R^1$ und $R^4$ | gleich oder verschieden sind und für Wasserstoff, Amino, Cyano, Formyl, Trifluormethyl oder für geradkettiges oder verzweigtes Alkyl mit bis zu 4 Kohlenstoffatomen stehen, das gegebenenfalls durch Hydroxy, geradkettiges oder verzweigtes Alkoxycarbonyl mit bis zu 4 Kohlenstoffatomen oder durch eine Gruppe der Formel -$NR^6R^7$, - O-CO-$R^8$, -O-$(CH_2)_a$-$OR^{8'}$ oder -O-$(CH_2)_b$-$NR^9R^{10}$ substituiert ist, worin |
| $R^6$, $R^7$, $R^9$ und $R^{10}$ | gleich oder verschieden sind und Wasserstoff oder geradkettiges oder verzweigtes Alkyl mit bis zu 4 Kohlenstoffatomen bedeuten, |
| $R^8$ und $R^{8'}$ | gleich oder verschieden sind und geradkettiges oder verzweigtes Alkyl mit bis zu 4 Kohlenstoffatomen bedeuten, und |
| a und b | gleich oder verschieden sind und eine Zahl 2, 3, 4 oder 5 bedeuten, |
| $R^2$ | für Cyano oder für eine Gruppe der Formel - CO-$NR^{11}R^{12}$ oder -CO-A-$R^{13}$ steht, |

worin

$R^{11}$ und $R^{12}$ gleich oder verschieden sind und Wasserstoff, einen geradkettigen, verzweigten oder cyclischen, gesättigten oder ungesättigten Kohlenwasserstoffrest mit bis zu 6 Kohlenstoffatomen bedeuten, der gegebenenfalls durch Fluor, Chlor, Hydroxy, Phenyl oder Pyridyl substituiert ist, wobei die Cyclen ihrerseits durch Fluor, Chlor oder durch Alkyl, Alkoxy, Alkylthio oder Alkoxycarbonyl mit jeweils bis zu 2 Kohlenstoffatomen, Trifluormethyl oder Trifluormethoxy substituiert sein können,

oder

Phenyl oder Pyridyl bedeuten, die gegebenenfalls durch Fluor, Chlor oder durch Alkyl, Alkoxy, Alkylthio oder Alkoxycarbonyl mit jeweils bis zu 2 Kohlenstoffatomen, Trifluormethyl oder Trifluormethoxy substituiert sind,

oder

$R^{11}$ und $R^{12}$ gemeinsam unter Einbezug des Stickstoffatoms einen 3-bis 8-gliedrigen, gesättigen oder ungesättigten Heterocyclus bilden, der gegebenenfalls durch ein Sauerstoffatom oder durch einen Rest der Formel $S(O)_d$, -CO- oder $-NR^{15}$-unterbrochen sein kann,

worin

d eine Zahl 0, 1 oder 2 bedeutet,

$R^{15}$ Wasserstoff oder Phenyl bedeutet, das gegebenenfalls durch Fluor, Chlor, Methyl, Ethyl, Methoxy, Ethoxy, Trifluormethyl oder Trifluormethoxy substituiert ist, oder

einen cyclischen, gesättigten oder ungesättigten, geradkettigen oder verzweigten Kohlenwasserstoffrest mit bis zu 4 Kohlenstoffatomen bedeutet, der gegebenenfalls durch Fluor, Chlor, Phenyl oder durch einen 5-bis 7-gliedrigen, gesättigten oder ungesättigten Heterocyclus mit bis zu 3 Heteroatomen aus der Reihe S, N oder O substituiert ist, wobei die Cyclen ihrerseits durch Fluor, Chlor, Methyl, Methoxy, Methylthio, Trifluormethyl oder Trifluormethoxy substituiert sein können,

A eine direkte Bindung oder ein Sauerstoffatom bedeutet,

$R^{13}$ Wasserstoff, Phenyl oder Pyridyl bedeutet, die gegebenenfalls durch Fluor, Chlor, Methyl, Methoxy, Methylthio, Trifluormethyl oder Trifluormethoxy substituiert sind, oder

einen cyclischen, geradkettigen oder verzweigten, gesättigten oder ungesättigten Kohlenwasserstoffrest mit bis zu 8 Kohlenstoffatomen bedeutet, der gegebenenfalls bis zu 2-fach gleich oder verschieden durch Sauerstoff oder durch - CO-, -CO-NH-, -O-CO-, -CO-O-, -NH-CO-, $-SO_2-NH-$, $-NH-SO_2-$, $-S(O)_e$-oder $-NR^{16}$- unterbrochen ist,

worin

e die oben angegebene Bedeutung von d hat und mit dieser gleich oder verschieden ist,

$R^{16}$ die oben angegebene Bedeutung von $R^{15}$ hat und mit dieser gleich oder verschieden ist,

oder der Kohlenwasserstoffrest gegebenenfalls bis zu 2-fach gleich oder verschieden durch Aryliden mit 6 bis 10 Kohlenstoffatomen oder heterocyclische Reste der Formeln

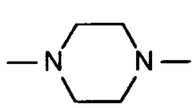
,
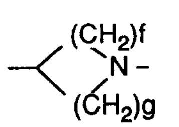
,
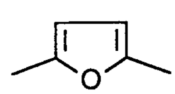
oder
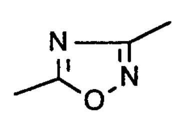

unterbrochen ist,

worin

f und g gleich oder verschieden sind und eine Zahl 1 oder 2 bedeuten,

und der Kohlenwasserstoffrest gegebenenfalls bis zu 2-fach gleich oder verschieden durch Cyclopropyl, Cyclobutyl, Cyclopentyl, Cyclohexyl, Fluor, Chlor, Nitro, Cyano, Hydroxy, - $O-NO_2$, geradkettiges oder verzweigtes Alkylthio, Alkoxy oder Acyloxy mit jeweils bis zu 4 Kohlenstoffatomen oder durch Phenyl, Phenoxy, Phenylthio oder Pyridyl substituiert ist, wobei die Cyclen ihrerseits durch Fluor, Chlor, Cyano, Methyl, Methoxy, Methylthio, Trifluormethyl oder Trifluormethoxy substituiert sein können, oder

der Kohlenwasserstoffrest gegebenenfalls durch eine Gruppe der Formel $-CO_2-R^{17}$, $-CONR^{18}R^{19}$, - $NR^{20}R^{21}$ oder $-NR^{22}-CO_2R^{23}$ substituiert ist,

worin

$R^{17}$ die oben angegebene Bedeutung von $R^{15}$ hat und mit dieser gleich oder verschieden ist

und

$R^{18}$, $R^{19}$, $R^{20}$, $R^{21}$, $R^{22}$ und $R^{23}$ die oben angegebene Bedeutung von $R^{11}$ und $R^{12}$ haben und mit diesen gleich oder verschieden sind,

$R^3$ für Cyano, Nitro, Formyl oder geradkettiges oder verzweigtes Alkoxycarbonyl mit bis zu 4 Kohlenstoffatomen steht, das gegebenenfalls durch geradkettiges oder verzweigtes Alkoxy mit bis zu 4 Kohlenstoffatomen substituiert ist, oder

für eine Gruppe der Formel - CO-NH-G steht,

worin

G Cyclopropyl, Cyclopentyl oder Cyclohexyl bedeutet,

oder

$R^3$ und $R^4$ gemeinsam einen Rest der Formel

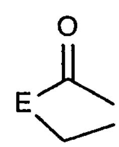

bilden,

worin

E ein Sauerstoff-oder Schwefelatom oder die - $(CH_2)_n$-Gruppe bedeutet,

worin

n eine Zahl 1 oder 2 bedeutet,

$R^5$ für einen Rest der Formel

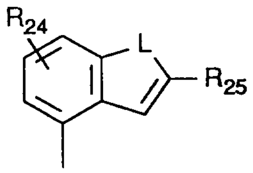 , 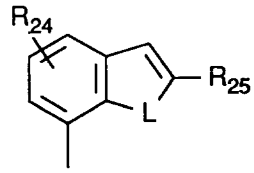 , 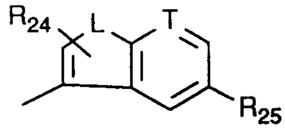 ,

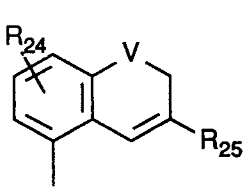 , 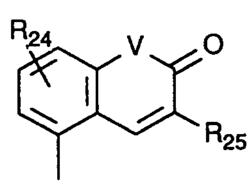 , 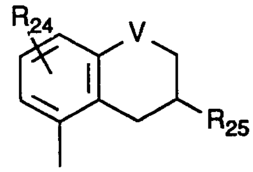 ,

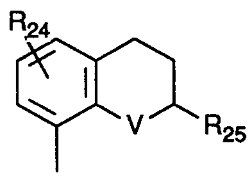 , 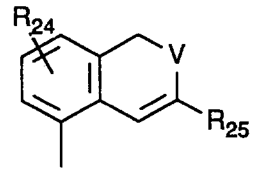 , 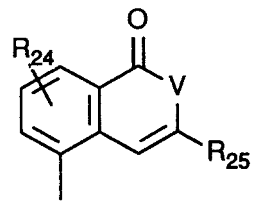 ,

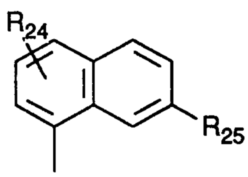 , 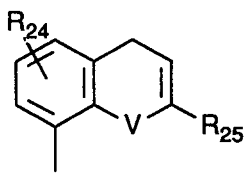 , 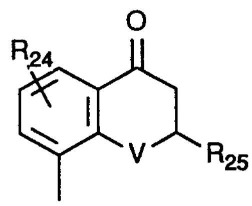 ,

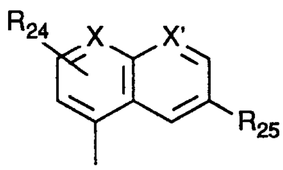 , 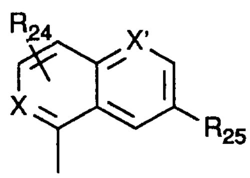 , 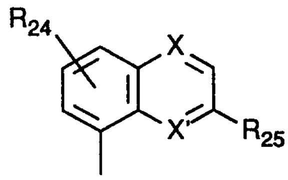 ,

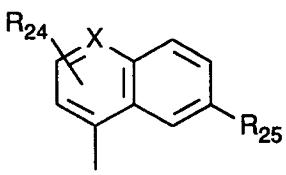 , 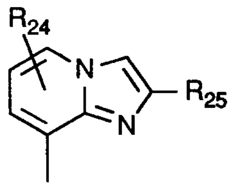 oder 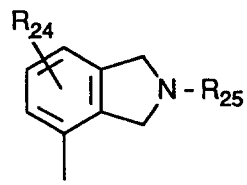

steht,
worin

| | |
|---|---|
| $R^{24}$ | Wasserstoff, Fluor oder Chlor bedeutet, |
| $R^{25}$ | Phenyl bedeutet, das gegebenenfalls bis zu 2-fach gleich oder verschieden durch Halogen, Nitro, Cyano, Trifluormethyl oder durch geradkettiges oder verzweigtes Alkyl oder Alkoxy mit jeweils bis zu 4 Kohlenstoffatomen oder durch eine Gruppe der Formel - $NR^{26}R^{27}$ substituiert ist, worin |
| $R^{26}$ und $R^{27}$ | gleich oder verschieden sind und Wasserstoff, geradkettiges oder verzweigtes Alkyl mit bis zu 6 Kohlenstoffatomen, Phenyl oder Benzyl bedeuten, |

oder

$R^{25}$ einen cyclischen, geradkettigen oder verzweigten, gesättigten oder ungesättigten Kohlenwasserstoffrest mit bis zu 10 Kohlenstoffatomen bedeutet, der gegebenenfalls durch Sauerstoff oder Schwefel unterbrochen ist,
und der gegebenenfalls bis zu 2-fach gleich oder verschieden durch Cyclopropyl, Cyclobutyl, Cyclopentyl, Cyclohexyl, Fluor, Chlor, Brom, Acyloxy mit bis zu 2 Kohlenstoffatomen, Cyano, Hydroxy oder durch Phenyl, Phenyloxy, Phenylthio oder durch einen 5-bis 7-gliedrigen, gesättigten oder ungesättigen Heterocyclus mit bis zu 3 Heteroatomen aus der Reihe S, N oder O substituiert ist, wobei die Phenyl- und die Hetero-Cyclen ihrerseits bis zu 2-fach gleich oder verschieden durch Fluor, Chlor, Brom, Hydroxy, Methyl, Ethyl, Methoxy, Ethoxy, Trifluormethyl, Trifluormethoxy oder Trifluormethylthio oder durch eine Gruppe der Formel $-NR^{28}R^{29}$ substituiert sein können,
worin

$R^{28}$ und $R^{29}$ die oben angegebene Bedeutung von $R^{11}$ und $R^{12}$ haben und mit dieser gleich oder verschieden sind,
oder der Kohlenwasserstoffrest gegebenenfalls durch eine Gruppe der Formel $-CO_2-R^{30}$, $-CONR^{31}R^{32}$, $-NR^{33}R^{34}$, $-NR^{35}-CO_2R^{36}$ oder $-NR^{37}-SO_2R^{38}$ substituiert ist,
worin

$R^{30}$ die oben angegebene Bedeutung von $R^{15}$ hat und mit dieser gleich oder verschieden ist
und

$R^{31}$, $R^{32}$, $R^{33}$, $R^{34}$, $R^{35}$, $R^{36}$, $R^{37}$ und $R^{38}$ die oben angegebene Bedeutung von $R^{11}$ und $R^{12}$ haben und mit diesen gleich oder verschieden sind,
oder

$R^{25}$ einen 5- bis 7-gliedrigen, gesättigten oder ungesättigten Heterocyclus mit bis zu 4 Heteroatomen aus der Reihe S, N oder O bedeutet, der gegebenenfalls bis zu 2-fach gleich oder verschieden durch Fluor, Chlor, Brom, Cyano, Nitro oder durch Alkyl, Alkoxy, Alkylthio, Alkoxycarbonyl, Halogenalkyl, Halogenalkoxy oder Halogenalkylthio mit jeweils bis zu 2 Kohlenstoffatomen, Amino oder durch $C_1$-$C_4$-Mono- oder - Dialkylamino substituiert ist,
oder

$R^{25}$ eine Gruppe der Formel $D-R^{39}$ bedeutet,
worin

D die CO- oder - $S(O)_h$-Gruppe oder ein Sauerstoffatom bedeutet,
worin

h eine Zahl 0, 1 oder 2 bedeutet,
und

$R^{39}$ Phenyl oder einen 5-bis 7-gliedrigen, gesättigten oder ungesättigten Heterocyclus mit bis zu 3 Heteroatomen aus der Reihe S, N oder O bedeutet, der gegebenenfalls bis zu 2-fach gleich oder verschieden durch Fluor, Chlor, Brom, Cyano, Nitro oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen, Amino oder durch $C_1$-$C_4$-Mono- oder - Dialkylamino substituiert ist,
oder

$R^{39}$ Wasserstoff oder einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel unterbrochen ist, und der gegebenenfalls durch Fluor, Chlor, Phenyl, Phenoxy oder Phenylthio substituiert sein kann, oder durch eine Gruppe der Formel - $NR^{40}R^{41}$ substituiert ist worin

$R^{40}$ und $R^{41}$ die oben angegebene Bedeutung von $R^{11}$ und $R^{12}$ haben und mit dieser gleich oder verschieden sind,

L ein Schwefel-oder Sauerstoffatom oder die - NH-Gruppe bedeutet,

T ein Stickstoffatom oder die N->O-Gruppe bedeutet,

V ein Schwefel-oder Sauerstoffatom bedeutet,

X und X' gleich oder verschieden sind und ein Stickstoffatom oder die N->O-Gruppe bedeuten

und deren Salze.

Besonders bevorzugt sind Verbindungen der allgemeinen Formel (I), in welcher

$R^1$ und $R^4$ gleich oder verschieden sind und für Wasserstoff, Amino oder geradkettiges oder verzweigtes Alkyl mit bis zu 3 Kohlenstoffatomen stehen, das gegebenenfalls durch Methoxycarbonyl oder durch die Gruppe der Formel $-O-CO-CH_3$ substituiert ist,

$R^2$ für Cyano oder für eine Gruppe der Formel - $CO-NR^{11}R^{12}$ oder $-CO-A-R^{13}$ steht, worin

$R^{11}$ und $R^{12}$ gleich oder verschieden sind und Wasserstoff, einen geradkettigen, verzweigten oder cyclischen, gesättigten oder ungesättigten Kohlenwasserstoffrest mit bis zu 6 Kohlenstoffatomen bedeuten, oder Phenyl bedeuten, das gegebenenfalls durch Fluor, Chlor, Methyl oder Methoxy substituiert ist,

A eine direkte Bindung oder ein Sauerstoffatom bedeutet,

$R^{13}$ Wasserstoff oder einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel oder durch -CO-NH-, -O-CO-, -CO-O-, -NH-CO-, $-SO_2-NH-$, $-NH-SO_2-$ oder $-NR^{16}$ unterbrochen ist, worin

$R^{16}$ Wasserstoff, geradkettiges oder verzweigtes Alkyl mit bis zu 4 Kohlenstoffatomen bedeutet,
oder der Kohlenwasserstoffrest durch heterocyclische Reste der Formeln

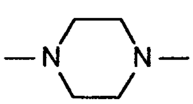

oder

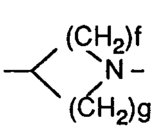

unterbrochen ist, worin

f und g gleich oder verschieden sind und eine Zahl 1 oder 2 bedeuten,

und der Kohlenwasserstoffrest gegebenenfalls durch Cyclopropyl, Cyclobutyl, Cyclopentyl, Cyclohexyl, Fluor, Chlor, Nitro, Cyano, Hydroxy oder durch Phenyl, Phenoxy, Phenylthio oder Pyridyl substituiert ist, die ihrerseits durch Fluor, Chlor, Methyl, Methoxy, Methylthio, Trifluormethyl oder Trifluormethoxy substituiert sein können, oder der Kohlenwasserstoffrest gegebenenfalls durch eine Gruppe der Formel $-CO_2R^{17}$, $-CONR^{18}R^{19}$, $-NR^{20}R^{21}$ oder - $NR^{22}-CO_2R^{23}$ substituiert ist, worin

$R^{20}$ und $R^{21}$ gleich oder verschieden sind und Wasserstoff, einen geradkettigen, verzweigten oder cyclischen, gesättigten oder ungesättigten Kohlenwasserstoffrest mit bis zu 6 Kohlenstoffatomen bedeuten, das gegebenenfalls durch Fluor, Chlor, Pyridyl oder Phenyl substituiert ist, das seinerseits durch Fluor, Chlor, Methyl oder Methoxy substituiert sein kann, oder

Phenyl bedeuten, das gegebenenfalls durch Fluor, Chlor, Methyl oder Methoxy substituiert ist,

oder

$R^{20}$ und $R^{21}$ gemeinsam unter Einbezug des Stickstoffatoms einen 5-bis 6-gliedrigen, gesättigen oder ungesättigten Heterocyclus bilden, der gegebenenfalls bis zu 2 weitere Heteroatome aus der Reihe S, N oder O enthalten kann und der gegebenenfalls durch geradkettiges oder verzweigtes Alkyl mit bis zu 4 Kohlenstoffatomen, Benzyl oder Phenyl substituiert ist,

$R^{17}$, $R^{18}$, $R^{19}$, $R^{22}$ und $R^{23}$ gleich oder verschieden sind und Wasserstoff, geradkettiges oder verzweigtes Alkyl mit bis zu 6 Kohlenstoffatomen bedeuten, das gegebenenfalls durch Phenyl substituiert ist, oder

Phenyl bedeuten, das gegebenenfalls durch Fluor, Chlor oder Brom substituiert ist,

$R^3$ für Cyano, Nitro, Formyl oder geradkettiges oder verzweigtes Alkoxycarbonyl mit bis zu 4 Kohlenstoffatomen steht, das gegebenenfalls durch geradkettiges oder verzweigtes Alkoxy mit bis zu 4 Kohlenstoffatomen substituiert ist, oder

für eine Gruppe der Formel -CO-NH-G steht,

worin

G Cyclopropyl oder Cyclopentyl bedeutet,

oder

$R^3$ und $R^4$ gemeinsam einen Rest der Formel

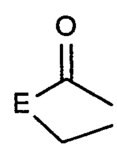

bilden,

worin

E ein Sauerstoff-oder Schwefelatom oder die - $(CH_2)_n$-Gruppe bedeutet,

worin

n eine Zahl 1 oder 2 bedeutet,

$R^5$ für einen Rest der Formel

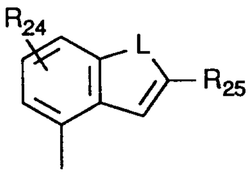 , 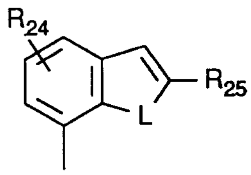 , 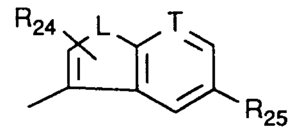 ,

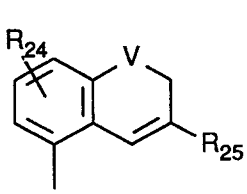 , 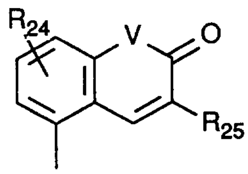 , 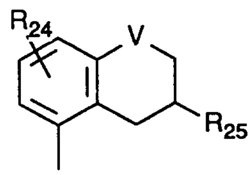 ,

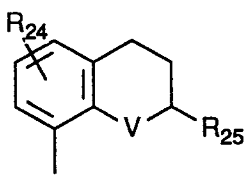 , 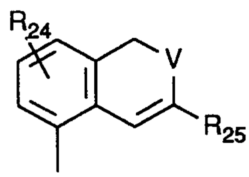 , 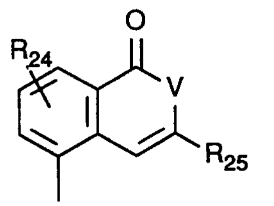 ,

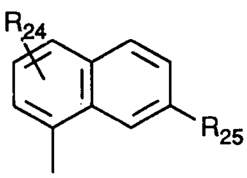 , 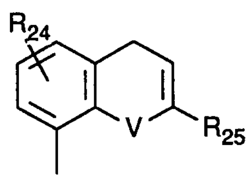 , 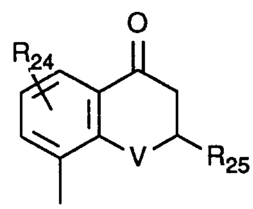 ,

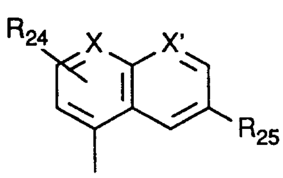 , 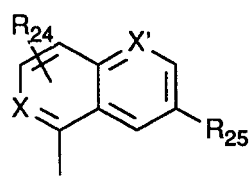 , 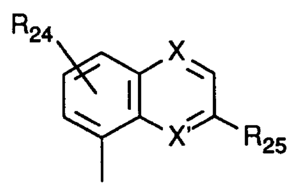 ,

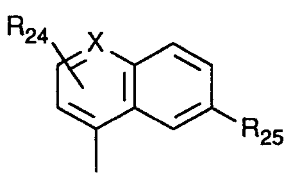 , 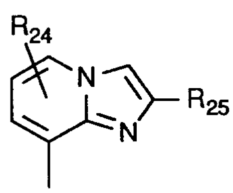 oder 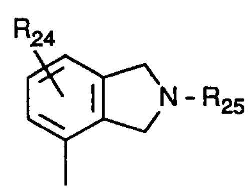

steht,
worin

$R^{24}$ Wasserstoff, Fluor oder Chlor bedeutet,

$R^{25}$ Phenyl bedeutet, das gegebenenfalls bis zu 2-fach gleich oder verschieden durch Fluor, Chlor, Nitro, Trifluormethyl, Methyl, Ethyl, Methoxy oder Ethoxy oder durch eine Gruppe der Formel - $NR^{26}R^{27}$ substituiert ist,
worin

$R^{26}$ und $R^{27}$ gleich oder verschieden sind und Wasserstoff oder geradkettiges oder verzweigtes Alkyl mit bis zu 4 Kohlenstoffatomen, Phenyl oder Benzyl bedeuten,
oder

| | |
|---|---|
| $R^{25}$ | einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel unterbrochen ist, und der gegebenenfalls durch Cyclopropyl, Cyclobutyl, Cyclopentyl, Cyclohexyl, Fluor, Chlor, Nitro, Cyano, Hydroxy oder durch Phenyl, Phenyloxy, Phenylthio oder durch einen 5-bis 7-gliedrigen, gesättigten oder ungesättigen Heterocyclus mit bis zu 2 Heteroatomen aus der Reihe S, N oder O substituiert ist, wobei die Phenyl-und die Hetero-Cyclen ihrerseits bis zu 2-fach gleich oder verschieden durch Fluor, Chlor, Methyl, Methoxy, Trifluormethyl oder Trifluormethoxy oder durch eine Gruppe der Formel - $NR^{28}R^{29}$ substituiert sein können, worin |
| $R^{28}$ und $R^{29}$ | die oben angegebene Bedeutung von $R^{11}$ und $R^{12}$ haben und mit dieser gleich oder verschieden sind, oder der Kohlenwasserstoffrest gegebenenfalls durch eine Gruppe der Formel $-CO_2-R^{30}$, $-CONR^{31}R^{32}$, - $NR^{33}R^{34}$, - $NR^{35}-CO_2R^{36}$ oder $-NR^{37}-SO_2R^{38}$ substituiert ist, worin |
| $R^{30}$ | Alkyl mit 1-4 C-Atomen oder Phenyl bedeutet, und |
| $R^{31}$, $R^{32}$, $R^{33}$, $R^{34}$, $R^{35}$, $R^{36}$, $R^{37}$ und $R^{38}$ | die oben angegebene Bedeutung von $R^{11}$ und $R^{12}$ haben und mit diesen gleich oder verschieden sind, oder |
| $R^{25}$ | einen 5- bis 6-gliedrigen, gesättigten oder ungesättigten Heterocyclus mit bis zu 2 Heteroatomen aus der Reihe S, N oder O bedeutet, der gegebenenfalls durch Fluor, Chlor, Methyl, Methoxy, Methylthio oder Trifluormethyl substituiert ist, oder |
| $R^{25}$ | eine Gruppe der Formel $D-R^{39}$ bedeutet, worin |
| D | die $-S(O)_h$-Gruppe oder ein Sauerstoffatom bedeutet, worin |
| h | eine Zahl 0, 1 oder 2 bedeutet, und |
| $R^{39}$ | Phenyl bedeutet, das gegebenenfalls durch Fluor, Chlor, Brom, Methyl, Methoxy, Methylthio,Trifluormethyl, Amino oder durch $C_1$-$C_2$-Mono- oder - Dialkylamino substituiert ist, oder |
| $R^{39}$ | Wasserstoff, einen cyclischen, geradkettigen oder verzweigten, gesättigten oder ungesättigten Kohlenwasserstoffrest mit bis zu 6 Kohlenstoffatomen bedeutet, der gegebenenfalls durch Sauerstoff oder Schwefel unterbrochen ist, und der gegebenenfalls durch Fluor, Chlor oder Phenyl substituiert ist, oder durch eine Gruppe der Formel - $NR^{40}R^{41}$ substituiert ist worin |
| $R^{40}$ und $R^{41}$ | die oben angegebene Bedeutung von $R^{11}$ und $R^{12}$ haben und mit dieser gleich oder verschieden sind, |
| L | ein Schwefel-oder Sauerstoffatom oder die - NH-Gruppe bedeutet, |
| T | ein Stickstoffatom oder die N->O-Gruppe bedeutet, |
| V | ein Schwefel-oder Sauerstoffatom bedeutet, |
| X und X' | gleich oder verschieden sind und ein Stickstoffatom oder die N->O-Gruppe bedeuten |

und deren Salze.

Außerdem wurden Verfahren zur Herstellung der erfindungsgemäßen Verbindungen der allgemeinen Formel (I) gefunden, dadurch gekennzeichnet,
daß man im Fall, daß $R^3$ für Cyano, Nitro oder Formyl steht,
[A] Verbindungen der allgemeinen Formel (II)

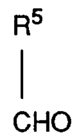

(II)

in welcher
$R^5$ die oben angegebene Bedeutung hat,
zunächst mit Acylverbindungen der allgemeinen Formel (III)

$R^3$-CO-$CH_2$-$R^4$ (III)

in welcher
$R^3$ und $R^4$ die oben angegebene Bedeutung haben
gegebenenfalls unter Isolierung der Ylidenverbindungen der allgemeinen Formel (IV)

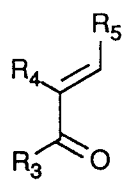

(IV)

in welcher
$R^3$, $R^4$ und $R^5$ die oben angegebene Bedeutung haben
umsetzt, anschließend mit Verbindungen der Formel (V)

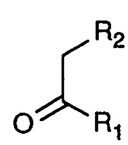

(V)

in welcher
$R^1$ und $R^2$ die oben angegebene Bedeutung haben,
und einer reaktiven Ammoniumverbindung, z.B. Ammoniumacetat, oder direkt mit Enamino-Verbindungen der allgemeinen Formel (VI)

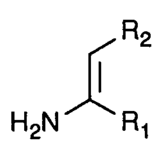

(VI)

in welcher
$R^1$ und $R^2$ die oben angegebene Bedeutung haben,
in inerten Lösemitteln umsetzt,
oder

[B] Verbindungen der allgemeinen Formel (II) zunächst mit Verbindungen der allgemeinen Formel (V), gegebenenfalls unter Isolierung der Ylidenverbindungen der allgemeinen Formel (VII)

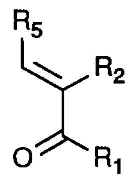
(VII)

in welcher

$R^1$, $R^2$ und $R^5$ die oben angegebene Bedeutung haben,

umsetzt und anschließend entweder mit Verbindungen der allgemeinen Formel (III) in Gegenwart von Ammoniumverbindungen oder direkt mit Verbindungen der allgemeinen Formel (VIII)

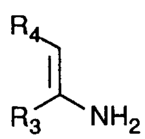
(VIII)

in welcher

$R^3$ und $R^4$ die oben angegebenene Bedeutung haben,

umsetzt

oder

[C] im Fall, daß $R^3$ und $R^4$ zusammen einen Rest der Formel

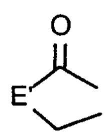

bilden,

worin

E' für ein Sauerstoff- oder Schwefelatom steht,

zunächst nach dem unter [A] und [B] aufgeführten Methoden Verbindungen der allgemeinen Formel (IX)

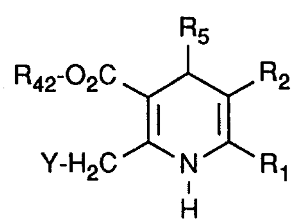
(IX)

in welcher

$R^1$, $R^2$ und $R^5$ die oben angegebene Bedeutung haben,

$R^{42}$ für $C_1$-$C_4$-Alkyl steht

und

Y für $C_1$-$C_4$-Acyloxy oder Acylthio steht,

herstellt und anschließend einen basischen oder sauren Ringschluß nach bekannten Methoden durchführt,
oder
[D] daß man im Fall, daß E für die $-(CH_2)_n$-Gruppe steht,
Verbindungen der allgemeinen Formel (II),zunächst mit Acylverbindungen der allgemeinen Formel (X)

$Z\text{-}CO\text{-}CH_2\text{-}R^2$ (X)

in welcher
$R^2$ die oben angegebene Bedeutung hat
und
Z die oben angegebene Bedeutung von $R^1$ hat, wobei im Fall der Hydroxy-und/oder Aminofunktionen, diese gegebenenfalls in geschützter Form vorliegen,
gegebenenfalls unter Isolierung der Ylidenverbindungen der allgemeinen Formel (XI)

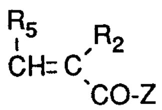
(XI)

in welcher
$R^2$, $R^5$ und Z die oben angegebene Bedeutung haben
umsetzt, anschließend mit der Verbindung der Formel (XII)

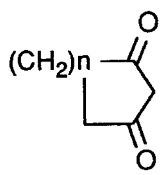
(XII)

in welcher
n eine Zahl 1 oder 2 bedeutet,
und einer reaktiven Ammoniumverbindung, z.B. Ammoniumacetat, gegebenenfalls unter Isolierung der Zwischenprodukte der allgemeinen Formel (XIII)

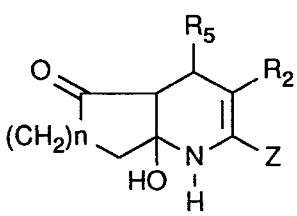
(XIII)

in welcher
$R^2$, $R^5$, n und Z die oben angegebene Bedeutung haben,
in inerten Lösemitteln umsetzt,
und anschließend in einem letzten Schritt, gegebenenfalls in Anwesenheit eines Hilfsmittels, Wasser abscheidet,
oder
[E] Verbindungen der allgemeinen Formel (II) mit Verbindungen der allgemeinen Formel (VIIIa) und (XIV)

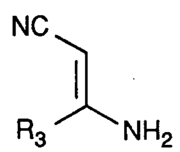 (VIIIa) und 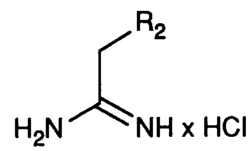 (XIV)

in welcher

$R^2$ und $R^3$ die oben angegebene Bedeutung haben,

in einem der oben aufgeführten Lösemittel umsetzt.

Die erfindungsgemäßen Verfahren können durch folgende Formelschema beispielhaft erläutert werden:

[A]

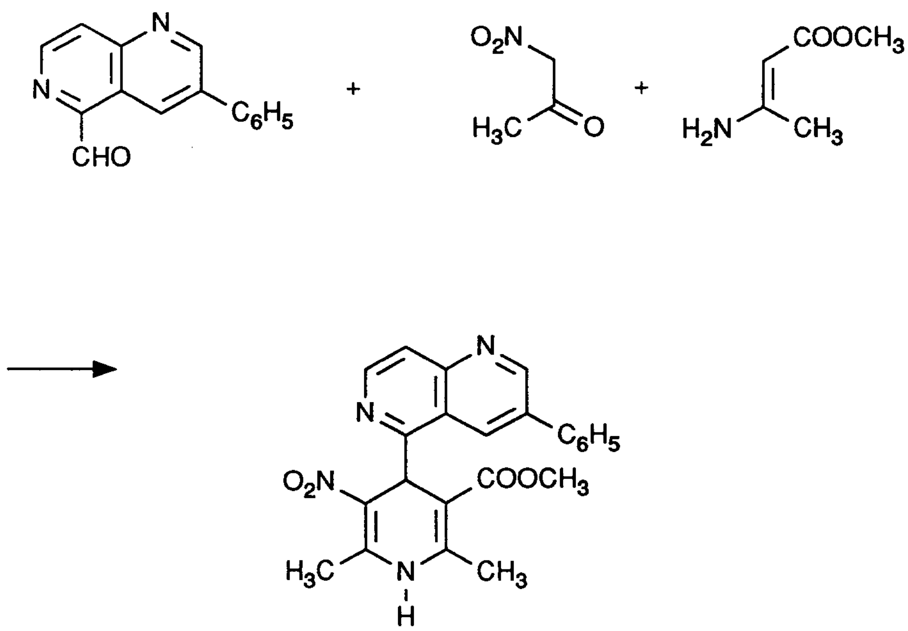

[B]

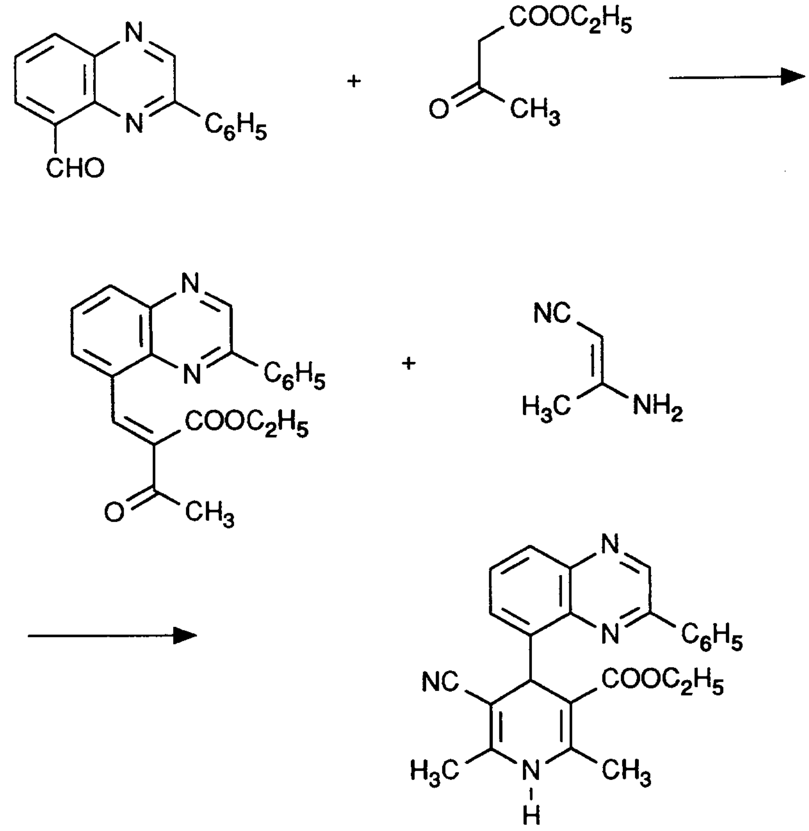

[C]

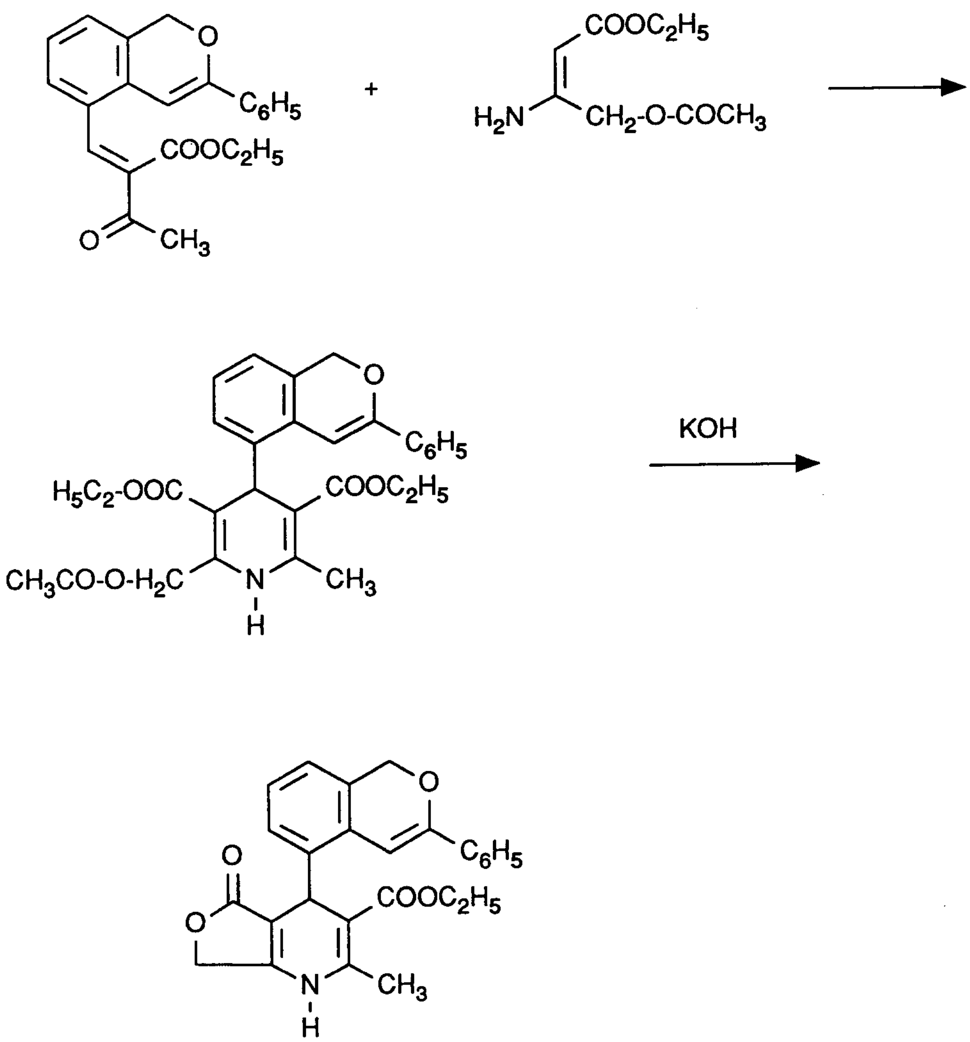

[E]

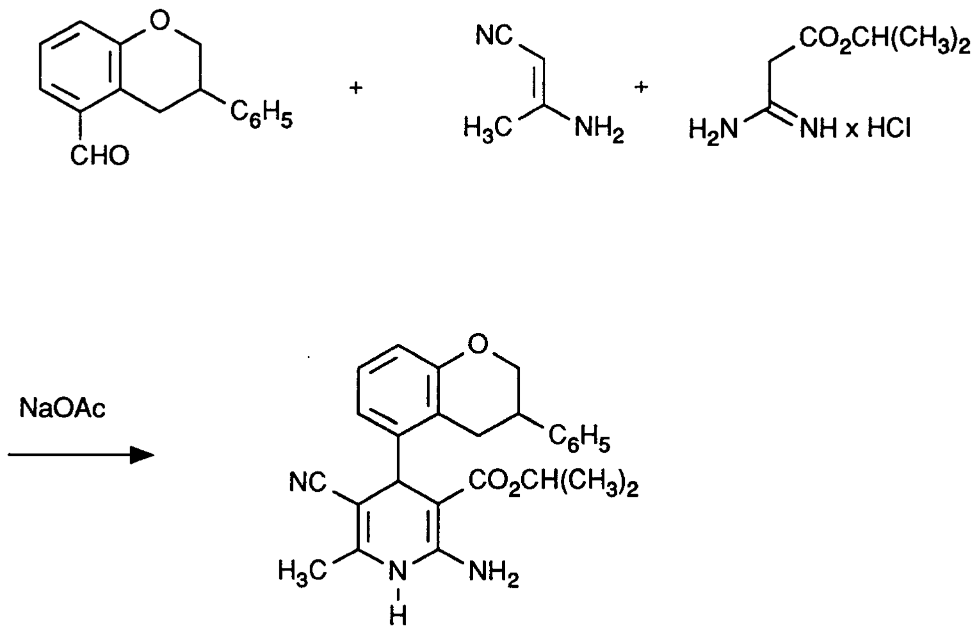


Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure oder halogenierte Kohlenwasserstoffe wie Methylenchlorid, Tetrachlorkohlenstoff oder Kohlenwasserstoffe wie Benzol oder Toluol. Ebenso ist es möglich, Gemische der genannten Lösemittel zu verwenden. Bevorzugt sind in Abhängigkeit von der jeweiligen Verfahrensvariante [A], [B], [C] und [D] Methanol, Isopropanol, Ethanol und n-Propanol, Acetonitril oder Tetrahydrofuran.

Die Reaktionstemperaturen können in einem größeren Bereich variiert werden. Im allgemeinen arbeitet man zwischen +10 °C und +150 °C, vorzugsweise zwischen +20 °C und +100 °C; insbesondere bei der Siedetemperatur des jeweiligen Lösemittels.

Die Umsetzung kann bei Normaldruck, aber auch bei erhöhtem oder erniedrigtem Druck (z.B. 0,5 bis 3 bar) durchgeführt werden. Im allgemeinen arbeitet man bei Normaldruck.

Geeignet als chirale Esterreste sind alle Ester enantiomerenreiner Alkohole wie beispielsweise 2-Butanol, 1-Phenylethanol, Milchsäure, Milchsäureester, Mandelsäure, Mandelsäureester, 2-Aminoalkohole, Zuckerderivate, Hydroxyaminosäurederivate und viele andere enantiomerenreine Alkohole mehr.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die Verbindungen der allgemeinen Formel (II), in welcher $R^5$ für den Rest der Formel

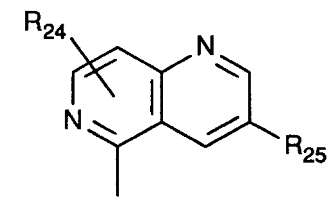

steht, sind bekannt oder können auf literaturanaloge Weise dargestellt werden.

Die übrigen Verbindungen der allgemeinen Formel (II) sind neu und können beispielsweise hergestellt werden, indem man

[I] im Fall, daß $R^5 = R^{5'}$ für einen der im folgenden aufgeführten Reste

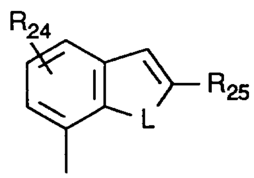
,
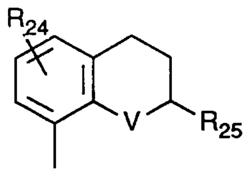
,
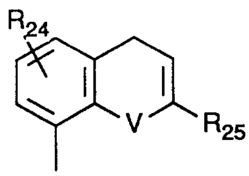
,

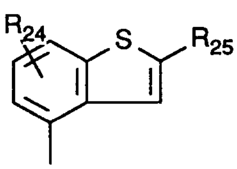
,
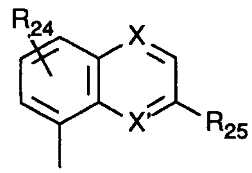
,
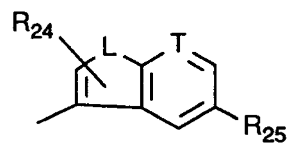
,

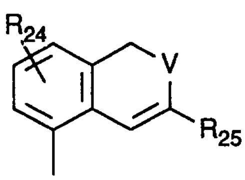
oder
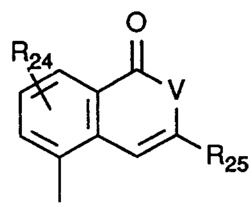

steht,

in welcher

$R^{24}$, $R^{25}$, L, T, V und X die oben angegebene Bedeutung haben,

Verbindungen der allgemeinen Formel (XV)

$R^{5'}$-$CO_2$-$R^{43}$ (XV)

in welcher

$R^{5'}$ für einen der oben aufgeführten Reste steht

und

$R^{43}$ für Wasserstoff oder $C_1$-$C_4$-Alkyl steht,

zunächst mit üblichen Reduktionsmitteln, wie beispielsweise Lithiumaluminiumhydrid oder über ein gemischtes Anhydrid mit Natriumborhydrid in die entsprechenden Alkohole der allgemeinen Formel (XVI)

$R^{5'}$-$CH_2$-OH (XVI)

in welcher

$R^{5'}$ die oben angegebene Bedeutung hat,

überführt,

und diese dann entweder nach Isolierung oder direkt in situ mit Oxidationsmitteln wie beispielsweise Manganoxid oxidiert,

oder

[II] im Fall, daß $R^5$ für den Rest der Formel

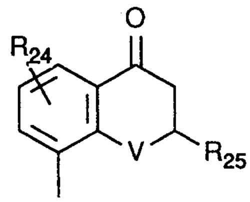

steht,
Verbindungen der allgemeinen Formel (XVII)

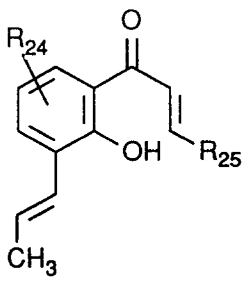

(XVII)

in welcher

$R^{24}$ und $R^{25}$ die oben angegebene Bedeutung haben,

zunächst durch Umsetzung mit Polyphosphorsäuren in Methylglycol zu den Verbindungen der allgemeinen Formel (XVIII)

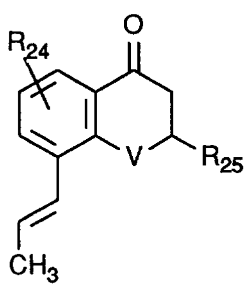

(XVIII)

in welcher

$R^{24}$ und $R^{25}$ die oben angegebene Bedeutung haben,

cyclisiert,

und anschließend in einem der oben aufgeführten Lösemitteln, vorzugsweise Methylenchlorid, eine Ozonolyse durchführt,

oder

[III] im Fall, daß $R^5$ für den Rest der Formel

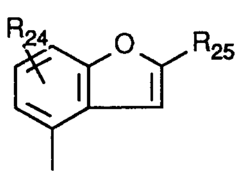

(a) oder

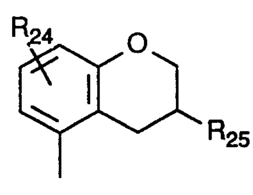

(b)

steht.

Verbindungen der allgemeinen Formel (XIX)

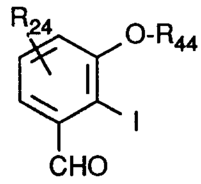

(XIX)

in welcher

$R^{24}$ die oben angegebene Bedeutung hat

und

$R^{44}$ für Wasserstoff oder für eine übliche, leicht abspaltbare Hydroxyschutzgruppe steht,

im Fall a) mit Verbindungen der allgemeinen Formel (XX)

$R^{25}$-≡-Cu (XX)

in welcher

$R^{25}$ die oben angegebene Bedeutung hat,

oder

im Fall b) zunächst mit Verbindungen der allgemeinen Formel (XXI)

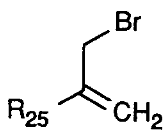

(XXI)

in welcher

$R^{25}$ die oben angegebene Bedeutung hat,

umsetzt und anschließend radikalisch z.B. mit Tributylzinnhydrid/AIBN cyclisiert und mit $MnO_2$ oxidiert, oder

[IV] im Fall, daß $R^5$ für den Rest der Formel

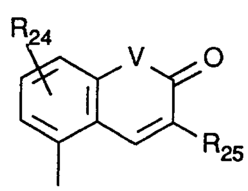

steht,

die entsprechenden Alkoholate der Verbindungen der allgemeinen Formel (XIX) zunächst im System Pd-$(P(C_6H_5)_3)_2Cl_2$ mit Verbindungen der allgemeinen Formel (XXII)

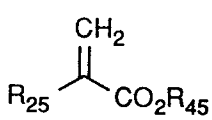

(XXII)

in welcher

$R^{25}$ die oben angegebene Bedeutung hat

und

$R^{45}$ für $C_1$-$C_4$-Alkyl steht,

zu den Verbindungen der allgemeinen Formel (XXIII)

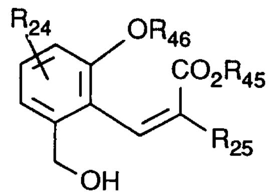

(XXIII)

in welcher

$R^{24}$ und $R^{45}$ die oben angegebene Bedeutung haben

und

$R^{46}$ für $C_1$-$C_4$-Alkyl steht,

umsetzt, anschließend wie oben beschrieben mit Oxidationsmitteln wie beispielsweise $MnO_2$ zu den entsprechenden Aldehyden oxidiert, und in einem letzten Schritt mit Säuren wie Bortribromid cyclisiert,

oder

[V] im Fall, daß $R^5$ für den Rest der Formel

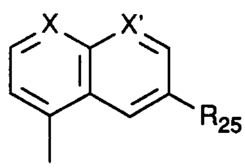

steht,

Verbindungen der allgemeinen Formel (XXIV)

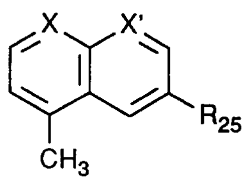

(XXIV)

in welcher

X, X' und $R^{25}$ die oben angegebene Bedeutung haben

in einem der oben aufgeführten Lösemittel, vorzugsweise Dioxan, mit Selendioxid oxidiert,

oder

[VI] im Fall, daß $R^5$ für den Rest der Formel

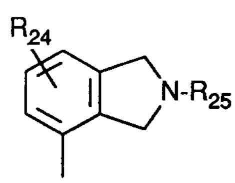

steht,

Verbindungen der allgemeinen Formel (XXV)

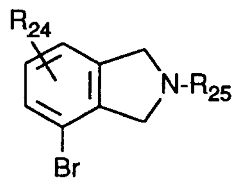

(XXV)

in welcher

$R^{24}$ und $R^{25}$ die oben angegebene Bedeutung haben,

unter Schutzgasatmosphäre, in einem der oben aufgeführten Losemittel, vorzugsweise Ether, mit Butyllithium metalliert und dann mit DMF umsetzt,

oder

[VII] im Fall, daß $R^5$ für den Rest der Formel

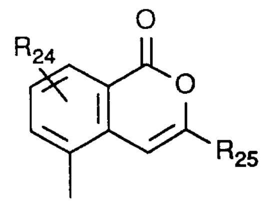

steht,

Verbindungen der allgemeinen Formel (XXVI)

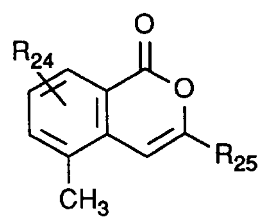

(XXVI)

in welcher

$R^{24}$ und $R^{25}$ die oben angegebene Bedeutung haben,

zunächst mit NBS bromiert, und in einem zweiten Schritt mit Kaliumacetat/Essigsäure, gefolgt von Schwefelsäure hydrolysiert,

und im Fall der N-oxide, ausgehend von den entsprechenden Verbindungen in welcher $R^5$ für einen stickstoffhaltigen Ring steht, zunächst mit MCPBA oxidiert und gegebenenfalls, wie oben beschrieben, in die Aldehyde überführt,

und gegebenenfalls auch die Verbindungen der allgemeinen Formel (I) durch die oben beschriebenen Arten der Oxidation oder Reduktion variiert.

Als Lösemittel für die einzelnen Schritte eignen sich die oben aufgeführten Lösemittel, vorzugsweise Tetrahydrofuran oder Methylenchlorid.

Die Reaktionen verlaufen im allgemeinen in einem Temperaturbereich von - 20 °C bis + 150 °C, vorzugsweise von 0 °C bis 100 °C und Normaldruck.

Gegebenenfalls werden einige Reaktionsschritte unter Schutzgasatmosphäre durchgeführt.

Die Verbindungen der allgemeinen Formel (XVI) sind größtenteils neu und können beispielsweise wie oben beschrieben hergestellt werden.

Die Verbindungen der allgemeinen Formel (XV) sind teilweise bekannt oder neu und können dann aber nach üblichen Methoden hergestellt werden.

Ebenso sind die Verbindungen der allgemeinen Formeln (XVII), (XVIII), (XX), (XXI), (XXII), (XXIII), (XXIV), (XXV) und (XXVI) teilweise bekannt oder neu und können dann aber in Analogie zu literaturbekannten Methoden hergestellt werden.

Die Acylverbindungen der allgemeinen Formel (III) und (X) sind bekannt oder können nach üblichen Methoden hergestellt werden.

Die Verbindungen der allgemeinen Formeln (V), (VI), (VIII), (VIIIa) und (XII) sind bekannt.

Die Ylidenverbindungen (IV), (VII) und (XI) sind neu, können aber nach üblichen Methoden hergestellt werden.

Die Verbindungen der allgemeinen Formel (IX) sind neu, können aber nach bekannten Methoden hergestellt werden, indem man beispielsweise Benzylidenverbindungen der allgemeinen Formel (IV) mit Chloracetessigsäureestern und Ammoniumverbindungen umsetzt.

Die Verbindungen der allgemeinen Formel (XIII) sind neu und können wie oben beschrieben hergestellt werden.

Die vorstehenden Herstellungsverfahren sind lediglich zur Verdeutlichung angegeben. Die Herstellung der Verbindungen der Formel (I) ist nicht auf diese Verfahren beschränkt, sondern jede Modifikation dieser Verfahren ist in gleicher Weise für die Herstellung der erfindungsgemäßen Verbindungen anwendbar.

Die erfindungsgemäßen Verbindungen zeigen ein nicht vorhersehbares, wertvolles pharmakologisches Wirkspektrum. Sie beeinflussen die Kontraktionskraft des Herzens und den Tonus der glatten Muskulatur, insbesondere zeigen sie Calcium-antagonistische und Calcium-agonistische Wirkungen.
Sie können deshalb in Arzneimitteln zur Beeinflussung des pathologisch veränderten Blutdrucks, als Koronartherapeutika und zur Behandlung der Herzinsuffizienz eingesetzt werden. Darüber hinaus können sie zur Behandlung von Herzrhythmusstörungen, zur Senkung des Blutzuckers, zur Abschwellung von Schleimhäuten und zur Beeinflussung des Salz- und Flüssigkeitshaushaltes verwendet werden.

Die Herz- und Gefäßwirkungen wurden am isoliert perfundierten Herzen des Meerschweinchens gefunden. Dazu werden die Herzen von 250 bis 350 g schweren Meerschweinchen verwendet. Die Tiere werden mit einem Schlag auf den Kopf getötet, der Thorax geöffnet, und in die freipräparierte Aorta eine Metallkanüle eingebunden. Das Herz wird mit den Lungen aus dem Thorax herausgetrennt und über eine Aortenkanüle an die Perfusionsapparatur bei laufender Perfusion angeschlossen. Die Lungen werden an den Lungenwurzeln abgetrennt. als Perfusionsmedium dient eine Krebs-Henseleit-Lösung (118,5 mmol/l NaCl, 4,75 mmol/l KCl, 1,19 mmol/l $KH_2PO_4$, 1,19 mmol/l $MgSO_4$, 25 mmol/l $NaHCO_3$, 0,013 mmol/l $Na_2EDTA$), deren $CaCl_2$-Gehalt 1,2 mmol/l beträgt. Als energielieferndes Substrat werden 10 mmol/l Glucose zugesetzt. vor der Perfusion wird die Lösung partikelfrei filtriert. Die Lösung wird mit Carbogen (95% $O_2$, 5% $CO_2$) zur Aufrechterhaltung des pH-Wertes 7,4 begast. Die Herzen werden mit konstantem Fluß (10 ml/min) bei 32°C mittels einer Rollenquetschpumpe perfundiert.

Zur Messung der Herzfünktion wird ein flüssigkeitsgefüllter Latexballon, der über eine Flüssigkeitssäule mit einem Druckaufnehmer verbunden ist, durch den linken Vorhof in den linken Ventrikel eingeführt, und die isovolumetrischen Kontraktionen auf einem Schnellschreiber registriert. Der Perfusionsdruck wird mittels eines Druckaufnehmers, der vor dem Herzen mit dem Perfusionssystem in Verbindung steht, registiert. Unter diesen Bedingungen zeigt eine Senkung des Perfusionsdrucks eine Koronardilatation, eine Zu- bzw. Abnahme der linksventrikulären Kontraktionsamplitude eine Senkung bzw. einen Anstieg der Herzkontraktilität an. Die erfindungsgemäßen Verbindungen werden in geeigneten Verdünnungen in das Perfusionssystem kurz vor dem isolierten Herzen perfundiert.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, uni den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu

welchen die Verabreichung erfolgt. So kann es in einigen Fällen ausreichend sein, mit weniger als der vorgenannten Mindestmenge auszukommen, wahrend in anderen Fällen die genannte obere Grenze überschritten werden muß. Im Falle der Applikation größerer Mengen kann es empfehlenswert sein, diese in mehreren Einzelgaben über den Tag zu verteilen.

Ausgangsverbindungen

Beispiel I

8-Formyl-2-phenyl-imidazo[1.2-a]-pyridin

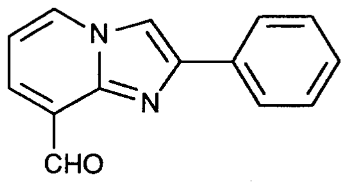

Zu 8,7 ml (110 mmol ) Oxalylchlorid in 220 ml Methylenchlorid wird bei - 60 °C unter inerten Bedingungen eine Mischung von 14,8 ml DMSO und 45 ml Methylenchlorid getropft. Anschließend werden unter Rühren bei -60 °C 19,5 g (87 mmol) 2-Phenyl-8-hydroxymethyl-imidazo[1,2-a]pyridin in 90 ml Methylenchlorid und 45 ml DMSO zugetropft, 15 min bei -60 °C und 1 h bei -10 °C gerührt. Man gibt nun 60,9 ml Triethylamin hinzu, rührt 5 min. bei -10 °C und läßt dann auf RT kommen. Zur Aufarbeitung wird mit Wasser versetzt, abgetrennt und die wäßrige Phase noch 2 mal mit Methylenchlorid extrahiert. Die vereinigten organischen Phasen werden mit gesättigter NaCl-Lösung gewaschen, über $Na_2SO_4$ getrocknet und am Rotationsverdampfer eingeengt. Durch Verrühren mit Ether erhält man 16,4 g Kristalle (= 73,8% d.Th.), die zur Abtrennung chlorierter Nebenprodukte auf Kieselgel chromatographiert werden.
Fp.: 96-99 °C
MS : 222(63%), 194(100%), 102(21%), 97(15%)

Beispiel II

2-Cinnamoyl-6-propenylphenol

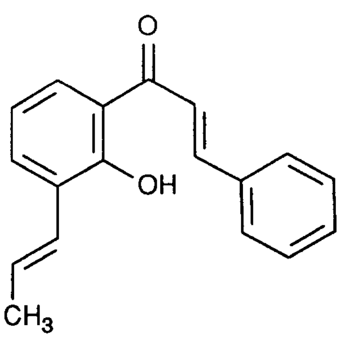

3,5 g (20 mmol) 2-Acetyl-6-(1-propenyl)phenol und 2,1 g (20 mmol) Benzaldehyd werden in 20 ml Ethanol gelöst, mit 4 ml konzentrierter NaOH versetzt, über Nacht gerührt, der rote Kristallbrei mit Methanol verdünnt, mit konz. HCl angesäuert und nach Kühlung abgesaugt. Man erhält orangerote Kristalle vom Fp. 95-98 °C

Beispiel III

8-(1-Propenyl)flavanon

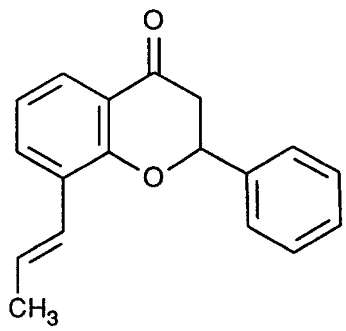

0,5 g der Verbindung aus Beispiel II werden in 20 ml Methylglycol gelöst und mit 2 ml Polyphosphorsäure 8 h am Rückfluß gekocht. Anschließend wird in Wasser gefällt, mit $CH_2Cl_2$ extrahiert und vom nicht umgesetzten Beispiel I chromatographisch getrennt. Man erhält 70% der Titelverbindung als farbloses Öl, das später wachsartig erstarrt.
Fp.: < 50 °C

Beispiel IV

Flavanon-8-carboxaldehyd

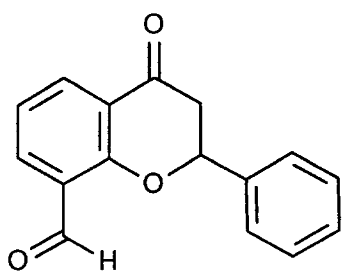

3,4 g (10 mmol) der Verbindung aus Beispiel III werden in 50 ml $CH_2Cl_2$ bei - 78 °C ozonolysiert. Nach üblicher Aufarbeitung erhält man 1,8 g der Titelverbindung.
Fp.: 112-113 °C

Beispiel V

2-[(2,2-Dimethoxy-1-phenylethyl)thio]benzoesäuremethylester

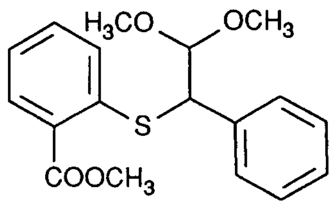

Je 10 ml 2-Mercaptobenzoesäuremethylester und 2-Brom-1,1-dimethoxy-2-phenylethan werden in 30 ml Methanol unter Zusatz von 10 ml NaOMe über Nacht refluxiert. Nach Aufarbeitung und chromatographischer Reinigung erhält man 70% zähes Öl.

Beispiel VI

2-Phenylbenzo[b]thiophen-7-carbonsäuremethylester

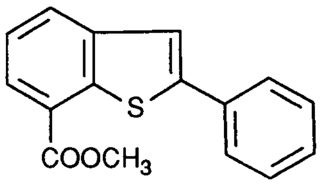

10 mmol der Verbindung aus Beispiel V werden in 30 g Polyphosphorsäure gelöst und nach 20 min auf Wasser gegossen und gereinigt.
Ausbeute: 85%
Fp.: 137-140 °C

Beispiel VII

7-Hydroxymethyl-2-phenylbenzo[b]thiophen

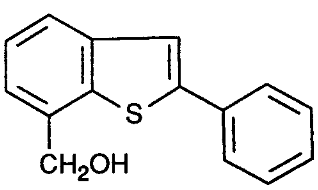

20 g der Verbindung aus Beispiel VI werden in 75 ml THF gelöst und zu 40 mmol $LiAlH_4$ in 50 ml THF zugetropft. Nach üblicher Aufarbeitung erhält man 85% der Titelverbindung.
Fp.: 125-126 °C

Beispiel VIII

2-Phenyl-7-benzo[b]thiophencarboxaldehyd

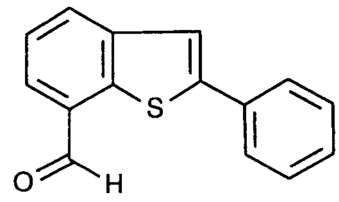

20 mmol der Verbindung aus Beispiel VII werden mit 100 g $MnO_2$ in 800 ml $CH_2Cl_2$, über Nacht gekocht, über Kieselgur abgesaugt und eingeengt.
Ausbeute: 90%
Fp.: 92-94 °C

Beispiel IX

8-Hydroxymethyl-3,4-dihydro-1(2H)-thiobenzopyran

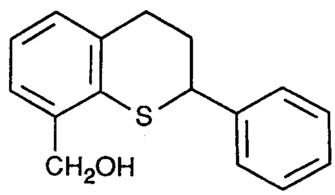

20,3 g 8-Hydroxymethyl-2-phenyl-4H-1-benzothiopyran (80 mmol) werden in 400 ml MeOH und 5 ml Eisessig gelöst und mit 5 g $PtO_2$ bei 3 bar hydriert. Man erhält die Titelverbindung als Öl, das gleich weiter umgesetzt wird.

Beispiel X

(±) 2-Phenyl-3,4-dihydro-1(2H)-benzothiopyran-8-carboxaldehyd

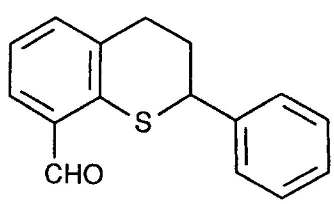

0,1 mol der Verbindung aus Beispiel IX werden in 500 ml $CH_2Cl_2$ gelöst und unter Zusatz von 75 g $MnO_2$ über Nacht refluxiert, abgesaugt, eingeengt und mit EtOH kristallisiert.
Fp.: 62-63 °C

Beispiel XI

2-Phenyl-benzo[b]furan-7-carboxaldehyd

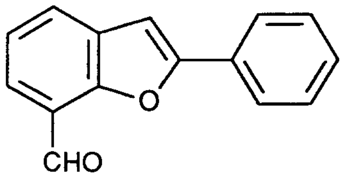

2 g 7-Hydroxymethyl-2-phenylbenzo[b]furan (hergestellt nach Beispiel 1 aus EP 306 226) werden in 100 ml Dichlormethan mit 12 g Mangandioxid versetzt und 1 Stunde zum Rückfluß erhitzt. Es wird abgekühlt, über ein Filtrierhilfsmittel abgesaugt und eingeengt. Man erhält 1,9 g eines farblosen Feststoffes vom Schmelzpunkt 60-61 °C.

Beispiel XII

S-(2,3-Dimethylphenyl)-N,N,N',N'-tetramethylphosphorodiamidothioat

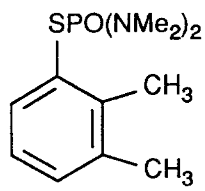

Die Herstellung erfolgt in Analogie zu einem bekannten Verfahren [M. Watanabe, M. Date, K. Kawanishi, R. Akiyoshi, S. Furukawa, J. Heterocyclic Chem. 1991, 28, 173] aus 138 g (1 mol) 2,3-Dimethylthiophenol.
$Kp_{0.3}$ = 150 °C

Beispiel XIII

S-(3-Methyl-2-phenacyl)phenyl-N,N,N',N'-tetramethylphosphorodiamidothioat

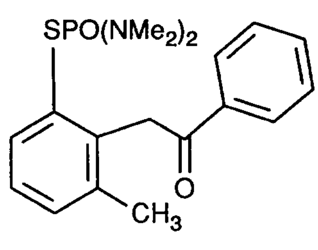

Zu 20 g (73 mmol) der Verbindung aus Beispiel XII in 300 ml abs. THF werden bei -70 °C 125 ml einer 1,4 N Lösung von sec-BuLi in Cyclohexan (175 mmol) getropft. Man rührt anschließend 1 h bei -70 °C und tropft 18 ml Benzoesäuremethylester hinzu. Man läßt auf RT kommen, gibt den Ansatz in eine gesättigte Ammoniumchloridlösung, verdampft im Vakuum den THF-Anteil, extrahiert mit Essigester, trocknet, verdampft und chromatographiert auf Kieselgel (Toluol -> Toluol/-Essigester 1:1)
Ausbeute: 10,7 g (39% d.Th.)
Als Nebenprodukt erhält man in wechselnden Mengen sec.-Butyl-(2,3-dimethylphenyl)thioether.

Beispiel XIV

4-Methyl-2-phenylbenzo[b]thiophen

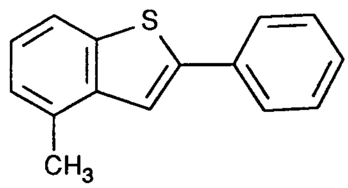

11 g (29,2 mmol) der Verbindung aus Beispiel XII werden in 50 ml Ameisensäure 1 h unter Rückfluß gekocht. Der Rückstand wird mit $NaHCO_3$-Lösung neutralisiert, mit Essigester extrahiert und auf Kieselgel chromatographiert. Durch Destillation im HV läßt sich das Nebenprodukt aus Beispiel XIII auf dieser Stufe besonders leicht entfernen, wobei die Titelverbindung als Feststoff zurückbleibt.
Ausbeute: 5,0 g (76,3%)
Mp.: 83 °C

Beispiel XV

4-Acetoxymethyl-3-brom-2-phenylbenzo[b]thiophen

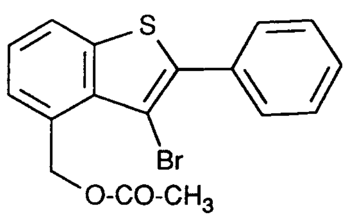

10 g (44,6 mmol) der Verbindung aus Beispiel XIV werden in 200 ml $CCl_4$ unter Belichtung gekocht, wahrend man nach und nach neben einer Spatelspitze AIBN insgesamt 24,1 g N-Bromsuccinimid zugibt. Nach etwa 1 h wird abfiltriert, mit $CCl_4$ nachgewaschen und die Lösung im Vakuum eingedampft. Der Rückstand (22,2 g) wird in 550 ml Eisessig mit 22 g Kaliumacetat 3 h gekocht. Nach Abdestillieren des Eisessiges im Vakuum wird mit Wasser geschüttelt und mit Essigester extrahiert. Nach Chromatographie erhält man als Hauptprodukt die Titelverbindung.
Ausbeute: 4 g (25%)

Beispiel XVI

3-Brom-4-hydroxymethyl-2-phenylbenzo[b]thiophen

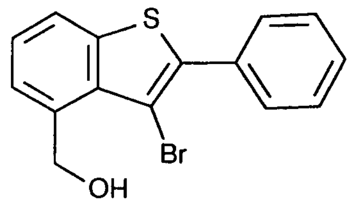

Die Verseifung der Verbindung aus Beispiel XV (1 g) in 100 ml Ethanol mit 20 ml 1 N NaOH bei RT ergibt nach 2 h 0,9 g der Titelverbindung.

Beispiel XVII

4-Hydroxymethyl-2-phenyl-benzo[b]thiophen

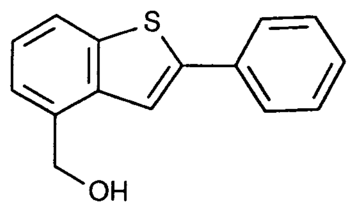

8 g (25,2 mmol) der Verbindung aus Beispiel XVI werden unter Argon in 160 ml Methanol bei 0°C mit 8,9 g (50,4 mmol) $PdCl_2$ versetzt. Anschließend werden 9,44 g (250 mmol) $NaBH_4$ in Kleinen Portionen zugegeben. Dabei kann es zur Entzündung des entstehenden Wasserstoffs kommen. Die Reaktion verläuft stark exotherm; ihr Umsatz wird mittels HPLC verfolgt. Nach Beendigung wird der Ansatz in 1 N HCl gegeben, mit Essigester extrahiert und die einrotierte organische Phase auf Kieselgel chromatographiert.
Ausbeute: 4,2 g (69%)

Beispiel XVIII

2-Phenyl-4-benzo[b]thiophen-carboxaldehyd

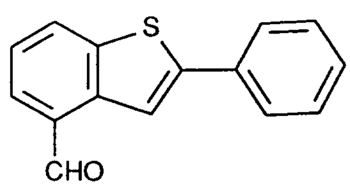

1 g (4,2 mmol) der Verbindung aus Beispiel XVII werden mit der 5-fachen Menge $MnO_2$ in $CHCl_3$ 2 h unter Rückfluß gekocht.
Ausbeute: 83%

Beispiel XIX

2-Phenyl-4-benzo[b]furan-carboxaldehyd

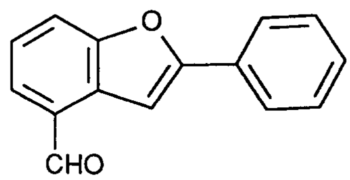

14,9 g (60 mmol) 3-Hydroxy-2-iod-benzaldehyd werden mit 10,4 g (63,2 mmol) Kupferphenylacetylid in 300 ml Pyridin 3 h bei 120 °C gerührt. Man engt ein, chromatographiert auf einer Kieselgelsäule und erhält 12,4 g (93%) der Titelverbindung.
Mp.: 103 °C
$R_f$ (Kieselgel, Toluol): 0,38.

Beispiel XX

2-Iod-3-[(2-phenyl-2-propenyl)oxy]benzaldehyd

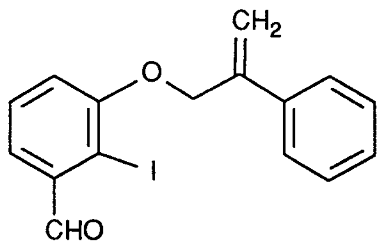

Man löst 6,72 g (0,28 mol) Natriumhydrid unter Argon in 660 ml Ether und 120 ml DMF und gibt langsam 64,1 g (0,26 mol) 3-Hydroxy-2-iodbenzaldehyd in 65 ml Ether hinzu. Nach 15 minütigem Rühren bei RT werden 61 g (0,28 mol) 3-Brom-2-phenyl-1-propen in 60 ml Ether gelöst hinzugetropft und über Nacht gerührt.
Ausbeute: 55,5 g (59%)
Mp.: 88 °C

Beispiel XXI

5-Hydroxymethyl-3-phenyl-1(2H)-dihydrobenzopyran

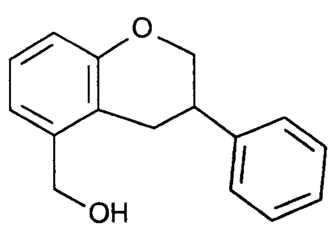

55,5 g (152 mmol) der Verbindung aus Beispiel XX in 3 l Benzol, 150 ml (553 mmol) Tributylstannan und 200 mg AIBN werden unter Argon 2 h zum Rückfluß erhitzt. Man schüttelt mit Wasser und verdünnter

Salzsäure, extrahiert mit Essigester und rotiert ein. Nach Stehen über Nacht liegt im wesentlichen der Alkohol der Titelverbindung vor, der durch Zugabe von Pentan auskristallisiert und von zinnorganischen Verbindungen abgetrennt werden kann.

Beispiel XXII

3-Phenyl-1(2H)-dihydrobenzo-pyran-5-carboxyaldehyd

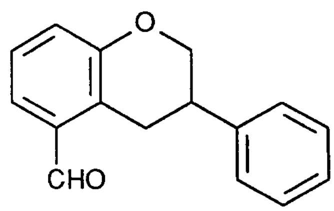

36 g des Alkohols der Verbindung aus Beispiel XXI werden mit 120 g $MnO_2$ in Chloroform 1 h gekocht, über Kieselgur abgesaugt und einrotiert. Man erhält 27 g (75%) der Titelverbindung als Öl.

Beispiel XXIII

Ethyl-3-(3-hydroxymethyl-1-methoxy-2-phenyl)-2-phenyl-2-propenoat

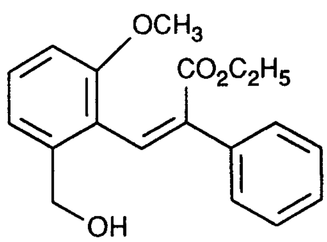

13,3 g (50,4 mmol) 3-Hydroxymethyl-2-iod-1-methoxybenzol, 8,87 g (50,4 mmol) Atropasäureethylester und 7,05 ml Triethylamin werden in 600 ml DMF gelöst und mit Argon gesättigt. Anschließend gibt man 350 mg Palladium-bis(triphenylphosphin)-dichlorid hinzu und rührt über Nacht bei 140 °C. Die schwarze Lösung wird im Vakuum eingedampft und über Kieselgel chromatographiert. Man erhält 7,1 g (45%) der Titelverbindung.

Beispiel XXIV

Ethyl-3-(3-formyl-1-methoxy-2-phenyl)-2-phenyl-2-propenoat

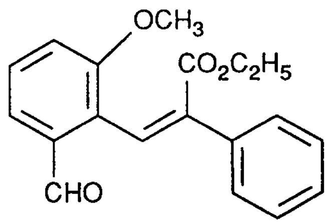

16,9 g (54 mmol) der Verbindung aus Beispiel XXIII in 350 ml Chloroform werden mit 85 g Mangandioxid 3 h gekocht. Man filtriert durch Kieselgur und verdampft im Vakuum.
Ausbeute: 16,7 g (99%)

Beispiel XXV

3-Phenyl-5-cumarin-carboxaldehyd

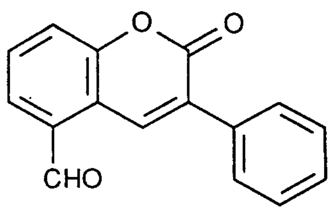

22,2 g (71,5 mmol) der Verbindung aus Beispiel XXIV in 500 ml Methylenchlorid werden bei 0 °C mit 110 ml einer 1 M Lösung von $BBr_3$ in Methylenchlorid versetzt. Man rührt erst 2 h bei 0 °C, dann 2 h bei Raumtemperatur. Der Ansatz wird in einem Überschuß wässriger $K_2HPO_4$-Lösung 1,5 h unter Rühren hydrolysiert. Aus der organischen Phase erhält man ein Gemisch, aus dem sich durch Chromatographie 2,5 g (14%) der Titelverbindung isolieren lassen. Die Titelverbindung zeigt auf dem DC mit Fluoreszenzindikator bei 366 nm ein charakteristisches Leuchten.
MS (EI): 250(100%), 221(18%), 165(15%)

Beispiel XXVI

6-Phenyl-4-chinolin-carboxaldehyd

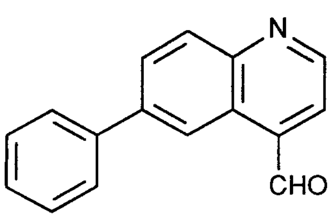

8,5 g (38,8 mmol) 4-Methyl-6-phenyl-chinolin werden in 85 ml Dioxan mit 1,4 ml Wasser und 17 g Selendioxid versetzt und 1 Stunde gekocht. Es wird vom Selen abgesaugt, mit Methanol gewaschen und eingeengt. Das erhaltene Gemisch wird durch Flash-Chromatographie getrennt, die sauberen Fraktionen

werden eingeengt, der Rückstand wird mit Ether verrührt, abgesaugt und mit Ether gewaschen. Man erhält 3,2 g farblose Kristalle vom Schmelzpunkt 115°C.

Beispiel XXVII

2-Phenyl-isoindolin-4-carboxaldehyd

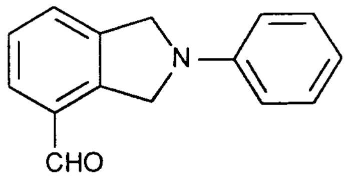

In eine trockene Apparatur werden unter Argon 2,3 ml (3,7 mmol) 1,6 molaren Butyllithiumlösung in Hexan zu 14 ml trockenem Ether gegeben. Bei -70°C wird dann eine Lösung von 930 mg (3,4 mmol) 4-Brom-2-phenylisoindolin in 10 ml trockenem Ether zugetropft. Es wird 30 Minuten bei -70°C gerührt und mit 0,3 ml trockenem DMF in 0,5 ml Ether versetzt. Es wird 3 Stunden bei 70°C gerührt, auf -5°C erwärmt und tropfenweise mit 13,6 ml 1 N Salzsäure versetzt. Es wird mit 50 ml Ether versetzt und getrennt. Die wäßrige Phase wird mit Ether ausgeschüttelt, die vereinigten Etherphasen 1 x mit Wasser gewaschen, getrocknet und eingeengt. Man erhält 600 mg gelbliche Kristalle vom Schmelzpunkt 120-122°C.

Beispiel XXVIII

3-Phenyl-5-chinoxalincarbonsäure

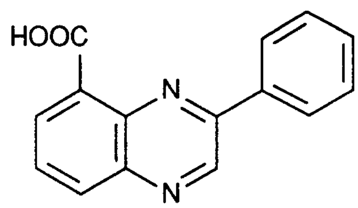

30 g (164,8 mmol) 2-Amino-3-nitrobenzoesäure werden in 900 ml Ethanol suspendiert, mit 6 g 5%iger Pd-Kohle versetzt und ca. 2 h bei 3 bar bis Ende der Wasserstoffaufnahme in der Parr-Apparatur hydriert. Man filtriert den Katalysator über Kieselgur ab und gibt zur Lösung 12,8 g (83 mmol) Phenylglyoxalhydrat. Anschließend wird 2,5 h bei RT unter Stickstoff gerührt, der entstandene Niederschlag abgesaugt, mit Ethanol nachgewaschen und getrocknet.
Ausbeute: 89% (bezogen auf Phenylglyoxal)
MS (EI): 250 ($M^+$, 6%), 206 ($M-CO_2$, 100%), 103(12%), 76(15%)

Beispiel XXIX

5-Hydroxymethyl-3-phenylchinoxalin

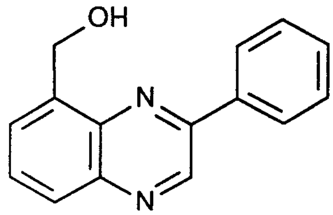

14,5 g (58 mmol) der Verbindung aus Beispiel XXVIII in 150 ml THF werden bei 0 °C unter Stickstoff vorgelegt. Man tropft nun unter Rühren eine Lösung von 11 g (290 mmol) $LiAlH_4$ in 60 ml THF hinzu und rührt nach DC-Kontrolle noch maximal 1 h bei RT nach. Anschließend wird durch Zutropfen von Wasser hydrolysiert und mit verdünnter Salzsäure auf pH 5 eingestellt. Nach Extraktion mit Essigester und Trocknung der organischen Phase über $Na_2SO_4$ erhält man die Titelverbindung in 58%iger Ausbeute, die direkt weiter umgesetzt wird.
MS (EI): 236 ($M^+$, 100%), 207(41%)

Beispiel XXX

3-Phenyl-chinoxalin-5-carboxaldehyd

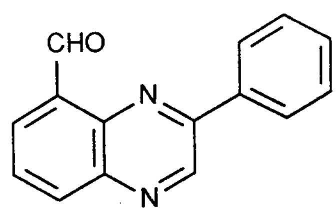

16,3 g (69 mmol) der Verbindung aus Beispiel XXIX werden in 350 ml Chloroform gelöst, mit 40 g $MnO_2$ versetzt und 2 h unter Rückfluß gekocht. Anschließend werden nochmals 16 g $MnO_2$ zugegeben und über Nacht gekocht. Man filtriert vom $MnO_2$ ab, engt das Filtrat am Rotationsverdampfer ein und erhält nach Chromatographie über Kieselgel (Laufmittel Toluol : Essigester 4:1) die Titelverbindung in 66%iger Ausbeute.
Mp.: 149 °C

Beispiel XXXI

5-Phenyl-3-thieno[2,3-b]pyridincarbonsäuremethylester

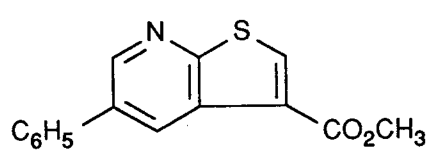

54,9 g (0,32 mol) 2-Nitro-4-thiophencarbonsäuremethylester werden in 1373 ml Methanol gelöst und mit 1373 ml konz. Salzsäure, gefolgt von 109,8 g Zinngranalien versetzt. Man rührt mit einem KPG-Rührer 1 1/2

- 3 h und kontrolliert den Umsatz mittels DC (Probe vorher neutralisieren). Die Reaktionszeit hängt von der Rührgeschwindigkeit ab. Ist kein Edukt mehr in Ansatz vorhanden, wird von den verbliebenen Zinngranalien abfiltriert. Dann gibt man eine Lösung von 54,9 g (0,37 mol) 2-Phenylmalondialdehyd in 500 ml Methanol hinzu und rührt 1,5 h bei Raumtemperatur. Die intermediär entstehende Schiff'sche Base ist auf dem DC als gelber Fleck erkennbar. Anschließend wird 2,5 h unter Rückfluß gekocht, die abgekühlte Lösung mit Methylenchlorid extrahiert, getrocknet, einrotiert und auf Kieselgel chromatographiert.
Ausbeute: 39 g (45,3%)
$R_f$ (Kieselgel, Toluol/Essigester 1:1) = 0,59

Beispiel XXXII und Beispiel XXXIII

3-Hydroxymethyl-5-phenylthieno[2,3-b]pyridin (XXXII)

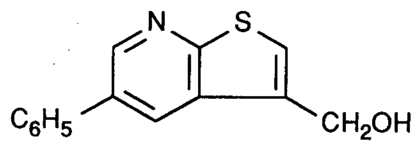

5-Phenyl-3-thieno[2,3-b]pyridincarboxaldehyd (XXXIII)

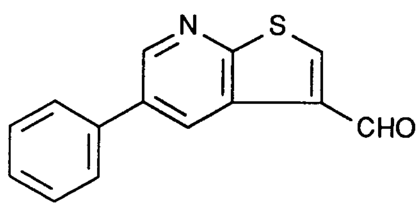

39 g (0,145 mol) der Verbindung aus Beispiel XXXI werden in 350 ml THF gelöst und unter Argon bei 0 °C unter Rühren tropfenweise mit 9,1 g Lithiumaluminiumhydrid in 300 ml THF versetzt. Man läßt auf RT kommen und rührt noch 1 h. Anschließend wird unter Eiskühlung vorsichtig mit Wasser hydrolysiert, mit HCl auf pH 3 - 4 angesäuert und 3 mal mit Essigester extrahiert Man erhält nach Verdampfen des Lösermittels im Vakuum 28,3 g der Verbindung aus Beispiel XXXII, die in 600 ml Chloroform gelöst, mit 280 g Mangandioxid versetzt und 5 h unter Rückfluß gekocht werden. Man filtriert über Kieselgur ab, rotiert ein und chroamtographiert über eine kurze Kieselgelsäule. Die Verbindung aus Beispiel XXXIII wird in einer Ausbeute von 20,0g (60%) erhalten.
$R_F$ (Kieselgel, Toluol/Essigester 1:1) = 0,69
MS (EI): 239(100%), 210(15%), 152(10%), 139(15%)

Beispiel XXXIV

5-Dibrommethyl-3-phenylisocumarin

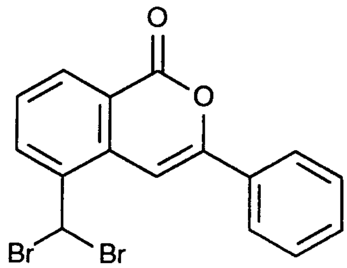

29,2 g (124 mmol) 5-Methyl-3-phenyl-isocumarin werden in 1 l Tetrachlorkohlenstoff mit 55 g N-Bromsuccinimid und einer katalytischen Menge AIBN 10 h gekocht, wobei nach 3 und 6 h jeweils nochmals 22 g NBS und etwas AIBN zugegeben werden. Nach Abkühlen wird der Ansatz mit 60 g Kieselgel versetzt, das Lösemittel im Vakuum verdampft und auf einer Kieselgelsäule (Toluol) chromatographiert.
Ausbeute: 47 g (96%)
Fp.: 197 °C
$R_F$ (Dibromid, Monobromid, Edukt): 0,24, 0,28, 0,34

Beispiel XXXV

3-Phenyl-5-isocumarincarboxaldehyd

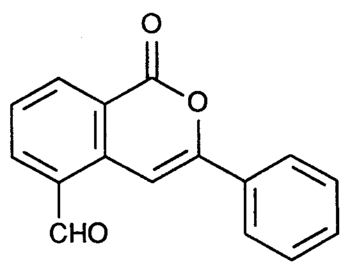

23,5 g (60 mmol) der Verbindung aus Beispiel XXXIV werden in 250 ml Eisessig mit 23,5 g Kaliumacetat 3 h gekocht. Anschließend wird der Ansatz auf Wasser gegeben und mit $NaHCO_3$ neutralisiert. Man schüttelt dreimal mit Methylenchlorid aus, trocknet und engt ein. Das erhaltene Produkt wird in Dioxan gelöst und nach Zugabe von 2N $H_2SO_4$ 2 h bei 50 °C gerührt. Man schüttelt mit Methylenchlorid aus und chromatographiert die getrocknete und eingeengte organische Phase.
Ausbeute: 11,5 g (77%)
Fp.: 154 °C
MS (DCI): 251(100%, M + H), 105(80%)

Beispiel XXXVI

3-Phenyl-5-isocoumarincarboxaldehyddimethylacetal

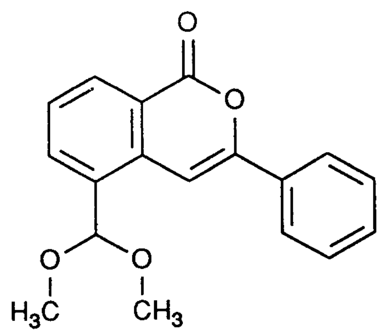

8 g (32 mmol) der Verbindung aus Beispiel XXXV in 30 ml Methanol werden mit 10 ml konz. HCl versetzt und 3 h bei RT gerührt. Man engt auf ein Drittel des Volumens ein und saugt die ausgefallenen Kristalle ab.
Ausbeute. 8,3 g (88%)
$R_f$ (Toluol/Essigester = 4:1): 0,53
MS (EI): 296 ($M^+$, 68%), 265(100%)

Beispiel XXXVII und XXXVIII

3-Phenyl-5-isochromencarboxaldehyddimethylacetal

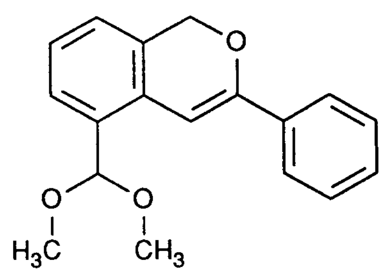

(XXXVII)

5-Hydroxymethyl-1-methoxy-3-phenylisochromen

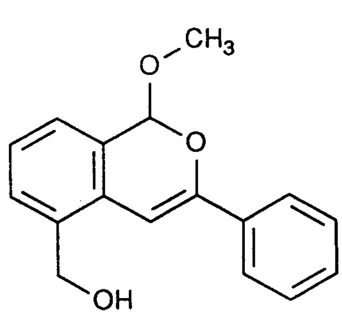

(XXXVIII)

Zu 8 g (27 mmol) der Verbindung aus Beispiel XXXVI werden in 400 ml Ether bei 0 °C langsam eine Lösung von 2,5 g $LiAlH_4$ in 400 ml Ether getropft. Man rührt 30 min bei RT und hydrolysiert vorsichtig mit Wasser. Die abgetrennte Etherphase wird im Vakuum verdampft und chromatographiert.

$R_f$ (Toluol/Essigester 4:1) = 0,25
Beispiel XXXVIII:
MS (DCI, $NH_3$): 269 ($M^+$ + 1, 100%), 251(45%), 237(20%), 105(30%)

Beispiel XXXIX

3-Phenyl-5-isochromencarboxaldehyd

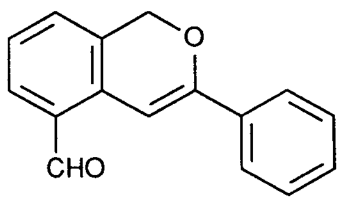

10 g der Mischung aus den Beispielen XXXVII und XXXVIII werden in 50 ml Dioxan gelöst, mit 60 ml 1 N HCl versetzt und 10 min gut gerührt.
$R_f$ (Toluol/Essigester 4:1): 0,4 (gelbe Fluoreszenz)
Mp.: 141 °C
MS (EI): 236 ($M^+$, 68%), 207(43%), 179(18%), 105(100%), 77(54%)

Herstellungsbeispiele

Beispiel 1

5-Cyano-1,4-dihydro-2,6-dimethyl-4-(2-phenyl-4H-1-benzothiopyran-8-yl)-3-pyridincarbonsäureethylester

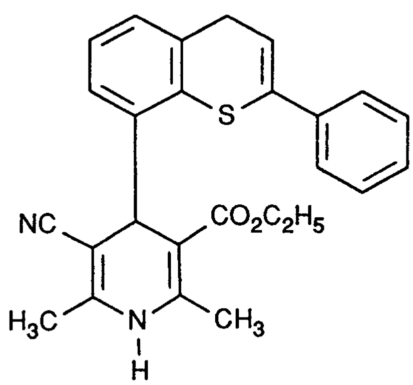

3 mmol 5-Cyano-1,4-dihydro-2,6-dimethyl-4-(4-oxo-2-phenyl-4H-1-benzothiopyran-8-yl)-3-pyridincarbonsäureethylester (DE 33 11 005) und 15 mmol $NaBH_4$ werden in 10 ml t-Butanol vorgelegt und bei 60 °C mit 1,8 ml MeOH versetzt und 8 h bei 65 °C gehalten. Nach üblicher Aufarbeitung erhält man die Titelverbindung.
Fp.: 206-207 °C

Beispiel 2

5-Cyano-1,4-dihydro-2,6-dimethyl-4-(3-phenyl-1,8-naphthyridin-5-yl)pyridin-3-carbonsäureethylester

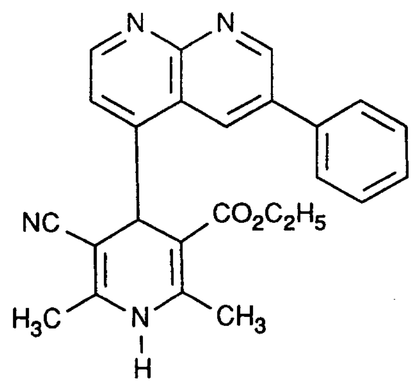

2 g (8,54 mmol) 3-Phenyl-1,8-naphthyridin-5-carboxaldehyd, 0,7 g (8,54 mmol) 3-Aminocrotonnitril und 1,08 ml Ethylacetoacetat (8,54 mmol) werden in 16 ml Ethanol 16 h in einer $N_2$-Atmosphäre gekocht. Nach Chromatographie auf Kieselgel und Elution mit Essigester erhält man 0,38 g (10,8% d.Th.) der Titelverbindung vom Schmp. 265 °C.

In Analogie zu der Vorschrift des Beispiels 1 werden die in der Tabelle 1 aufgeführten Beispiele hergestellt:

Tabelle 1:

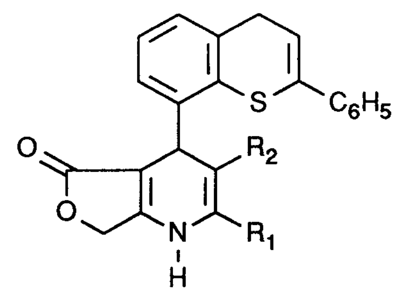

| Bsp.-Nr. | $R^1$ | $R^2$ | F°C |
|---|---|---|---|
| 3 | $-CH_3$ | $-CO_2C_2H_5$ | amorph |
| 4 | $-CH_3$ | $-CO_2CH_3$ | 260 (Zers.) |
| 5 | $-CH_3$ | $-CO_2C_2H_5$ | |

Analog zur Vorschrift des Beispiels 2 werden die in den Tabellen 2 - 19 aufgeführten Beispiele hergestellt:

Tabelle 2:

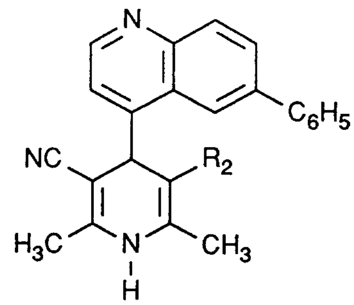

| Bsp.-Nr. | $R^2$ | F°C |
|---|---|---|
| 6 | $-CO_2-CH(CH_3)_2$ | 189-191 |
| 7 | $-CO_2C_2H_5$ | 209 |

Tabelle 3:

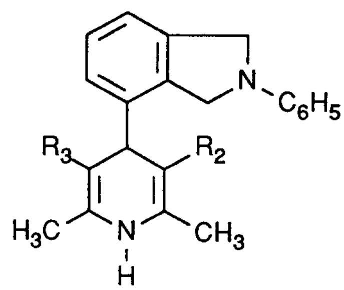

| Bsp.-Nr. | $R^2$ | $R^3$ | F°C |
|---|---|---|---|
| 8 | $-CO_2-CH(CH_3)_2$ | -CN | 194-197 |
| 9 | $-CO_2CH_3$ | $-NO_2$ | 208 |
| 10 | $-CO_2(CH_2)_2-C_2H_5$ | -CN | 221 |
| 11 | $-CO_2-CH(CH_3)_2$ | $-NO_2$ | 135 |

Tabelle 4:

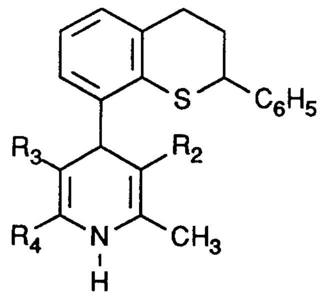

| Bsp.-Nr. | $R^2$ | $R^3$ | $R^4$ | F°C |
|---|---|---|---|---|
| 12 | $-CO_2C_2H_5$ | 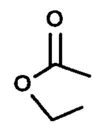 | | 244-248 |
| 13 | $-CO_2CH_3$ | 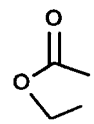 | | 255 |
| 14 | $-CO_2C_2H_5$ | $-NO_2$ | $-CH_3$ | 160-163 |
| 15 | $-CO_2CH_3$ | $-NO_2$ | $-CH_3$ | 210-212 (Zers.) |
| 16 | $-CO_2(CH_2)_2OCH_3$ | 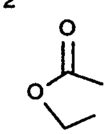 | | 199 |
| 17 | $-CO_2-CH(CH_3)_2$ | 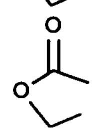 | | 239 |
| 18 | $-CO_2-CH(CH_3)_2$ | -CN | $-CH_3$ | 142-143 |
| 19 | $-CO_2C_2H_5$ | -CN | $-CH_3$ | 193-195 |
| 20 | $-CO_2C_2H_5$ | -CN | $-CH_3$ | 96 amorph. |

Tabelle 5:

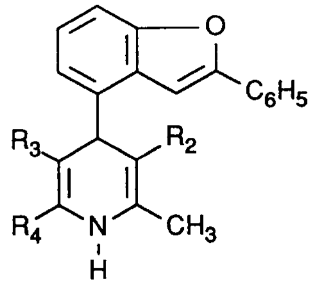

| Bsp.-Nr. | $R^2$ | $R^3$ | $R^4$ | F°C |
|---|---|---|---|---|
| 21 | -CN | -CN | $-CH_3$ | 231 |
| 22 | $-CO_2(CH_2)_2CH_3$ | -CN | $-CH_3$ | 181 |
| 23 | $-CO_2-CH(CH_3)_2$ | 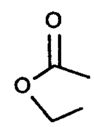 | | 201 |
| 24 | $-CO_2-CH(CH_3)_2$ | $-NO_2$ | $-CH_3$ | 201 |
| 25 | $-CO_2-CH(CH_3)_2$ | -CN | $-CH_3$ | 201 |
| 26 | $-CO_2C_2H_5$ | 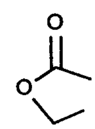 | | 250 |

Tabelle 6:

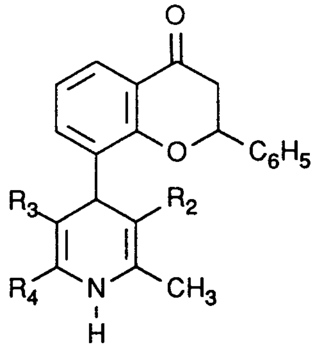

| Bsp.-Nr. | $R^2$ | $R^3$ | $R^4$ | F°C |
|---|---|---|---|---|
| 27 | $-CO_2-C_2H_5$ | 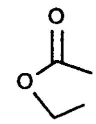 | | 137 (Zers.) |
| 28 | $-CO_2-C_2H_5$ | $-NO_2$ | $-CH_3$ | 150 |
| 29 | $-CO_2C_2H_5$ | -CN | $-CH_3$ | 177 |

Tabelle 7:

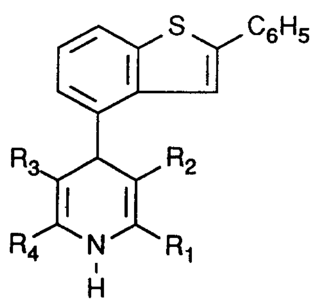

| Bsp.-Nr. | $R^1$ | $R^2$ | $R^3$ | $R^4$ | F°C |
|---|---|---|---|---|---|
| 30 | $-NH_2$ | $-CO_2-CH(CH_3)_2$ | -CN | $-CH_3$ | 258 |
| 31 | $-CH_3$ | $-CO_2-CH(CH_3)_2$ | -CN | $-CH_3$ | 187 |
| 32 | $-CH_3$ | CN | -CN | $-CH_3$ | 223 |
| 33 | $-CH_3$ | $-CO_2-CH(CH_3)_2$ | 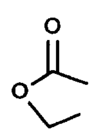 | | 233 |
| 34 | $-CH_3$ | $-CO_2-CH(CH_3)_2$ | $-NO_2$ | $-CH_3$ | 233 |
| 35 | $-CH_2-CO_2CH_3$ | $-CO_2C_2H_5$ | $-CO_2-CH(CH_3)_2$ | $-CH_3$ | 156 |

Tabelle 8:

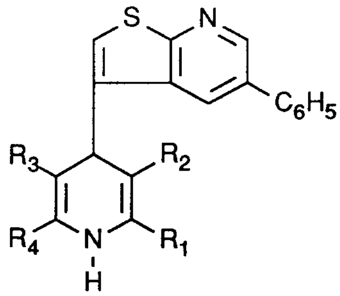

| Bsp.-Nr. | $R^1$ | $R^2$ | $R^3$ | $R^4$ | F°C |
|---|---|---|---|---|---|
| 36 | $-CH_3$ | $-CO_2C_2H_5$ | $-NO_2$ | $-CH_3$ | 263 |
| 37 | $-CH_3$ | $-CO_2-CH(CH_3)_2$ | -CN | $-CH_3$ | 250 |
| 38 | $-CH_3$ | $-CO_2C_2H_5$ | 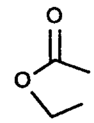 | | 239 |
| 39 | $-CH_3$ | $-CO_2CH_3$ | 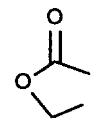 | | 226 |
| 40 | $-CH_3$ | $-CO_2-CH(CH_3)_2$ | $-NO_2$ | $-CH_3$ | 234 |

Fortsetzung Tabelle 8:

| Bsp.-Nr. | $R^1$ | $R^2$ | $R^3$ | $R^4$ | F°C |
|---|---|---|---|---|---|
| 41 | $-CH_3$ | $-CO_2-CH(CH_3)_2$ | 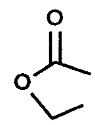 | | 270 |
| 42 | $-CH_3$ | $-CO_2-CH(CH_3)_2$ | $-CO_2(CH_2)_2OCH_3$ | $-CH_3$ | 165-167 |
| 43 | $-CH_3$ | -CN | 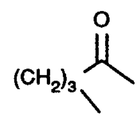 | | 265 |
| 44 | $-NH_2$ | $-CO_2-CH(CH_3)_2$ | -CN | $-CH_3$ | 240 |
| 45 | $-CH_3$ | $-CO_2CH_2CO_2H$ | -CN | $-CH_3$ | 212 |
| 46 | $-CH_3$ | $-CO_2(CH_2)_2OCH_3$ | $-CO_2(CH_2)_2OCH_3$ | $-CH_3$ | 174-176 |
| 47 | $-CH_3$ | $-CO_2C_2H_5$ | -CN | $-CH_3$ | 220 |

Tabelle 9:

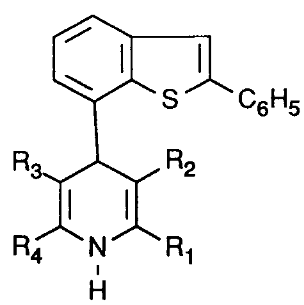

| Bsp.-Nr. | $R^1$ | $R^2$ | $R^3$ | $R^4$ | F°C |
|---|---|---|---|---|---|
| 48 | $-CH_3$ | $-CO_2C_2H_5$ | $-NO_2$ | $-CH_3$ | Schaum |
| 49 | $-CH_3$ | $-CO_2C_2H_5$ | 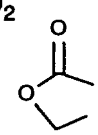 | | Schaum |
| 50 | $-CH_3$ | $-CO_2C_2H_5$ | -CN | $-CH_3$ | 240 |

Tabelle 10:

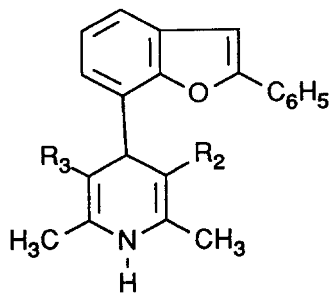

| Bsp.-Nr. | $R^2$ | $R^3$ | F°C |
|---|---|---|---|
| 51 | $-CO_2C_2H_5$ | -CN | 217 |
| 52 | $-CO_2-CH(CH_3)_2$ | -CN | 210 |
| 53 | $-CO_2CH_3$ | $-NO_2$ | 228 |

Tabelle 11:

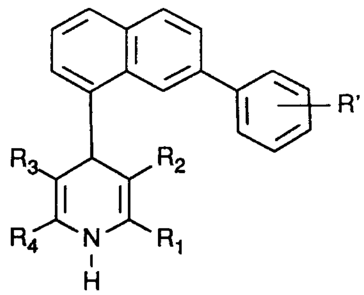

| Bsp.-Nr. | $R^1$ | $R^2$ | $R^3$ | $R^4$ | R' | F°C |
|---|---|---|---|---|---|---|
| 54 | $-CH_3$ | $-CO_2C_2H_5$ | -CN | $-CH_3$ | -o-Cl | 213 |
| 55 | $-CH_3$ | $-CO_2C_2H_5$ | -CN | $-CH_3$ | -m-Cl | 222 |
| 56 | $-CH_3$ | $-CO_2C_2H_5$ | 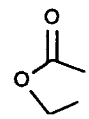 | | -o-F | 140 |
| 57 | $-CH_3$ | $-CO_2C_2H_5$ | $-NO_2$ | $-CH_3$ | -o-F | 182 |
| 58 | $-CH_3$ | $-CO_2C_2H_5$ | -CN | $-CH_3$ | -o-F | 189 |
| 59 | $-CH_3$ | $-CO_2CH_3$ | 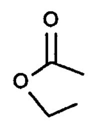 | | H | 282 |

Fortsetzung Tabelle 11:

| Bsp.-Nr. | $R^1$ | $R^2$ | $R^3$ | $R^4$ | R' | F°C |
|---|---|---|---|---|---|---|
| 60 | $-CH_3$ | $-CO_2-CH(CH_3)_2$ | 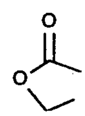 | | H | 237 |
| 61 | $-CH_3$ | $-CO_2-(CH_2)_2OCH_3$ | 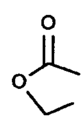 | | H | 119-120 (Zers.) |
| 62 | $-CH_3$ | $-CO_2C_2H_5$ | 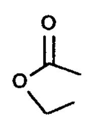 | | H | 245 |
| 63 | $-CH_3$ | $-CO_2C_2H_5$ | -CN | $-CH_3$ | H | 216 |
| 64 | $-CH_3$ | $-CO_2C_2H_5$ | $-NO_2$ | $-CH_3$ | $-CH_3$ | 209 |

Tabelle 12:

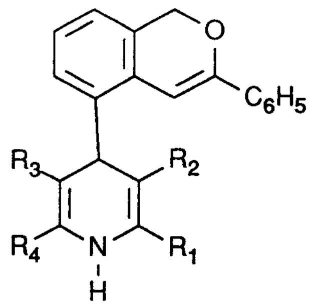

| Bsp.-Nr. | $R^1$ | $R^2$ | $R^3$ | $R^4$ | F°C |
|---|---|---|---|---|---|
| 65 | $-CH_3$ | $-CO_2C_2H_5$ | $-NO_2$ | $-CH_3$ | 257 |
| 66 | $-CH_3$ | $-CO_2CH(CH_3)_2$ | -CN | $-CH_3$ | 140 |
| 67 | $-CH_3$ | $-CO_2CH(CH_3)_2$ | 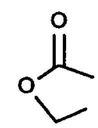 | | 240 |
| 68 | $-CH_3$ | -CN | -CN | $-CH_3$ | 100 |
| 69 | $-CH_3$ | $-CO_2C_2H_5$ | 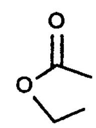 | | 249 |

Tabelle 13:

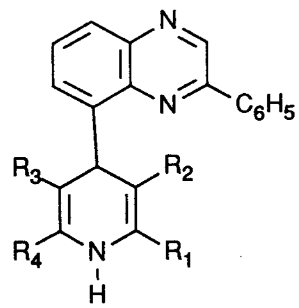

| Bsp.-Nr. | $R^1$ | $R^2$ | $R^3$ | $R^4$ | F°C |
|---|---|---|---|---|---|
| 70 | $-CH_3$ | $-CO_2C_2H_5$ | $-NO_2$ | $-CH_3$ | 245 (Zers.) |
| 71 | $-CH_3$ | $-CO_2C_2H_5$ | 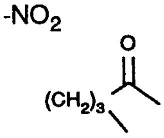 | | 277 |
| 72 | $-CH_3$ | $-CO_2CH_3$ | 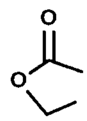 | | 282 (Zers.) |
| 73 | $-CH_3$ | $-CO_2C_2H_5$ | 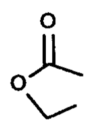 | | 265 (Zers.) |
| 74 | $-CH_3$ | $-CO_2CH(CH_3)_2$ | 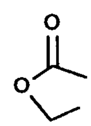 | | 266 |
| 75 | $-CH_3$ | $-CO_2CH(CH_3)_2$ | -CN | $-CH_3$ | 230 |
| 76 | $-CH_3$ | $-CO_2CH_3$ | -CN | $-CH_3$ | 252 |
| 77 | $-CH_3$ | $-CO_2CH(CH_3)_2$ | $-NO_2$ | $-CH_3$ | 228 |
| 78 | $-CH_3$ | $-CO_2C_2H_5$ | -CN | $-CH_3$ | 270 |
| 79 | $-CH_3$ | $-CO_2CH_3$ | -CO-NH 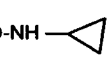 | $-CH_3$ | 150 |

Tabelle 14:

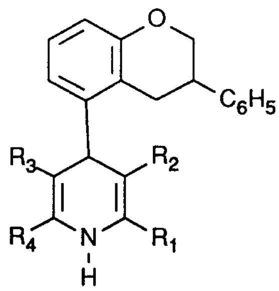

| Bsp.-Nr. | $R^1$ | $R^2$ | $R^3$ | $R^4$ | F°C |
|---|---|---|---|---|---|
| 80 | $-CH_3$ | $-CO_2CH(CH_3)_2$ | -CN | $-CH_3$ | amorpher Schaum * |
| 81 | $-CH_3$ | $-CO_2CH(CH_3)_2$ | $-NO_2$ | $-CH_3$ | amorpher Schaum * |
| 82 | $-CH_3$ | $-CO_2CH(CH_3)_2$ | 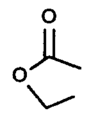 | | amorpher Schaum * |
| 83 | $-CH_3$ | $-CO_2CH(CH_3)_2$ | -CN | $-NH_2$ | amorpher Schaum * |
| 84 | $-CH_3$ | -CN | -CN | $-CH_3$ | amorpher Schaum |

* = Diastereomerenmischung

Tabelle 15:

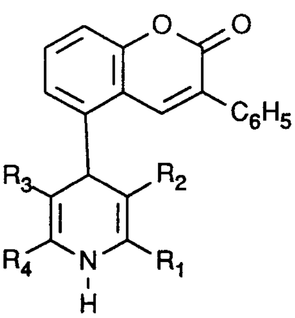

| Bsp.-Nr. | $R^1$ | $R^2$ | $R^3$ | $R^4$ | F°C |
|---|---|---|---|---|---|
| 85 | $-CH_3$ | $-CO_2CH(CH_3)_2$ | $-CO_2C_2H_5$ | $-CH_2-O-CO-CH_3$ | 166 |
| 86 | $-CH_3$ | $-CO_2CH(CH_3)_2$ | 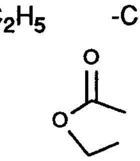 | | 170 |
| 87 | $-CH_3$ | -CN | -CN | $-CH_3$ | 261 |

Tabelle 16:

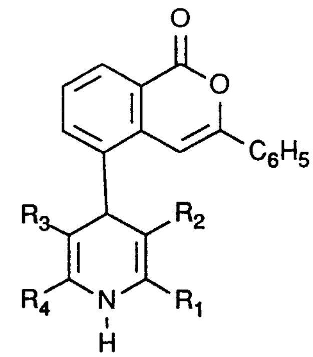

| Bsp.-Nr. | $R^1$ | $R^2$ | $R^3$ | $R^4$ | F°C |
|---|---|---|---|---|---|
| 88 | $-CH_3$ | $-CO_2CH(CH_3)_2$ | $-CO_2C_2H_5$ | $-CH_2-O-CO-CH_3$ | 270 |
| 89 | $-CH_3$ | $-CO_2C_2H_5$ | $-CO_2C_2H_5$ | $-CH_2-O-CO-CH_3$ | 186 |
| 90 | $-CH_3$ | $-CO_2-C_2H_5$ | 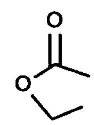 | | 272 |
| 91 | $-CH_3$ | $-CO_2CH(CH_3)_2$ | -CN | $-CH_3$ | 222 |
| 92 | $-CH_3$ | $-CO_2(CH_2)_2CH_3$ | -CN | $-CH_3$ | 218 |
| 93 | $-CH_3$ | $-CO_2C_2H_5$ | -CN | $-CH_3$ | 242 |
| 94 | $-CH_3$ | $-CO_2CH_3$ | $-NO_2$ | $-CH_3$ | 188 |
| 95 | $-CH_3$ | $-CO_2CH(CH_3)_2$ | 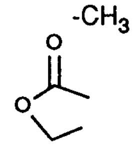 | | 270 |

Tabelle 17:

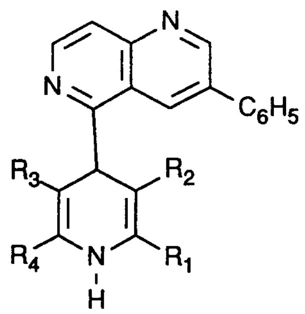

| Bsp.-Nr. | $R^1$ | $R^2$ | $R^3$ | $R^4$ | F°C |
|---|---|---|---|---|---|
| 96 | $-CH_3$ | $-CO_2CH(CH_3)_2$ | $-CO_2C_2H_5$ | $-CH_2-O-CO-CH_3$ | 195 |
| 97 | $-CH_3$ | $-CO_2C_2H_5$ | -CN | $-CH_2-O-CO-CH_3$ | 208 |
| 98 | $-CH_3$ | $-CO_2CH(CH_3)_2$ | -CN | $-CH_3$ | 235 |
| 99 | $-CH_3$ | $-CO_2(CH_2)_2CH_3$ | -CN | $-CH_3$ | 251 |
| 100 | $-CH_3$ | $-CO_2C_2H_5$ | -CN | $-CH_3$ | 243 |
| 101 | $-CH_3$ | $-CO_2C_2H_5$ | 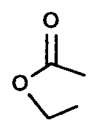 | | 285 |
| 102 | $-CH_3$ | $-CO_2CH(CH_3)_2$ | 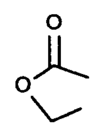 | | 267 |

Tabelle 18:

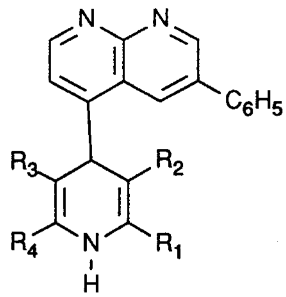

| Bsp.-Nr. | $R^1$ | $R^2$ | $R^3$ | $R^4$ | F°C |
|---|---|---|---|---|---|
| 103 | $-CH_3$ | $-CO_2C_2H_5$ | $-NO_2$ | $-CH_3$ | 282 |
| 104 | $-CH_3$ | $-CO_2CH(CH_3)_2$ | $-NO_2$ | $-CH_3$ | 280 |
| 105 | $-CH_3$ | $-CO_2CH_3$ | 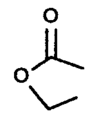 | | 205 |
| 106 | $-CH_3$ | $-CO_2CH(CH_3)_2$ | 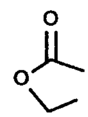 | | 258 |
| 107 | $-CH_3$ | $-CO_2C_2H_5$ | 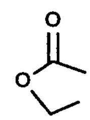 | | 268 |
| 108 | $-CH_3$ | $-CO_2CH_3$ | -CN | $-CH_3$ | 255 |
| 109 | $-CH_3$ | $-CO_2CH_3$ | -CO-NH 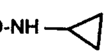 | $-CH_3$ | 230 (Zers.) |
| 110 | $-CH_3$ | $-CO_2CH(CH_3)_2$ | -CN | $-CH_3$ | 269 |

Tabelle 19:

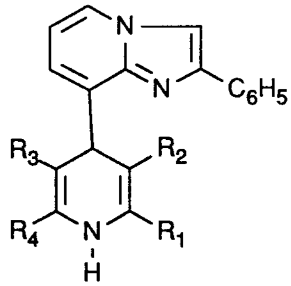

| Bsp.-Nr. | $R^1$ | $R^2$ | $R^3$ | $R^4$ | F°C |
|---|---|---|---|---|---|
| 111 | $-CH_3$ | $-CO_2CH(CH_3)_2$ | 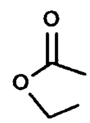 | | 226 |
| 112 | $-CH_3$ | $-CO_2C_2H_5$ | 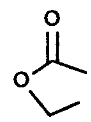 | | 218 |
| 113 | $-CH_3$ | $-CO_2CH(CH_3)_2$ | $-NO_2$ | $-CH_3$ | 229 |
| 114 | $-CH_3$ | $-CO_2C_2H_5$ | $-NO_2$ | $-CH_3$ | 234 |
| 115 | $-CH_3$ | $-CO_2C_2H_5$ | -CN | $-CH_3$ | 220 |
| 116 | $-CH_3$ | $-CO_2CH(CH_3)_2$ | -CN | $-CH_3$ | 150 |
| 117 | $-CH_3$ | -CN | -CN | $-CH_3$ | 280 |

**Patentansprüche**

**1.** Dihydropyridine der allgemeinen Formel (I)

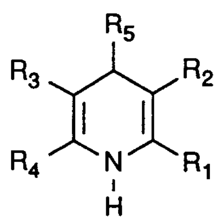

(I)

in welcher

$R^1$ und $R^4$ gleich oder verschieden sind und für Wasserstoff, Amino, Cyano, Formyl, Trifluormethyl oder für geradkettiges oder verzweigtes Alkyl mit bis zu 8 Kohlenstoffatomen stehen, das gegebenenfalls durch Hydroxy, geradkettiges oder verzweigtes Alkoxycarbonyl mit bis zu 6 Kohlenstoffatomen oder durch eine Gruppe der Formel $-NR^6R^7$, $-O-CO-R^8$, $-O-(CH_2)_a-OR^{8'}$ oder $-O-(CH_2)_b-NR^9R^{10}$ substituiert ist,

worin

$R^6$, $R^7$, $R^9$ und $R^{10}$ gleich oder verschieden sind und Wasserstoff, Phenyl oder geradkettiges oder verzweigtes Alkyl mit bis zu 6 Kohlenstoffatomen bedeuten,

$R^8$ und $R^{8'}$ gleich oder verschieden sind und geradkettiges oder verzweigtes Alkyl mit bis zu 6 Kohlenstoffatomen bedeuten,
und
a und b gleich oder verschieden sind und eine Zahl 2, 3, 4 oder 5 bedeuten,
$R^2$ für Cyano oder für eine Gruppe der Formel - $CO-NR^{11}R^{12}$ oder $-CO-A-R^{13}$ steht,
worin
$R^{11}$ und $R^{12}$ gleich oder verschieden sind und Wasserstoff, einen geradkettigen, verzweigten oder cyclischen, gesättigten oder ungesättigten Kohlenwasserstoffrest mit bis zu 8 Kohlenstoffatomen bedeuten, der gegebenenfalls durch Halogen, Hydroxy, Cyano oder durch Aryl, Aryloxy, Arylthio mit jeweils 6 bis 10 Kohlenstoffatomen oder durch einen 5-bis 7-gliedrigen, gesättigen oder ungesättigten Heterocyclus mit bis zu 3 Heteroatomen aus der Reihe S, N oder O substituiert ist, wobei die Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder
Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O bedeuten, die gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind,
oder
$R^{11}$ und $R^{12}$ gemeinsam unter Einbezug des Stickstoffatoms einen 3-bis 8-gliedrigen, gesättigen oder ungesättigten Heterocyclus bilden, der gegebenenfalls durch ein Sauerstoffatom oder durch einen Rest der Formel $S(O)_d$, - CO- oder $-NR^{15}$- unterbrochen sein kann,
worin
d eine Zahl 0, 1 oder 2 bedeutet,
$R^{15}$ Wasserstoff oder Aryl mit 6 bis 10 Kohlenstoffatomen bedeutet, das gegebenenfalls bis zu 2-fach gleich oder verschieden durch Halogen, Cyano oder durch geradkettiges oder verzweigtes Alkyl, Alkoxy, Alkylthio, Alkoxycarbonyl mit jeweils bis zu 8 Kohlenstoffatomen, Halogenalkyl, Halogenalkoxy oder Halogenalkylthio mit jeweils bis zu 4 Kohlenstoffatomen substituiert ist, oder
einen cyclischen, gesättigten oder ungesättigten, geradkettigen oder verzweigten Kohlenwasserstoffrest mit bis zu 8 Kohlenstoffatomen bedeutet, der gegebenenfalls durch Hydroxy, Halogen oder durch Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O substituiert ist, wobei die Cyclen ihrerseits bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,

und der Heterocyclus gegebenenfalls durch geradkettiges oder verzweigtes Alkoxy oder Alkylthio mit jeweils bis zu 4 Kohlenstoffatomen, Halogen, Aryl mit 6 bis 10 Kohlenstoffatomen, einen 5-bis 7-gliedrigen, gesättigten oder ungesättigten Heterocyclus mit bis zu 3 Heteroatomen aus der Reihe S, N oder O oder durch geradkettiges oder verzweigtes Alkyl mit bis zu 4 Kohlenstoffatomen substituiert ist, das seinerseits durch Aryl mit 6 bis 10

Kohlenstoffatomen substituiert sein kann,

A eine direkte Bindung oder ein Sauerstoffatom bedeutet,

$R^{13}$ Wasserstoff oder Aryl mit 6 bis 10 Kohlenstoffatomen oder einen 5- bis 7-gliedrigen, gesättigten oder ungesättigten Heterocyclus mit bis zu 3 Heteroatomen aus der Reihe S, N oder O bedeutet, wobei die Cyclen gegebenenfalls bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind, oder
einen cyclischen, geradkettigen oder verzweigten, gesättigten oder ungesättigten Kohlenwasserstoffrest mit bis zu 10 Kohlenstoffatomen bedeutet, der gegebenenfalls bis zu 3-fach gleich oder verschieden durch Sauerstoff oder durch - CO-, -CO-NH-, -O-CO-, -CO-O-, -NH-CO-, $-SO_2$-NH-, NH-$SO_2$-, $-S(O)_e$-oder $-NR^{16}$- unterbrochen ist,
worin

e die oben angegebene Bedeutung von d hat und mit dieser gleich oder verschieden ist,

$R^{16}$ die oben angegebene Bedeutung von $R^{15}$ hat und mit dieser gleich oder verschieden ist,

oder der Kohlenwasserstoffrest gegebenenfalls bis zu 3-fach gleich oder verschieden durch Aryliden mit 6 bis 10 Kohlenstoffatomen oder heterocyclische Reste der Formeln

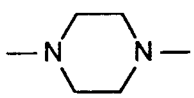 , 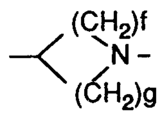 , 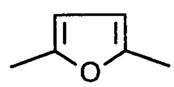 oder 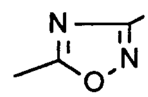

unterbrochen ist,
worin

f und g gleich oder verschieden sind und eine Zahl 1 oder 2 bedeuten,

und wobei Aryliden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein kann,

und der Kohlenwasserstoffrest gegebenenfalls bis zu 3-fach gleich oder verschieden durch Cycloalkyl mit 3 bis 8 Kohlenstoffatomen, Halogen, Nitro, Cyano, Hydroxy, - $O-NO_2$, geradkettiges oder verzweigtes Alkylthio, Alkoxy oder Acyloxy mit jeweils bis zu 8 Kohlenstoffatomen oder durch Aryl, Aryloxy oder Arylthio mit jeweils 6 bis 10 Kohlenstoffatomen oder durch einen 5- bis 7-gliedrigen, gesättigten oder ungesättigen Heterocyclus mit bis zu 3 Heteroatomen aus der Reihe S, N oder O substituiert ist, wobei die Cyclen ihrerseits bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder
der Kohlenwasserstoffrest gegebenenfalls durch eine Gruppe der Formel $-CO_2-R^{17}$, $-CONR^{18}R^{19}$, - $NR^{20}R^{21}$ oder $-NR^{22}-CO_2R^{23}$ substituiert ist,
worin

$R^{17}$ die oben angegebene Bedeutung von $R^{15}$ hat und mit dieser gleich oder verschieden ist
und

$R^{18}$, $R^{19}$, $R^{20}$, $R^{21}$, $R^{22}$ und $R^{23}$ die oben angegebene Bedeutung von $R^{11}$ und $R^{12}$ haben und mit diesen gleich oder verschieden sind,

$R^3$ für Cyano, Nitro, Formyl oder geradkettiges oder verzweigtes Alkoxycarbonyl mit bis zu 6 Kohlenstoffatomen steht, das gegebenenfalls durch geradkettiges oder verzweigtes Alkoxy mit bis zu 6 Kohlenstoffatomen substituiert ist, oder für eine Gruppe der Formel - CO-NH-G steht, worin

G Cycloalkyl mit 3 bis 6 Kohlenstoffatomen bedeutet,

oder

$R^3$ und $R^4$ gemeinsam einen Rest der Formel

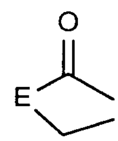

bilden, worin

E ein Sauerstoff- oder Schwefelatom oder die $-(CH_2)_n$-Gruppe bedeutet, worin

n eine Zahl 1 oder 2 bedeutet,

$R^5$ für einen Rest der Formel

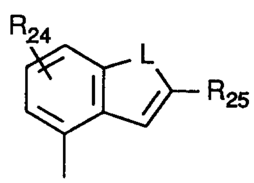
,
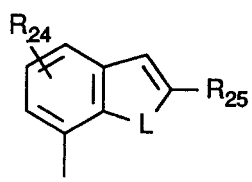
,
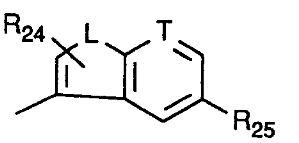
,

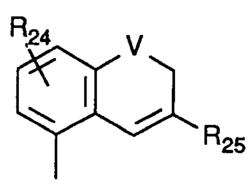
,
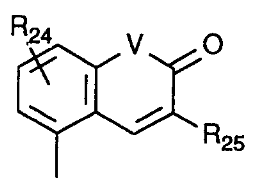
,
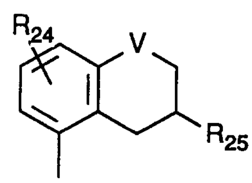
,

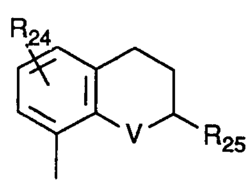
,
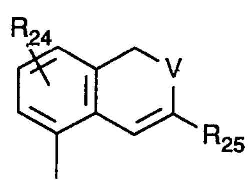
,
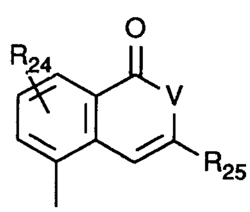
,

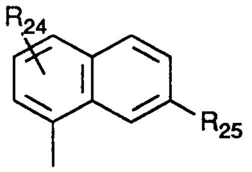 , 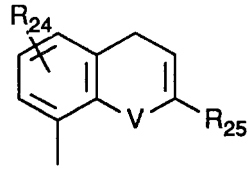 , 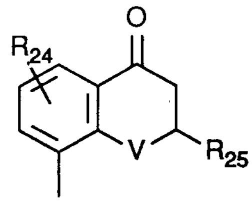 ,

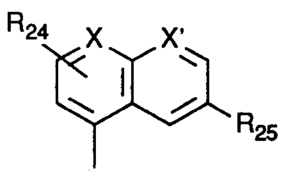 , 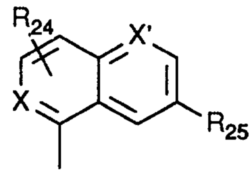 , 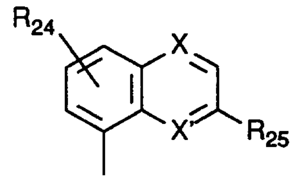 ,

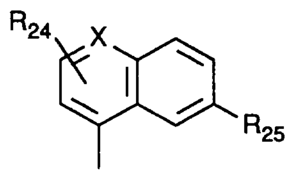 , 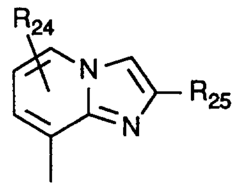 oder 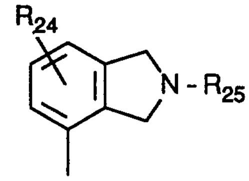

steht,

worin

$R^{24}$ Wasserstoff, Halogen oder geradkettiges oder verzweigtes Alkyl oder Alkoxy mit jeweils bis zu 8 Kohlenstoffatomen bedeutet,

$R^{25}$ Aryl mit 6 bis 10 Kohlenstoffatomen bedeutet, das gegebenenfalls bis zu 3-fach gleich oder verschieden durch Halogen, Nitro, Cyano, Trifluormethyl, Trifluormethoxy, Trifluormethylthio,
oder durch geradkettiges oder verzweigtes Alkyl mit bis zu 8 Kohlenstoffatomen substituiert ist, das seinerseits durch Aryl mit 6 bis 10 Kohlenstoffatomen substituiert sein kann,
oder durch geradkettiges oder verzweigtes Alkoxy oder Alkoxycarbonyl mit jeweils bis zu 8 Kohlenstoffatomen, Carboxy, Amino oder durch eine Gruppe der Formel $-NR^{26}R^{27}$ substituiert ist,
worin

$R^{26}$ und $R^{27}$ gleich oder verschieden sind und Wasserstoff, geradkettiges oder verzweigtes Alkyl mit bis zu 8 Kohlenstoffatomen, Phenyl oder Benzyl bedeuten,

oder

$R^{25}$ einen cyclischen, geradkettigen oder verzweigten, gesättigten oder ungesättigten Kohlenwasserstoffrest mit bis zu 12 Kohlenstoffatomen bedeutet, der gegebenenfalls bis zu 2-fach gleich oder verschieden durch Sauerstoff oder Schwefel unterbrochen ist,
und der gegebenenfalls bis zu 3-fach gleich oder verschieden durch Cycloalkyl mit 3 bis 8 Kohlenstoffatomen, geradkettiges oder verzweigtes Acyloxy mit bis zu 4 Kohlenstoffatomen, Halogen, Nitro, Cyano, Hydroxy oder durch Aryl, Aryloxy oder Arylthio mit jeweils 6 bis 10 Kohlenstoffatomen oder durch einen 5-bis 7-gliedrigen, gesättigten oder ungesättigen gegebenenfalls kondensierten Heterocyclus mit bis zu 5 Heteroatomen aus der Reihe S, N oder O substituiert ist, wobei die Cyclen ihrerseits bis zu 3-fach gleich oder verschieden durch Halogen, Cyano, Nitro, Hydroxy oder durch geradkettiges oder verzweigtes Alkyl oder Alkoxy mit jeweils bis zu 4 Kohlenstoffatomen, Trifluormethyl, Trifluormethoxy,

Trifluormethylthio oder durch eine Gruppe der Formel $-NR^{28}R^{29}$ substituiert sein können,
worin

$R^{28}$ und $R^{29}$ die oben angegebene Bedeutung von $R^{11}$ und $R^{12}$ haben und mit dieser gleich oder verschieden sind,
oder der Kohlenwasserstoffrest gegebenenfalls durch eine Gruppe der Formel $-CO_2-R^{30}$, $-CONR^{31}R^{32}$, $-NR^{33}R^{34}$, $-NR^{35}-CO_2R^{36}$ oder $-NR^{37}-SO_2R^{38}$ substituiert ist,
worin

$R^{30}$ die oben angegebene Bedeutung von $R^{15}$ hat und mit dieser gleich oder verschieden ist
und

$R^{31}$, $R^{32}$, $R^{33}$, $R^{34}$, $R^{35}$, $R^{36}$, $R^{37}$ und $R^{38}$ die oben angegebene Bedeutung von $R^{11}$ und $R^{12}$ haben und mit diesen gleich oder verschieden sind,
oder

$R^{25}$ einen 5-bis 7-gliedrigen, gesättigten oder ungesättigten Heterocyclus mit bis zu 4 Heteroatomen aus der Reihe S, N oder O bedeutet, der gegebenenfalls bis zu 3-fach gleich oder verschieden durch Halogen, Amino, Cyano, Nitro oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen oder durch $C_1$-$C_4$-Mono-oder - Dialkylamino substituiert ist,

oder

$R^{25}$ eine Gruppe der Formel $D-R^{39}$ bedeutet,
worin

D die CO- oder $-S(O)_h$-Gruppe oder ein Sauerstoffatom bedeutet,
worin

h eine Zahl 0, 1 oder 2 bedeutet,
und

$R^{39}$ Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O bedeutet, der gegebenenfalls bis zu 3-fach gleich oder verschieden durch Halogen, Amino, Cyano, Nitro oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen oder durch $C_1$-$C_4$-Mono- oder -Dialkylamino substituiert ist,
oder

$R^{39}$ Wasserstoff oder einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel unterbrochen ist,
und der gegebenenfalls durch Halogen, Aryl, Aryloxy oder Arylthio mit jeweils 6 bis 10 Kohlenstoffatomen oder durch einen 5- bis 7-gliedrigen, gesättigten oder ungesättigen Heterocyclus mit bis zu 3 Heteroatomen aus der Reihe S, N oder O substituiert ist, wobei die Cyclen ihrerseits durch Halogen, Trifluormethyl, Methyl, Methoxy, Nitro oder Methylthio substituiert sein können, oder
durch eine Gruppe der Formel $-NR^{40}R^{41}$ substituiert ist
worin

$R^{40}$ und $R^{41}$ die oben angegebene Bedeutung von $R^{11}$ und $R^{12}$ haben und mit dieser gleich oder verschieden sind,

L ein Schwefel- oder Sauerstoffatom oder die - NH-Gruppe bedeutet,

T ein Stickstoffatom oder die N->O-Gruppe bedeutet,

V ein Schwefel- oder Sauerstoffatom bedeutet,

X und X' gleich oder verschieden sind und ein Stickstoffatom oder die N->O-Gruppe bedeuten,

und deren Salze.

**2.** Verbindungen der allgemeinen Formel (I) gemaß Anspruch 1 in welcher

$R^1$ und $R^4$ gleich oder verschieden sind und für Wasserstoff, Amino, Cyano, Formyl, Trifluormethyl oder für geradkettiges oder verzweigtes Alkyl mit bis zu 4 Kohlenstoffatomen stehen, das gegebenenfalls durch Hydroxy, geradkettiges oder verzweigtes Alkoxycarbonyl mit bis zu 4 Kohlenstoffatomen oder durch eine Gruppe der Formel - $NR^6R^7$, -O-CO-$R^8$, -O-$(CH_2)_a$-$OR^{8'}$ oder -O-$(CH_2)_b$-$NR^9R^{10}$ substituiert ist,
worin

$R^6$, $R^7$, $R^9$ und $R^{10}$ gleich oder verschieden sind und Wasserstoff oder geradkettiges oder verzweigtes Alkyl mit bis zu 4 Kohlenstoffatomen bedeuten,

$R^8$ und $R^{8'}$ gleich oder verschieden sind und geradkettiges oder verzweigtes Alkyl mit bis zu 4 Kohlenstoffatomen bedeuten,
und

a und b gleich oder verschieden sind und eine Zahl 2, 3, 4 oder 5 bedeuten,

$R^2$ für Cyano oder für eine Gruppe der Formel - CO-$NR^{11}R^{12}$ oder -CO-A-$R^{13}$ steht,
worin

$R^{11}$ und $R^{12}$ gleich oder verschieden sind und Wasserstoff, einen geradkettigen, verzweigten oder cyclischen, gesättigten oder ungesättigten Kohlenwasserstoffrest mit bis zu 6 Kohlenstoffatomen bedeuten, der gegebenenfalls durch Fluor, Chlor, Hydroxy, Phenyl oder Pyridyl substituiert ist, wobei die Cyclen ihrerseits durch Fluor, Chlor oder durch Alkyl, Alkoxy, Alkylthio oder Alkoxycarbonyl mit jeweils bis zu 2 Kohlenstoffatomen, Trifluormethyl oder Trifluormethoxy substituiert sein können, oder
Phenyl oder Pyridyl bedeuten, die gegebenenfalls durch Fluor, Chlor oder durch Alkyl, Alkoxy, Alkylthio oder Alkoxycarbonyl mit jeweils bis zu 2 Kohlenstoffatomen, Trifluormethyl oder Trifluormethoxy substituiert sind,
oder

$R^{11}$ und $R^{12}$ gemeinsam unter Einbezug des Stickstoffatoms einen 3-bis 8-gliedrigen, gesättigen oder ungesättigten Heterocyclus bilden, der gegebenenfalls durch ein Sauerstoffatom oder durch einen Rest der Formel $S(O)_d$, - CO- oder -$NR^{15}$- unterbrochen sein kann,
worin

d eine Zahl 0, 1 oder 2 bedeutet,

$R^{15}$ Wasserstoff oder Phenyl bedeutet, das gegebenenfalls durch Fluor, Chlor, Methyl, Ethyl, Methoxy, Ethoxy, Trifluormethyl oder Trifluormethoxy substituiert ist, oder
einen cyclischen, gesättigten oder ungesättigten, geradkettigen oder verzweigten Kohlenwasserstoffrest mit bis zu 4 Kohlenstoffatomen bedeutet, der gegebenenfalls durch Fluor, Chlor, Phenyl oder durch einen 5- bis 7-gliedrigen, gesättigten oder ungesättigten Heterocyclus mit bis zu 3 Heteroatomen aus der Reihe S, N oder O substituiert ist, wobei die Cyclen ihrerseits durch Fluor, Chlor, Methyl, Methoxy, Methylthio, Trifluormethyl oder Trifluormethoxy substituiert sein können,

A eine direkte Bindung oder ein Sauerstoffatom bedeutet,

$R^{13}$ Wasserstoff, Phenyl oder Pyridyl bedeutet, die gegebenenfalls durch Fluor, Chlor, Methyl, Methoxy, Methylthio, Trifluormethyl oder Trifluormethoxy substituiert sind, oder
einen cyclischen, geradkettigen oder verzweigten, gesättigten oder ungesättigten Kohlenwasserstoffrest mit bis zu 8 Kohlenstoffatomen bedeutet, der gegebenenfalls bis zu 2-fach gleich oder verschieden durch Sauerstoff oder durch - CO-, -CO-NH-, -O-CO-,

-CO-O-, -NH-CO-, $-SO_2-NH-$, $-NH-SO_2-$, $-S(O)_e$-oder $-NR^{16}-$ unterbrochen ist,
worin

e — die oben angegebene Bedeutung von d hat und mit dieser gleich oder verschieden ist,

$R^{16}$ — die oben angegebene Bedeutung von $R^{15}$ hat und mit dieser gleich oder verschieden ist,

oder der Kohlenwasserstoffrest gegebenenfalls bis zu 2-fach gleich oder verschieden durch Aryliden mit 6 bis 10 Kohlenstoffatomen oder heterocyclische Reste der Formeln

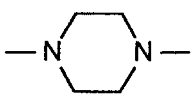 , 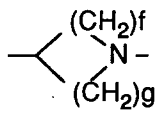 , 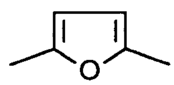 oder 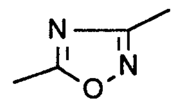

unterbrochen ist,
worin

f und g — gleich oder verschieden sind und eine Zahl 1 oder 2 bedeuten,

und der Kohlenwasserstoffrest gegebenenfalls bis zu 2-fach gleich oder verschieden durch Cyclopropyl, Cyclobutyl, Cyclopentyl, Cyclohexyl, Fluor, Chlor, Nitro, Cyano, Hydroxy, $-O-NO_2$, geradkettiges oder verzweigtes Alkylthio, Alkoxy oder Acyloxy mit jeweils bis zu 4 Kohlenstoffatomen oder durch Phenyl, Phenoxy, Phenylthio oder Pyridyl substituiert ist, wobei die Cyclen ihrerseits durch Fluor, Chlor, Cyano, Methyl, Methoxy, Methylthio, Trifluormethyl oder Trifluormethoxy substituiert sein können, oder der Kohlenwasserstoffrest gegebenenfalls durch eine Gruppe der Formel $-CO_2-R^{17}$, $-CONR^{18}R^{19}$, $-NR^{20}R^{21}$ oder $-NR^{22}-CO_2R^{23}$ substituiert ist,
worin

$R^{17}$ — die oben angegebene Bedeutung von $R^{15}$ hat und mit dieser gleich oder verschieden ist
und

$R^{18}$, $R^{19}$, $R^{20}$, $R^{21}$, $R^{22}$ und $R^{23}$ — die oben angegebene Bedeutung von $R^{11}$ und $R^{12}$ haben und mit diesen gleich oder verschieden sind,

$R^3$ — für Cyano, Nitro, Formyl oder geradkettiges oder verzweigtes Alkoxycarbonyl mit bis zu 4 Kohlenstoffatomen steht, das gegebenenfalls durch geradkettiges oder verzweigtes Alkoxy mit bis zu 4 Kohlenstoffatomen substituiert ist, oder
für eine Gruppe der Formel - CO-NH-G steht,
worin

G — Cyclopropyl, Cyclopentyl oder Cyclohexyl bedeutet,

oder

$R^3$ und $R^4$ — gemeinsam einen Rest der Formel

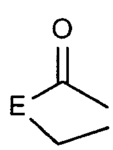

bilden,
worin

E — ein Sauerstoff-oder Schwefelatom oder die - $(CH_2)_n$-Gruppe bedeutet,
worin

n — eine Zahl 1 oder 2 bedeutet,

$R^5$ für einen Rest der Formel

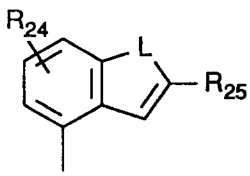 , 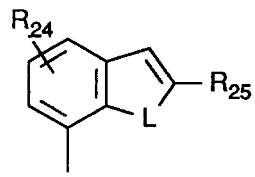 , 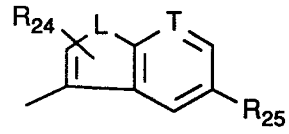 ,

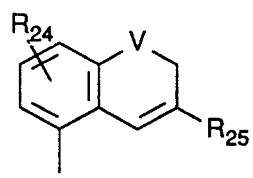 , 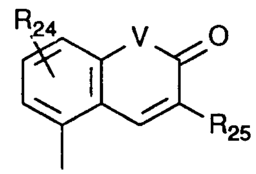 , 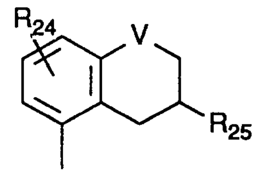 ,

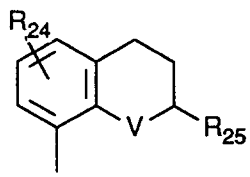 , 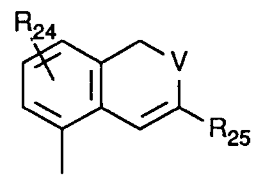 , 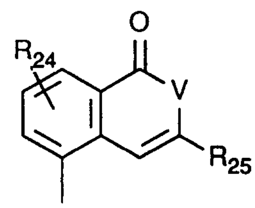 ,

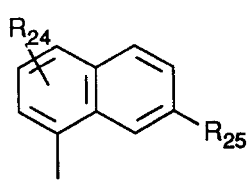 , 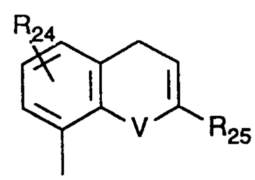 , 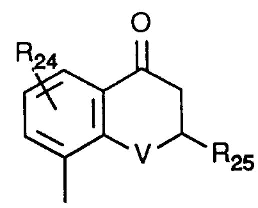 ,

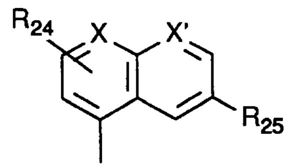 , 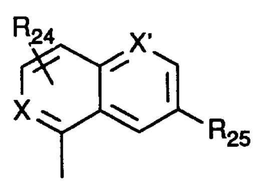 , 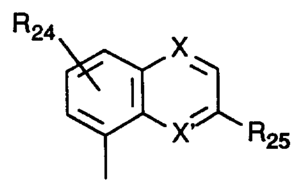 ,

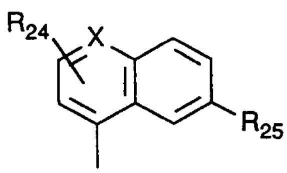 , 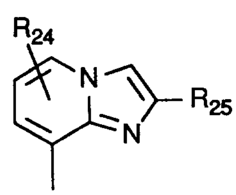 oder 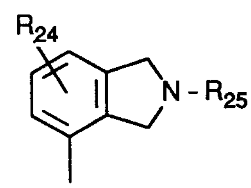

steht,

worin

$R^{24}$ Wasserstoff, Fluor oder Chlor bedeutet,

$R^{25}$ Phenyl bedeutet, das gegebenenfalls bis zu 2-fach gleich oder verschieden durch Halogen, Nitro, Cyano, Trifluormethyl oder durch geradkettiges oder verzweigtes Alkyl oder Alkoxy mit jeweils bis zu 4 Kohlenstoffatomen oder durch eine Gruppe der Formel - $NR^{26}R^{27}$ substituiert ist,

worin

$R^{26}$ und $R^{27}$ gleich oder verschieden sind und Wasserstoff, geradkettiges oder verzweigtes Alkyl mit bis zu 6 Kohlenstoffatomen, Phenyl oder Benzyl bedeuten,

oder

$R^{25}$ einen cyclischen, geradkettigen oder verzweigten, gesättigten oder ungesättigten Kohlenwasserstoffrest mit bis zu 10 Kohlenstoffatomen bedeutet, der gegebenenfalls durch Sauerstoff oder Schwefel unterbrochen ist, und der gegebenenfalls bis zu 2-fach gleich oder verschieden durch Cyclopropyl, Cyclobutyl, Cyclopentyl, Cyclohexyl, Fluor, Chlor, Brom, Acyloxy mit bis zu 2 Kohlenstoffatomen, Cyano, Hydroxy oder durch Phenyl, Phenyloxy, Phenylthio oder durch einen 5-bis 7-gliedrigen, gesättigten oder ungesättigen Heterocyclus mit bis zu 3 Heteroatomen aus der Reihe S, N oder O substituiert ist, wobei die Phenyl-und die Hetero-Cyclen ihrerseits bis zu 2-fach gleich oder verschieden durch Fluor, Chlor, Brom, Hydroxy, Methyl, Ethyl, Methoxy, Ethoxy, Trifluormethyl, Trifluormethoxy oder Trifluormethylthio oder durch eine Gruppe der Formel $-NR^{28}R^{29}$ substituiert sein können,

worin

$R^{28}$ und $R^{29}$ die oben angegebene Bedeutung von $R^{11}$ und $R^{12}$ haben und mit dieser gleich oder verschieden sind,

oder der Kohlenwasserstoffrest gegebenenfalls durch eine Gruppe der Formel $-CO_2-R^{30}$, $-CONR^{31}R^{32}$, $-NR^{33}R^{34}$, $-NR^{35}-CO_2R^{36}$ oder $-NR^{37}-SO_2R^{38}$ substituiert ist,

worin

$R^{30}$ die oben angegebene Bedeutung von $R^{15}$ hat und mit dieser gleich oder verschieden ist

und

$R^{31}$, $R^{32}$, $R^{33}$, $R^{34}$, $R^{35}$, $R^{36}$, $R^{37}$ und $R^{38}$ die oben angegebene Bedeutung von $R^{11}$ und $R^{12}$ haben und mit diesen gleich oder verschieden sind,

oder

$R^{25}$ einen 5-bis 7-gliedrigen, gesättigten oder ungesättigten Heterocyclus mit bis zu 4 Heteroatomen aus der Reihe S, N oder O bedeutet, der gegebenenfalls bis zu 2-fach gleich oder verschieden durch Fluor, Chlor, Brom, Cyano, Nitro oder durch Alkyl, Alkoxy, Alkylthio, Alkoxycarbonyl, Halogenalkyl, Halogenalkoxy oder Halogenalkylthio mit jeweils bis zu 2 Kohlenstoffatomen, Amino oder durch $C_1$-$C_4$-Mono-oder -Dialkylamino substituiert ist,

oder

$R^{25}$ eine Gruppe der Formel $D-R^{39}$ bedeutet,

worin

D die CO-oder $-S(O)_h$-Gruppe oder ein Sauerstoffatom bedeutet,

worin

h eine Zahl 0, 1 oder 2 bedeutet,

und

$R^{39}$ Phenyl oder einen 5-bis 7-gliedrigen, gesättigten oder ungesättigten Heterocyclus mit bis zu 3 Heteroatomen aus der Reihe S, N oder O bedeutet, der gegebenenfalls bis zu 2-fach gleich oder verschieden durch Fluor, Chlor, Brom, Cyano, Nitro oder durch geradkettiges oder verzweigtes Alkyl, Alkoxy, Alkylthio, Alkoxycarbonyl, Halogenalkyl, Halogenalkoxy oder Halogenalkylthio

| | |
|---|---|
| | mit jeweils bis zu 4 Kohlenstoffatomen, Amino oder durch $C_1$-$C_4$-Mono-oder - Dialkylamino substituiert ist, oder |
| $R^{39}$ | Wasserstoff oder einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel unterbrochen ist, und der gegebenenfalls durch Fluor, Chlor, Phenyl, Phenoxy oder Phenylthio substituiert sein kann, oder durch eine Gruppe der Formel - $NR^{40}R^{41}$ substituiert ist worin |
| $R^{40}$ und $R^{41}$ | die oben angegebene Bedeutung von $R^{11}$ und $R^{12}$ haben und mit dieser gleich oder verschieden sind, |
| L | ein Schwefel-oder Sauerstoffatom oder die - NH-Gruppe bedeutet, |
| T | ein Stickstoffatom oder die N->O-Gruppe bedeutet, |
| V | ein Schwefel-oder Sauerstoffatom bedeutet, |
| X und X' | gleich oder verschieden sind und ein Stickstoffatom oder die N->O-Gruppe bedeuten |

und deren Salze.

**3.** Verbindungen der allgemeinen Formel (I) gemäß Anspruch 1 in welcher

| | |
|---|---|
| $R^1$ und $R^4$ | gleich oder verschieden sind und für Wasserstoff, Amino oder geradkettiges oder verzweigtes Alkyl mit bis zu 3 Kohlenstoffatomen stehen, das gegebenenfalls durch Methoxycarbonyl oder durch die Gruppe der Formel -O-CO-$CH_3$ substituiert ist, |
| $R^2$ | für Cyano oder für eine Gruppe der Formel - CO-$NR^{11}R^{12}$ oder -CO-A-$R^{13}$ steht, worin |
| $R^{11}$ und $R^{12}$ | gleich oder verschieden sind und Wasserstoff, einen geradkettigen, verzweigten oder cyclischen, gesättigten oder ungesättigten Kohlenwasserstoffrest mit bis zu 6 Kohlenstoffatomen bedeuten, oder Phenyl bedeuten, das gegebenenfalls durch Fluor, Chlor, Methyl oder Methoxy substituiert ist, |
| A | eine direkte Bindung oder ein Sauerstoffatom bedeutet, |
| $R^{13}$ | Wasserstoff oder einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel oder durch -CO-NH-, -O-CO-, -CO-O-, -NH-CO-, -$SO_2$-NH-, -NH-$SO_2$- oder -$NR^{16}$- unterbrochen ist, worin |
| $R^{16}$ | Wasserstoff, geradkettiges oder verzweigtes Alkyl mit bis zu 4 Kohlenstoffatomen bedeutet, oder der Kohlenwasserstoffrest durch heterocyclische Reste der Formeln |

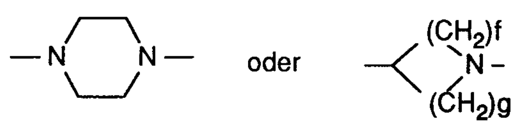

unterbrochen ist,
worin

| | |
|---|---|
| f und g | gleich oder verschieden sind und eine Zahl 1 oder 2 bedeuten, und der Kohlenwasserstoffrest gegebenenfalls durch Cyclopropyl, |

Cyclobutyl, Cyclopentyl, Cyclohexyl, Fluor, Chlor, Nitro, Cyano, Hydroxy oder durch Phenyl, Phenoxy, Phenylthio oder Pyridyl substituiert ist, die ihrerseits durch Fluor, Chlor, Methyl, Methoxy, Methylthio, Trifluormethyl oder Trifluormethoxy substituiert sein können, oder der Kohlenwasserstoffrest gegebenenfalls durch eine Gruppe der Formel $-CO_2R^{17}$, $-CONR^{18}R^{19}$, $-NR^{20}R^{21}$ oder $-NR^{22}-CO_2R^{23}$ substituiertist,

worin

$R^{20}$ und $R^{21}$ gleich oder verschieden sind und Wasserstoff, einen geradkettigen, verzweigten oder cyclischen, gesättigten oder ungesättigten Kohlenwasserstoffrest mit bis zu 6 Kohlenstoffatomen bedeuten, das gegebenenfalls durch Fluor, Chlor, Pyridyl oder Phenyl substituiert ist, das seinerseits durch Fluor, Chlor, Methyl oder Methoxy substituiert sein kann, oder

Phenyl bedeuten, das gegebenenfalls durch Fluor, Chlor, Methyl oder Methoxy substituiert ist,

oder

$R^{20}$ und $R^{21}$ gemeinsam unter Einbezug des Stickstoffatoms einen 5-bis 6-gliedrigen, gesättigen oder ungesättigten Heterocyclus bilden, der gegebenenfalls bis zu 2 weitere Heteroatome aus der Reihe S, N oder O enthalten kann und der gegebenenfalls durch geradkettiges oder verzweigtes Alkyl mit bis zu 4 Kohlenstoffatomen, Benzyl oder Phenyl substituiert ist,

$R^{17}$, $R^{18}$, $R^{19}$, $R^{22}$ und $R^{23}$ gleich oder verschieden sind und Wasserstoff, geradkettiges oder verzweigtes Alkyl mit bis zu 6 Kohlenstoffatomen bedeuten, das gegebenenfalls durch Phenyl substituiert ist, oder

Phenyl bedeuten, das gegebenenfalls durch Fluor, Chlor oder Brom substituiert ist,

$R^3$ für Cyano, Nitro, Formyl oder geradkettiges oder verzweigtes Alkoxycarbonyl mit bis zu 4 Kohlenstoffatomen steht, das gegebenenfalls durch geradkettiges oder verzweigtes Alkoxy mit bis zu 4 Kohlenstoffatomen substituiert ist, oder

für eine Gruppe der Formel - CO-NH-G steht,

worin

G Cyclopropyl oder Cyclopentyl bedeutet,

oder

$R^3$ und $R^4$ gemeinsam einen Rest der Formel

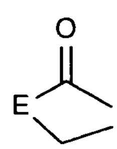

bilden,

worin

E ein Sauerstoff-oder Schwefelatom oder die $-(CH_2)_n$-Gruppe bedeutet,

worin

n eine Zahl 1 oder 2 bedeutet,

$R^5$ für einen Rest der Formel

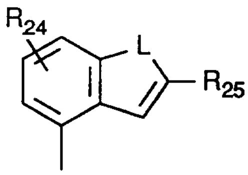 , 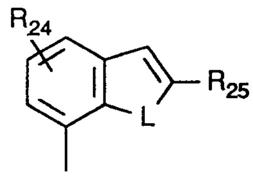 , 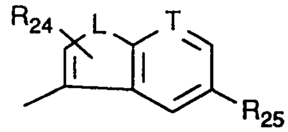 ,

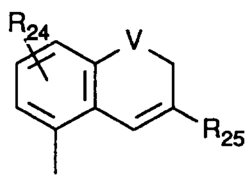 , 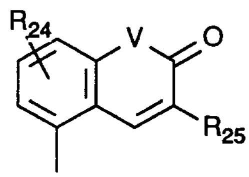 , 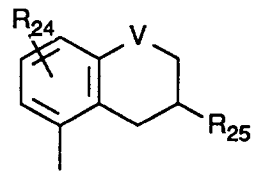 ,

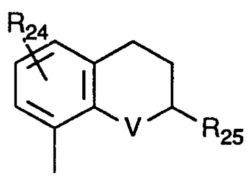 , 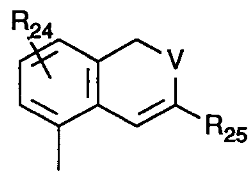 , 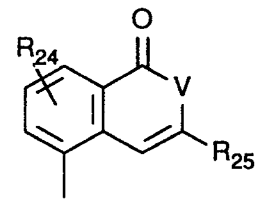 ,

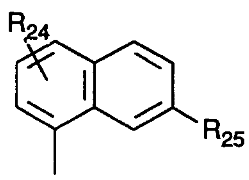 , 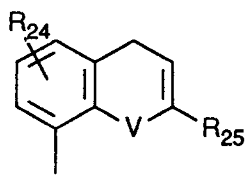 , 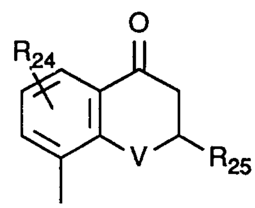 ,

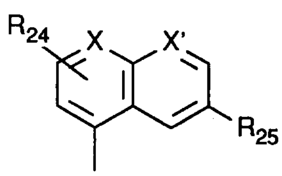 , 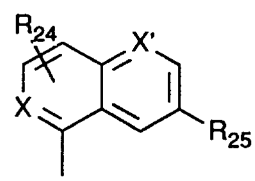 , 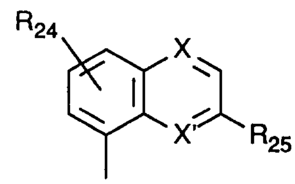 ,

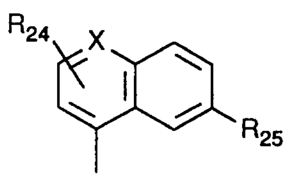 , 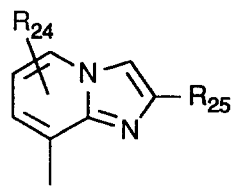 oder 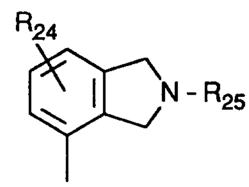

steht,

worin

$R^{24}$ Wasserstoff, Fluor oder Chlor bedeutet,

$R^{25}$ Phenyl bedeutet, das gegebenenfalls bis zu 2-fach gleich oder verschieden durch Fluor, Chlor, Nitro, Trifluormethyl, Methyl, Ethyl, Methoxy oder Ethoxy oder durch eine Gruppe der Formel $-NR^{26}R^{27}$ substituiert ist,

worin

$R^{26}$ und $R^{27}$ gleich oder verschieden sind und Wasserstoff oder geradkettiges oder verzweigtes Alkyl mit bis zu 4 Kohlenstoffatomen, Phenyl oder Benzyl bedeuten,

oder

$R^{25}$ einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel unterbrochen ist, und der gegebenenfalls durch Cyclopropyl, Cyclobutyl, Cyclopentyl, Cyclohexyl, Fluor, Chlor, Nitro, Cyano, Hydroxy oder durch Phenyl, Phenyloxy, Phenylthio oder durch einen 5-bis 7-gliedrigen, gesättigten oder ungesättigen Heterocyclus mit bis zu 2 Heteroatomen aus der Reihe S, N oder O substituiert ist, wobei die Phenyl- und die Hetero-Cyclen ihrerseits bis zu 2-fach gleich oder verschieden durch Fluor, Chlor, Methyl, Methoxy, Trifluormethyl oder Trifluormethoxy oder durch eine Gruppe der Formel $-NR^{28}R^{29}$ substituiert sein können,

worin

$R^{28}$ und $R^{29}$ die oben angegebene Bedeutung von $R^{11}$ und $R^{12}$ haben und mit dieser gleich oder verschieden sind,

oder der Kohlenwasserstoffrest gegebenenfalls durch eine Gruppe der Formel $-CO_2-R^{30}$, $-CONR^{31}R^{32}$, $-NR^{33}R^{34}$, $-NR^{35}-CO_2R^{36}$ oder $-NR^{37}-SO_2R^{38}$ substituiert ist,

worin

$R^{30}$ Alkyl mit 1-4 C-Atomen oder Phenyl bedeutet,

und

$R^{31}$, $R^{32}$, $R^{33}$, $R^{34}$, $R^{35}$, $R^{36}$, $R^{37}$ und $R^{38}$ die oben angegebene Bedeutung von $R^{11}$ und $R^{12}$ haben und mit diesen gleich oder verschieden sind,

oder

$R^{25}$ einen 5-bis 6-gliedrigen, gesättigten oder ungesättigten Heterocyclus mit bis zu 2 Heteroatomen aus der Reihe S, N oder O bedeutet, der gegebenenfalls durch Fluor, Chlor, Methyl, Methoxy, Methylthio oder Trifluormethyl substituiert ist,

oder

$R^{25}$ eine Gruppe der Formel $D-R^{39}$ bedeutet,

worin

D die $-S(O)_h$-Gruppe oder ein Sauerstoffatom bedeutet,

worin

h eine Zahl 0, 1 oder 2 bedeutet,

und

$R^{39}$ Phenyl bedeutet, das gegebenenfalls durch Fluor, Chlor, Brom, Methyl, Methoxy, Methylthio,Trifluormethyl, Amino oder durch $C_1$-$C_2$-Mono-oder - Dialkylamino substituiert ist,

oder

$R^{39}$ Wasserstoff, einen cyclischen, geradkettigen oder verzweigten, gesättigten oder ungesättigten Kohlenwasserstoffrest mit bis zu 6 Kohlenstoffatomen bedeutet, der gegebenenfalls durch Sauerstoff oder Schwefel unterbrochen ist,

und der gegebenenfalls durch Fluor, Chlor oder Phenyl substituiert ist, oder

durch eine Gruppe der Formel $-NR^{40}R^{41}$ substituiert ist

worin

$R^{40}$ und $R^{41}$ die oben angegebene Bedeutung von $R^{11}$ und $R^{12}$ haben und mit dieser gleich oder verschieden sind,

L ein Schwefel-oder Sauerstoffatom oder die - NH-Gruppe bedeutet,

T ein Stickstoffatom oder die N->O-Gruppe bedeutet,

| | |
|---|---|
| V | ein Schwefel-oder Sauerstoffatom bedeutet, |
| X und X' | gleich oder verschieden sind und ein Stickstoffatom oder die N->O-Gruppe bedeuten |

und deren Salze.

**4.** Verbindungen der allgemeinen Formel (I) gemaß Anspruch 1 zur Verwendung bei der Bekämpfung von Herz-Kreislauferkrankungen.

**5.** Verfahren zur Herstellung von Verbindungen der allgemeinen Formel (I)

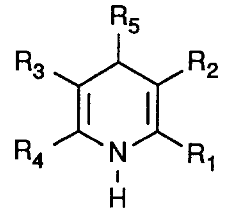

(I)

in welcher

| | |
|---|---|
| $R^1$ und $R^4$ | gleich oder verschieden sind und für Wasserstoff, Amino, Cyano, Formyl, Trifluormethyl oder für geradkettiges oder verzweigtes Alkyl mit bis zu 8 Kohlenstoffatomen stehen, das gegebenenfalls durch Hydroxy, geradkettiges oder verzweigtes Alkoxycarbonyl mit bis zu 6 Kohlenstoffatomen oder durch eine Gruppe der Formel - $NR^6R^7$, $-O-CO-R^8$, $-O-(CH_2)_a-OR^{8'}$ oder $-O-(CH_2)_b-NR^9R^{10}$ substituiert ist, worin |
| $R^6$, $R^7$, $R^9$ und $R^{10}$ | gleich oder verschieden sind und Wasserstoff, Phenyl oder geradkettiges oder verzweigtes Alkyl mit bis zu 6 Kohlenstoffatomen bedeuten, |
| $R^8$ und $R^{8'}$ | gleich oder verschieden sind und geradkettiges oder verzweigtes Alkyl mit bis zu 6 Kohlenstoffatomen bedeuten, und |
| a und b | gleich oder verschieden sind und eine Zahl 2, 3, 4 oder 5 bedeuten, |
| $R^2$ | für Cyano oder für eine Gruppe der Formel - $CO-NR^{11}R^{12}$ oder $-CO-A-R^{13}$ steht, worin |
| $R^{11}$ und $R^{12}$ | gleich oder verschieden sind und Wasserstoff, einen geradkettigen, verzweigten oder cyclischen, gesättigten oder ungesättigten Kohlenwasserstoffrest mit bis zu 8 Kohlenstoffatomen bedeuten, der gegebenenfalls durch Halogen, Hydroxy, Cyano oder durch Aryl, Aryloxy, Arylthio mit jeweils 6 bis 10 Kohlenstoffatomen oder durch einen 5-bis 7-gliedrigen, gesättigen oder ungesättigten Heterocyclus mit bis zu 3 Heteroatomen aus der Reihe S, N oder O substituiert ist, wobei die Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder<br>Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O bedeuten, die gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylt- |

hio mit jeweils bis zu 4 Kohlenstoffatomen substituiert sind, oder

$R^{11}$ und $R^{12}$ gemeinsam unter Einbezug des Stickstoffatoms einen 3-bis 8-gliedrigen, gesättigten oder ungesättigten Heterocyclus bilden, der gegebenenfalls durch ein Sauerstoffatom oder durch einen Rest der Formel $S(O)_d$, - CO- oder $-NR^{15}-$ unterbrochen sein kann, worin

d eine Zahl 0, 1 oder 2 bedeutet,

$R^{15}$ Wasserstoff oder Aryl mit 6 bis 10 Kohlenstoffatomen bedeutet, das gegebenenfalls bis zu 2-fach gleich oder verschieden durch Halogen, Cyano oder durch geradkettiges oder verzweigtes Alkyl, Alkoxy, Alkylthio, Alkoxycarbonyl mit jeweils bis zu 8 Kohlenstoffatomen, Halogenalkyl, Halogenalkoxy oder Halogenalkylthio mit jeweils bis zu 4 Kohlenstoffatomen substituiert ist, oder einen cyclischen, gesättigten oder ungesättigten, geradkettigen oder verzweigten Kohlenwasserstoffrest mit bis zu 8 Kohlenstoffatomen bedeutet, der gegebenenfalls durch Hydroxy, Halogen oder durch Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O substituiert ist, wobei die Cyclen ihrerseits bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,

und der Heterocyclus gegebenenfalls durch geradkettiges oder verzweigtes Alkoxy oder Alkylthio mit jeweils bis zu 4 Kohlenstoffatomen, Halogen, Aryl mit 6 bis 10 Kohlenstoffatomen, einen 5-bis 7-gliedrigen, gesättigten oder ungesättigten Heterocyclus mit bis zu 3 Heteroatomen aus der Reihe S, N oder O oder durch geradkettiges oder verzweigtes Alkyl mit bis zu 4 Kohlenstoffatomen substituiert ist, das seinerseits durch Aryl mit 6 bis 10 Kohlenstoffatomen substituiert sein kann,

A eine direkte Bindung oder ein Sauerstoffatom bedeutet,

$R^{13}$ Wasserstoff oder Aryl mit 6 bis 10 Kohlenstoffatomen oder einen 5- bis 7-gliedrigen, gesättigten oder ungesättigten Heterocyclus mit bis zu 3 Heteroatomen aus der Reihe S, N oder O bedeutet, wobei die Cyclen gegebenenfalls bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind, oder einen cyclischen, geradkettigen oder verzweigten, gesättigten oder ungesättigten Kohlenwasserstoffrest mit bis zu 10 Kohlenstoffatomen bedeutet, der gegebenenfalls bis zu 3-fach gleich oder verschieden durch Sauerstoff oder durch - CO-, -CO-NH-, -O-CO-, -CO-O-, -NH-CO-, $-SO_2-NH-$, $-NH-SO_2-$, $-S(O)_e$-oder $NR^{16}-$ unterbrochen ist, worin

e die oben angegebene Bedeutung von d hat und mit dieser gleich oder verschieden ist,

$R^{16}$ die oben angegebene Bedeutung von $R^{15}$ hat und mit dieser gleich oder verschieden ist,

oder der Kohlenwasserstoffrest gegebenenfalls bis zu 3-fach gleich oder verschieden durch Aryliden mit 6 bis 10 Kohlenstoffatomen oder heterocyclische Reste der Formeln

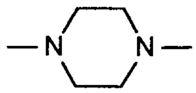 , 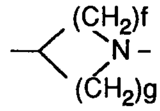 , 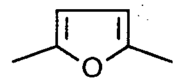 oder 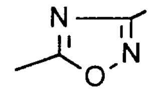

unterbrochen ist,
worin

f und g — gleich oder verschieden sind und eine Zahl 1 oder 2 bedeuten,

und wobei Aryliden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein kann,

und der Kohlenwasserstoffrest gegebenenfalls bis zu 3-fach gleich oder verschieden durch Cycloalkyl mit 3 bis 8 Kohlenstoffatomen, Halogen, Nitro, Cyano, Hydroxy, - $O\text{-}NO_2$, geradkettiges oder verzweigtes Alkylthio, Alkoxy oder Acyloxy mit jeweils bis zu 8 Kohlenstoffatomen oder durch Aryl, Aryloxy oder Arylthio mit jeweils 6 bis 10 Kohlenstoffatomen oder durch einen 5- bis 7-gliedrigen, gesättigten oder ungesättigten Heterocyclus mit bis zu 3 Heteroatomen aus der Reihe S, N oder O substituiert ist, wobei die Cyclen ihrerseits bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder

der Kohlenwasserstoffrest gegebenenfalls durch eine Gruppe der Formel $-CO_2\text{-}R^{17}$, $-CONR^{18}R^{19}$, - $NR^{20}R^{21}$ oder $-NR^{22}\text{-}CO_2R^{23}$ substituiert ist,
worin

$R^{17}$ — die oben angegebene Bedeutung von $R^{15}$ hat und mit dieser gleich oder verschieden ist
und

$R^{18}$, $R^{19}$, $R^{20}$, $R^{21}$, $R^{22}$ und $R^{23}$ — die oben angegebene Bedeutung von $R^{11}$ und $R^{12}$ haben und mit diesen gleich oder verschieden sind,

$R^3$ — für Cyano, Nitro, Formyl oder geradkettiges oder verzweigtes Alkoxycarbonyl mit bis zu 6 Kohlenstoffatomen steht, das gegebenenfalls durch geradkettiges oder verzweigtes Alkoxy mit bis zu 6 Kohlenstoffatomen substituiert ist, oder

für eine Gruppe der Formel - CO-NH-G steht,
worin

G — Cycloalkyl mit 3 bis 6 Kohlenstoffatomen bedeutet,

oder

$R^3$ und $R^4$ — gemeinsam einen Rest der Formel

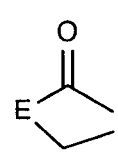

bilden,
worin

E — ein Sauerstoff- oder Schwefelatom oder die $-(CH_2)_n$-Gruppe bedeutet,
worin

n — eine Zahl 1 oder 2 bedeutet,

$R^5$ — für einen Rest der Formel

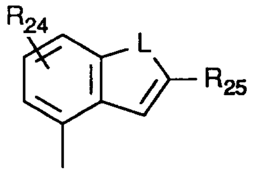 , 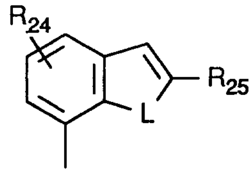 , 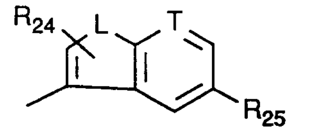 ,

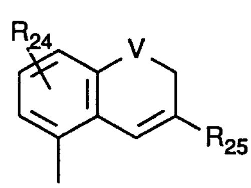 , 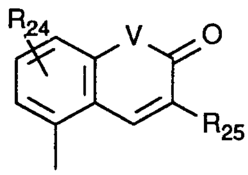 , 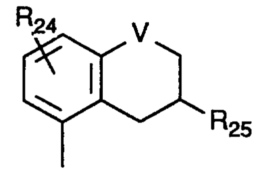 ,

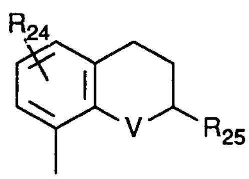 , 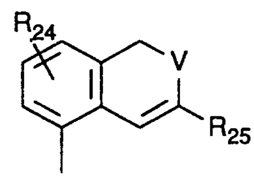 , 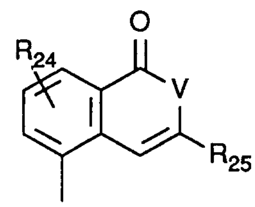 ,

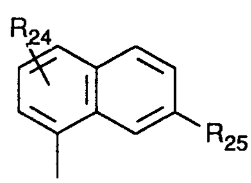 , 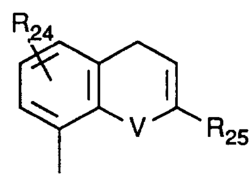 , 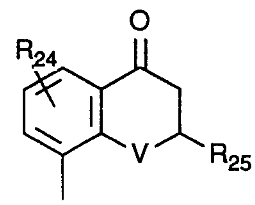 ,

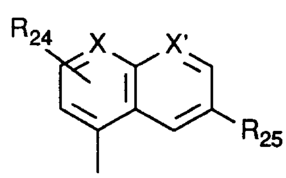 , 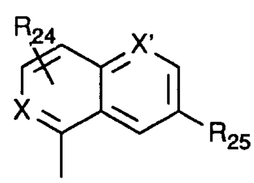 , 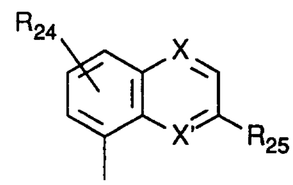 ,

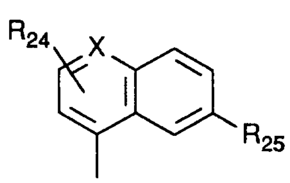 , 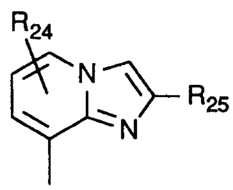 oder 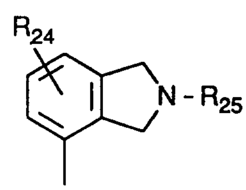

steht,
worin

$R^{24}$ Wasserstoff, Halogen oder geradkettiges oder verzweigtes Alkyl oder Alkoxy mit jeweils bis zu 8 Kohlenstoffatomen bedeutet,

$R^{25}$ Aryl mit 6 bis 10 Kohlenstoffatomen bedeutet, das gegebenenfalls bis zu 3-fach gleich oder verschieden durch Halogen, Nitro, Cyano, Trifluormethyl, Trifluormethoxy, Trifluormethylthio,
oder durch geradkettiges oder verzweigtes Alkyl mit bis zu 8 Kohlenstoffatomen substituiert ist, das seinerseits durch Aryl mit 6 bis 10 Kohlenstoffatomen substituiert sein kann,
oder durch geradkettiges oder verzweigtes Alkoxy oder Alkoxycarbonyl mit jeweils bis zu 8 Kohlenstoffatomen, Carboxy, Amino oder durch eine Gruppe der Formel

$-NR^{26}R^{27}$ substituiert ist,
worin

$R^{26}$ und $R^{27}$ gleich oder verschieden sind und Wasserstoff, geradkettiges oder verzweigtes Alkyl mit bis zu 8 Kohlenstoffatomen, Phenyl oder Benzyl bedeuten,
oder

$R^{25}$ einen cyclischen, geradkettigen oder verzweigten, gesättigten oder ungesättigten Kohlenwasserstoffrest mit bis zu 12 Kohlenstoffatomen bedeutet, der gegebenenfalls bis zu 2-fach gleich oder verschieden durch Sauerstoff oder Schwefel unterbrochen ist,
und der gegebenenfalls bis zu 3-fach gleich oder verschieden durch Cycloalkyl mit 3 bis 8 Kohlenstoffatomen, geradkettiges oder verzweigtes Acyloxy mit bis zu 4 Kohlenstoffatomen, Halogen, Nitro, Cyano, Hydroxy oder durch Aryl, Aryloxy oder Arylthio mit jeweils 6 bis 10 Kohlenstoffatomen oder durch einen 5- bis 7-gliedrigen, gesättigten oder ungesättigen gegebenenfalls kondensierten Heterocyclus mit bis zu 5 Heteroatomen aus der Reihe S, N oder O substituiert ist, wobei die Cyclen ihrerseits bis zu 3-fach gleich oder verschieden durch Halogen, Cyano, Nitro, Hydroxy oder durch geradkettiges oder verzweigtes Alkyl oder Alkoxy mit jeweils bis zu 4 Kohlenstoffatomen, Trifluormethyl, Trifluormethoxy, Trifluormethylthio oder durch eine Gruppe der Formel $-NR^{28}R^{29}$ substituiert sein können,
worin

$R^{28}$ und $R^{29}$ die oben angegebene Bedeutung von $R^{11}$ und $R^{12}$ haben und mit dieser gleich oder verschieden sind,

oder der Kohlenwasserstoffrest gegebenenfalls durch eine Gruppe der Formel $-CO_2-R^{30}$, $-CONR^{31}R^{32}$, $-NR^{33}R^{34}$, $-NR^{35}-CO_2R^{36}$ oder $-NR^{37}-SO_2R^{38}$ substituiert ist,
worin

$R^{30}$ die oben angegebene Bedeutung von $R^{15}$ hat und mit dieser gleich oder verschieden ist
und

$R^{31}$, $R^{32}$, $R^{33}$, $R^{34}$, $R^{35}$, $R^{36}$, $R^{37}$ und $R^{38}$ die oben angegebene Bedeutung von $R^{11}$ und $R^{12}$ haben und mit diesen gleich oder verschieden sind,
oder

$R^{25}$ einen 5-bis 7-gliedrigen, gesättigten oder ungesättigten Heterocyclus mit bis zu 4 Heteroatomen aus der Reihe S, N oder O bedeutet, der gegebenenfalls bis zu 3-fach gleich oder verschieden durch Halogen, Amino, Cyano, Nitro oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen oder durch $C_1$-$C_4$-Mono-oder - Dialkylamino substituiert ist,

oder

$R^{25}$ eine Gruppe der Formel $D-R^{39}$ bedeutet,
worin

D die CO- oder $-S(O)_h$-Gruppe oder ein Sauerstoffatom bedeutet,
worin

h eine Zahl 0, 1 oder 2 bedeutet,
und

$R^{39}$ Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O bedeutet der gegebenenfalls bis zu 3-fach gleich oder verschieden durch Halogen, Amino, Cyano, Nitro oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen oder durch $C_1$-$C_4$-Mono- oder -Dialkylamino substituiert ist,
oder

$R^{39}$ Wasserstoff oder einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel unterbrochen ist, und der

gegebenenfalls durch Halogen, Aryl, Aryloxy oder Arylthio mit jeweils 6 bis 10 Kohlenstoffatomen oder durch einen 5- bis 7-gliedrigen, gesättigten oder ungesättigen Heterocyclus mit bis zu 3 Heteroatomen aus der Reihe S, N oder O substituiert ist, wobei die Cyclen ihrerseits durch Halogen, Trifluormethyl, Methyl, Methoxy, Nitro oder Methylthio substituiert sein können, oder
durch eine Gruppe der Formel $-NR^{40}R^{41}$ substituiert ist
worin

$R^{40}$ und $R^{41}$ die oben angegebene Bedeutung von $R^{11}$ und $R^{12}$ haben und mit dieser gleich oder verschieden sind,

L ein Schwefel- oder Sauerstoffatom oder die - NH-Gruppe bedeutet,

T ein Stickstoffatom oder die N->O-Gruppe bedeutet,

V ein Schwefel- oder Sauerstoffatom bedeutet,

X und X' gleich oder verschieden sind und ein Stickstoffatom oder die N->O-Gruppe bedeuten,

und deren Salze, dadurch gekennzeichnet, daß man
im Fall daß $R^3$ für Cyano, Nitro oder Formyl steht,

[A] Verbindungen der allgemeinen Formel (II)

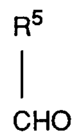

(II)

in welcher
$R^5$ die oben angegebene Bedeutung hat,
zunächst mit Acylverbindungen der allgemeinen Formel (III)

$R^3$-CO-$CH_2$-$R^4$ (III)

in welcher
$R^3$ und $R^4$ die oben angegebene Bedeutung haben
gegebenenfalls unter Isolierung der Ylidenverbindungen der allgemeinen Formel (IV)

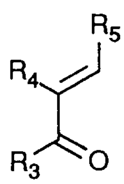

(IV)

in welcher
$R^3$, $R^4$ und $R^5$ die oben angegebene Bedeutung haben
umsetzt, anschließend mit Verbindungen der Formel (V)

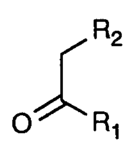

(V)

in welcher
$R^1$ und $R^2$ die oben angegebene Bedeutung haben,
und einer reaktiven Ammoniumverbindung, z.B. Ammoniumacetat, oder direkt mit Enamino-Verbindungen der allgemeinen Formel (VI)

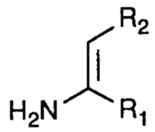

(VI)

in welcher

$R^1$ und $R^2$ die oben angegebene Bedeutung haben,

in inerten Lösemitteln umsetzt,

oder

[B] Verbindungen der allgemeinen Formel (II) zunächst mit Verbindungen der allgemeinen Formel (V), gegebenenfalls unter Isolierung der Ylidenverbindungen der allgemeinen Formel (VII)

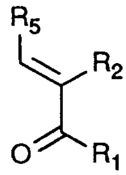

(VII)

in welcher

$R^1$, $R^2$ und $R^5$ die oben angegebene Bedeutung haben,

umsetzt und anschließend entweder mit Verbindungen der allgemeinen Formel (III) in Gegenwart von Ammoniumverbindungen oder direkt mit Verbindungen der allgemeinen Formel (VIII)

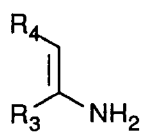

(VIII)

in welcher

$R^3$ und $R^4$ die oben angegebenene Bedeutung haben,

umsetzt

oder

[C] im Fall, daß $R^3$ und $R^4$ zusammen einen Rest der Formel

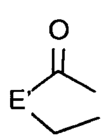

bilden,

worin

E' für ein Sauerstoff- oder Schwefelatom steht,

zunächst nach dem unter [A] und [B] aufgeführten Methoden Verbindungen der allgemeinen Formel (IX)

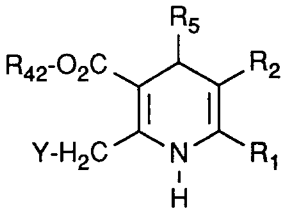

(IX)

in welcher

$R^1$, $R^2$ und $R^5$ die oben angegebene Bedeutung haben,

$R^{42}$ für $C_1$-$C_4$-Alkyl steht

und

Y für $C_1$-$C_4$-Acyloxy oder Acylthio steht,

herstellt und anschließend einen basischen oder sauren Ringschluß nach bekannten Methoden durchführt,

oder

[D] daß man im Fall, daß E für die $-(CH_2)_n$-Gruppe steht,

Verbindungen der allgemeinen Formel (II), zunächst mit Acylverbindungen der allgemeinen Formel (X)

$Z-CO-CH_2-R^2$ (X)

in welcher

$R^2$ die oben angegebene Bedeutung hat

und

Z die oben angegebene Bedeutung von $R^1$ hat, wobei im Fall der Hydroxy- und/oder Aminofunktionen, diese gegebenenfalls in geschützter Form vorliegen,

gegebenenfalls unter Isolierung der Ylidenverbindungen der allgemeinen Formel (XI)

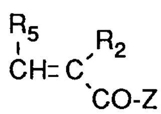

(XI)

in welcher

$R^2$, $R^5$ und Z die oben angegebene Bedeutung haben

umsetzt, anschließend mit der Verbindung der Formel (XII)

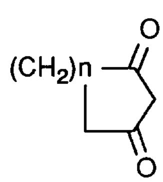

(XII)

in welcher

n eine Zahl 1 oder 2 bedeutet,

und einer reaktiven Ammoniumverbindung, z.B. Ammoniumacetat, gegebenenfalls unter Isolierung der Zwischenprodukte der allgemeinen Formel (XIII)

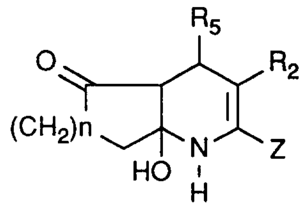

(XIII)

in welcher

$R^2$, $R^5$, n und Z die oben angegebene Bedeutung haben,

in inerten Lösemitteln umsetzt,

und anschließend in einem letzten Schritt, gegebenenfalls in Anwesenheit eines Hilfsmittels, Wasser abscheidet,

oder

[E] Verbindungen der allgemeinen Formel (II) mit Verbindungen der allgemeinen Formel (VIIIa) und (XIV)

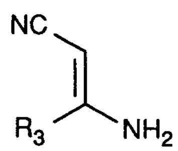

(VIIIa) und

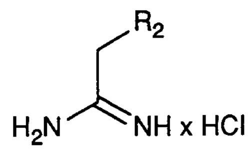

(XIV)

in welcher

$R^2$ und $R^3$ die oben angegebene Bedeutung haben,

in inerten Lösemittel umsetzt.

**6.** Arzneimittel enthaltend mindestens eine Verbindung der allgemeinen Formel (I) gemäß Anspruch 1.

**7.** Verbindungen der allgemeinen Formel (II)

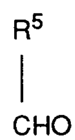

(II)

in welcher

$R^5$ für einen Rest der Formel

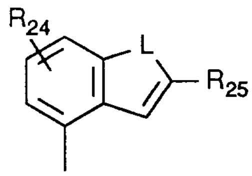,
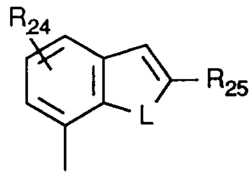,
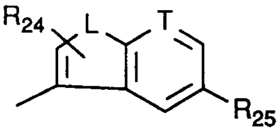,

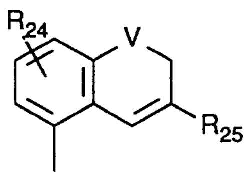,
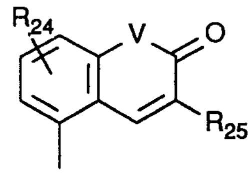,
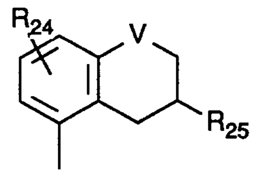,

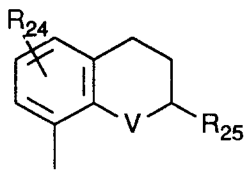,
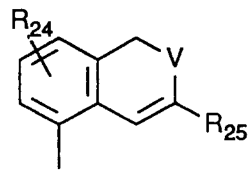,
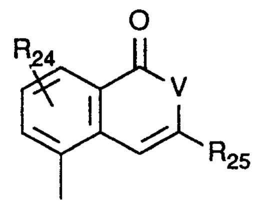,

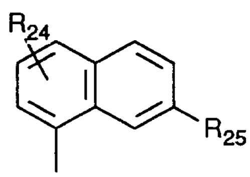,
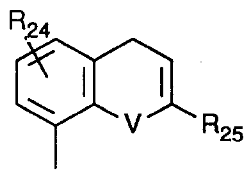,
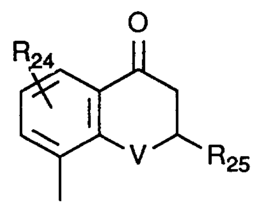,

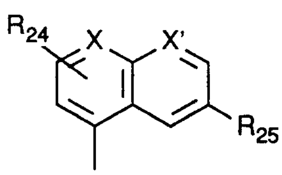,
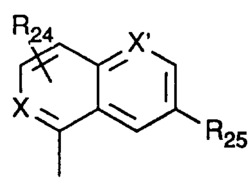,
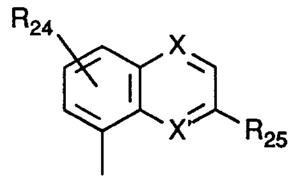,

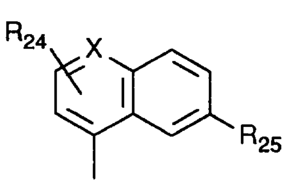,
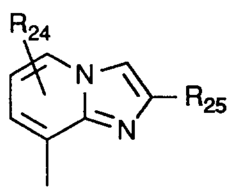
oder
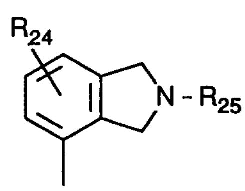

steht, aber nicht für den Rest der Formel

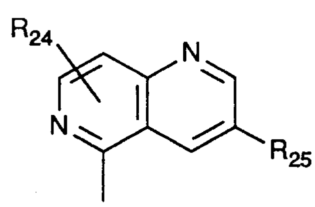

steht,

worin

$R^{24}$ Wasserstoff, Halogen oder geradkettiges oder verzweigtes Alkyl oder Alkoxy mit jeweils bis zu 8 Kohlenstoffatomen bedeutet,

$R^{25}$ Aryl mit 6 bis 10 Kohlenstoffatomen bedeutet, das gegebenenfalls bis zu 3-fach gleich oder verschieden durch Halogen, Nitro, Cyano, Trifluormethyl, Trifluormethoxy, Trifluormethylthio,
oder durch geradkettiges oder verzweigtes Alkyl mit bis zu 8 Kohlenstoffatomen substituiert ist, das seinerseits durch Aryl mit 6 bis 10 Kohlenstoffatomen substituiert sein kann,
oder durch geradkettiges oder verzweigtes Alkoxy oder Alkoxycarbonyl mit jeweils bis zu 8 Kohlenstoffatomen, Carboxy, Amino oder durch eine Gruppe der Formel $-NR^{26}R^{27}$ substituiert ist,
worin

$R^{26}$ und $R^{27}$ gleich oder verschieden sind und Wasserstoff, geradkettiges oder verzweigtes Alkyl mit bis zu 8 Kohlenstoffatomen, Phenyl oder Benzyl bedeuten,

oder

$R^{25}$ einen cyclischen, geradkettigen oder verzweigten, gesättigten oder ungesättigten Kohlenwasserstoffrest mit bis zu 12 Kohlenstoffatomen bedeutet, der gegebenenfalls bis zu 2-fach gleich oder verschieden durch Sauerstoff oder Schwefel unterbrochen ist,
und der gegebenenfalls bis zu 3-fach gleich oder verschieden durch Cycloalkyl mit 3 bis 8 Kohlenstoffatomen, geradkettiges oder verzweigtes Acyloxy mit bis zu 4 Kohlenstoffatomen, Halogen, Nitro, Cyano, Hydroxy oder durch Aryl, Aryloxy oder Arylthio mit jeweils 6 bis 10 Kohlenstoffatomen oder durch einen 5-bis 7-gliedrigen, gesättigten oder ungesättigen gegebenenfalls kondensierten Heterocyclus mit bis zu 5 Heteroatomen aus der Reihe S, N oder O substituiert ist, wobei die Cyclen ihrerseits bis zu 3-fach gleich oder verschieden durch Halogen, Cyano, Nitro, Hydroxy oder durch geradkettiges oder verzweigtes Alkyl oder Alkoxy mit jeweils bis zu 4 Kohlenstoffatomen, Trifluormethyl, Trifluormethoxy, Trifluormethylthio oder durch eine Gruppe der Formel $-NR^{28}R^{29}$ substituiert sein können,
worin

$R^{28}$ und $R^{29}$ die oben angegebene Bedeutung von $R^{11}$ und $R^{12}$ haben und mit dieser gleich oder verschieden sind,

oder der Kohlenwasserstoffrest gegebenenfalls durch eine Gruppe der Formel $-CO_2-R^{30}$, $-CONR^{31}R^{32}$, $-NR^{33}R^{34}$, $-NR^{35}-CO_2R^{36}$ oder $-NR^{37}-SO_2R^{38}$ substituiert ist,
worin

$R^{30}$ die oben angegebene Bedeutung von $R^{15}$ hat und mit dieser gleich oder verschieden ist

und

$R^{31}$, $R^{32}$, $R^{33}$, $R^{34}$, $R^{35}$, $R^{36}$, $R^{37}$ und $R^{38}$ die oben angegebene Bedeutung von $R^{11}$ und $R^{12}$ haben und mit diesen gleich oder verschieden sind,

oder

$R^{25}$ einen 5-bis 7-gliedrigen, gesättigten oder ungesättigten Heterocyclus mit bis zu 4 Heteroatomen aus der Reihe S, N oder O bedeutet, der gegebenenfalls bis zu 3-fach

gleich oder verschieden durch Halogen, Amino, Cyano, Nitro oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen oder durch $C_1$-$C_4$-Mono-oder - Dialkylamino substituiert ist,
oder

| | |
|---|---|
| $R^{25}$ | eine Gruppe der Formel D-$R^{39}$ bedeutet, worin |
| D | die CO-oder - $S(O)_h$-Gruppe oder ein Sauerstoffatom bedeutet, worin |
| h | eine Zahl 0, 1 oder 2 bedeutet, und |
| $R^{39}$ | Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O bedeutet, der gegebenenfalls bis zu 3-fach gleich oder verschieden durch Halogen, Amino, Cyano, Nitro oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen oder durch $C_1$-$C_4$-Mono-oder - Dialkylamino substituiert ist, oder |
| $R^{39}$ | Wasserstoff oder einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel unterbrochen ist, und der gegebenenfalls durch Halogen, Aryl, Aryloxy oder Arylthio mit jeweils 6 bis 10 Kohlenstoffatomen oder durch einen 5-bis 7-gliedrigen, gesättigten oder ungesättigen Heterocyclus mit bis zu 3 Heteroatomen aus der Reihe S, N oder O substituiert ist, wobei die Cyclen ihrerseits durch Halogen, Trifluormethyl, Methyl, Methoxy, Nitro oder Methylthio substituiert sein können, oder durch eine Gruppe der Formel - $NR^{40}R^{41}$ substituiert ist worin |
| $R^{40}$ und $R^{41}$ | die oben angegebene Bedeutung von $R^{11}$ und $R^{12}$ haben und mit dieser gleich oder verschieden sind, |
| L | ein Schwefel-oder Sauerstoffatom oder die - NH-Gruppe bedeutet, |
| T | ein Stickstoffatom oder die N->O-Gruppe bedeutet, |
| V | ein Schwefel-oder Sauerstoffatom bedeutet, |
| X und X' | gleich oder verschieden sind und ein Stickstoffatom oder die N->O-Gruppe bedeuten. |

**8.** Verfahren zur Herstellung von Verbindungen der allgemeinen Formel (II) gemaß Anspruch 7, dadurch gekennzeichnet, daß man
[I] im Fall, daß $R^5$ = $R^{5'}$ für einen der im folgenden aufgeführten Reste

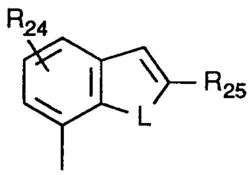 , 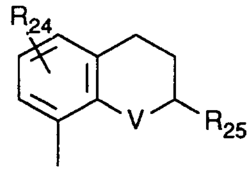 , 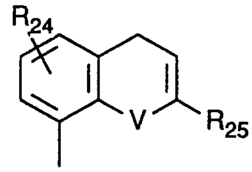 ,

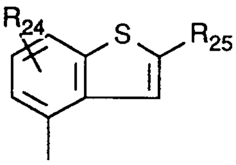 , 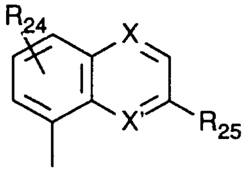 , 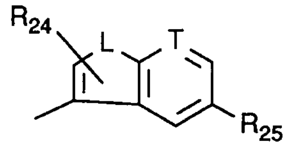 ,

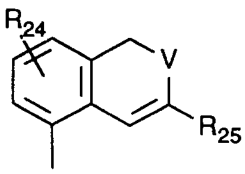 oder 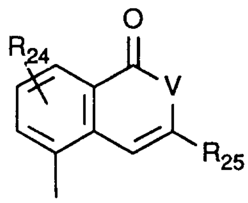

steht,
in welcher
$R^{24}$, $R^{25}$, L, T, V und X die oben angegebene Bedeutung haben,
Verbindungen der allgemeinen Formel (XV)

$R^{5'}$-$CO_2$-$R^{43}$ (XV)

in welcher
$R^{5'}$ für einen der oben aufgeführten Reste steht
und
$R^{43}$ für Wasserstoff oder $C_1$-$C_4$-Alkyl steht,
zunächst mit üblichen Reduktionsmitteln, wie beispielsweise Lithiumaluminiumhydrid oder über ein gemischtes Anhydrid mit Natriumborhydrid in die entsprechenden Alkohole der allgemeinen Formel (XVI)

$R^{5'}$-$CH_2$-OH (XVI)

in welcher
$R^{5'}$ die oben angegebene Bedeutung hat,
überführt,
und diese dann entweder nach Isolierung oder direkt in situ mit Oxidationsmitteln wie beispielsweise Manganoxid oxidiert,
oder
[II] im Fall, daß $R^5$ für den Rest der Formel

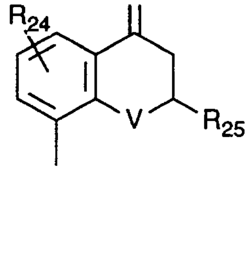

steht,
Verbindungen der allgemeinen Formel (XVII)

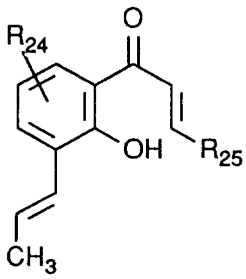

(XVII)

in welcher

$R^{24}$ und $R^{25}$ die oben angegebene Bedeutung haben,

zunächst durch Umsetzung mit Polyphosphorsäuren in Methylglycol zu den Verbindungen der allgemeinen Formel (XVIII)

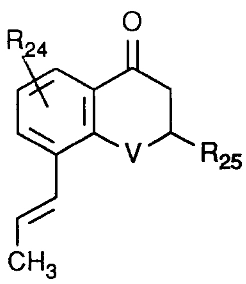

(XVIII)

in welcher

$R^{24}$ und $R^{25}$ die oben angegebene Bedeutung haben,

cyclisiert,

und anschließend in einem der oben aufgeführten Lösemitteln, vorzugsweise Methylenchlorid, eine Ozonolyse durchführt,

oder

[III] im Fall, daß $R^5$ für den Rest der Formel

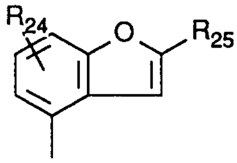

(a) oder

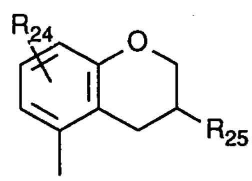

(b)

steht.

Verbindungen der allgemeinen Formel (XIX)

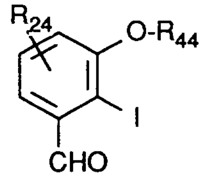

(XIX)

in welcher

$R^{24}$ die oben angegebene Bedeutung hat

und

$R^{44}$ für Wasserstoff oder für eine übliche, leicht abspaltbare Hydroxyschutzgruppe steht,
im Fall a) mit Verbindungen der allgemeinen Formel (XX)

$R^{25}$-≡-Cu (XX)

in welcher
$R^{25}$ die oben angegebene Bedeutung hat,
oder
im Fall b) zunächst mit Verbindungen der allgemeinen Formel (XXI)

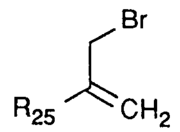
(XXI)

in welcher
$R^{25}$ die oben angegebene Bedeutung hat,
umsetzt und anschließend radikalisch z.B. mit Tributylzinnhydrid/AIBN cyclisiert und mit $MnO_2$ oxidiert,
oder
[IV] im Fall, daß $R^5$ für den Rest der Formel

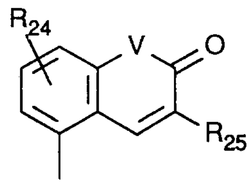

steht,
die entsprechenden Alkoholate der Verbindungen der allgemeinen Formel (XIX) zunächst im System $Pd(P(C_6H_5)_3)_2Cl_2$ mit Verbindungen der allgemeinen Formel (XXII)

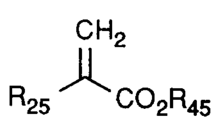
(XXII)

in welcher
$R^{25}$ die oben angegebene Bedeutung hat
und
$R^{45}$ für $C_1$-$C_4$-Alkyl steht,
zu den Verbindungen der allgemeinen Formel (XXIII)

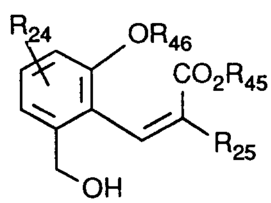
(XXIII)

in welcher

$R^{24}$ und $R^{45}$ die oben angegebene Bedeutung haben
und
$R^{46}$ für $C_1$-$C_4$-Alkyl steht,
umsetzt, anschließend wie oben beschrieben mit Oxidationsmitteln wie beispielsweise $MnO_2$ zu den entsprechenden Aldehyden oxidiert, und in einem letzten Schritt mit Säuren wie Bortribromid cyclisiert,
oder
[V] im Fall, daß $R^5$ für den Rest der Formel

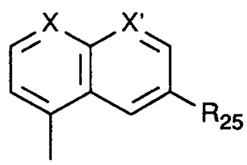

steht,
Verbindungen der allgemeinen Formel (XXIV)

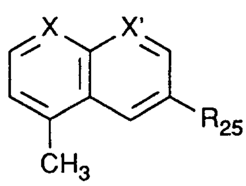

(XXIV)

in welcher
X, X' und $R^{25}$ die oben angegebene Bedeutung haben
in einem der oben aufgeführten Lösemittel, vorzugsweise Dioxan, mit Selendioxid oxidiert,
oder
[VI] im Fall, daß $R^5$ für den Rest der Formel

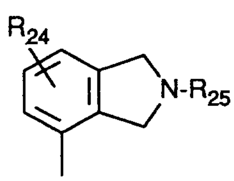

steht,
Verbindungen der allgemeinen Formel (XXV)

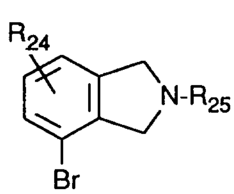

(XXV)

in welcher
$R^{24}$ und $R^{25}$ die oben angegebene Bedeutung haben,
unter Schutzgasatmosphäre, in einem der oben aufgeführten Lösemittel, vorzugsweise Ether, mit Butyllithium metalliert und dann mit DMF umsetzt,
oder

[VII] im Fall, daß $R^5$ für den Rest der Formel

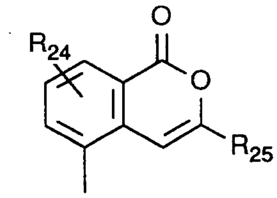

steht,
Verbindungen der allgemeinen Formel (XXVI)

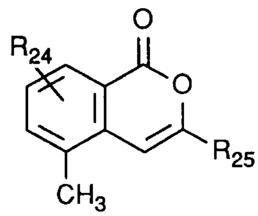

(XXVI)

in welcher

$R^{24}$ und $R^{25}$ die oben angegebene Bedeutung haben,

zunächst mit NBS bromiert, und in einem zweiten Schritt mit Kaliumacetat/Essigsäure, gefolgt von Schwefelsäure hydrolysiert,
und im Fall der N-oxide, ausgehend von den entsprechenden Verbindungen in welcher $R^5$ für einen stickstoffhaltigen Ring steht, zunächst mit MCPBA oxidiert und gegebenenfalls, wie oben beschrieben, in die Aldehyde überführt,
und gegebenenfalls auch die Verbindungen der allgemeinen Formel (I) durch die oben beschriebenen Arten der Oxidation oder Reduktion variiert.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung
EP 94 10 9019

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.5) |
|---|---|---|---|
| P,A | EP-A-0 555 657 (BAYER) 18. August 1993<br>* das ganze Dokument *<br>--- | 1-8 | C07D409/04<br>C07D417/04<br>C07D491/04 |
| A | EP-A-0 538 690 (BAYER) 28. April 1993<br>* das ganze Dokument *<br>--- | 1-8 | C07D401/04<br>C07D405/04<br>A61K31/445 |
| D,A | EP-A-0 452 712 (BAYER) 23. Oktober 1991<br>* das ganze Dokument *<br>& US-A-5 100 900<br>--- | 1-8 | A61K31/47<br>C07D495/04<br>C07D521/00<br>C07D211/90 |
| A | EP-A-0 450 420 (BAYER) 9. Oktober 1991<br>* das ganze Dokument *<br>--- | 1-8 | C07D471/04<br>C07D215/14<br>C07D209/10 |
| A | DE-A-37 14 438 (BAYER) 10. November 1988<br>* das ganze Dokument *<br>--- | 1-8 | C07D209/44<br>C07D307/79<br>C07D333/54 |
| A | EP-A-0 223 744 (BAYER) 27. Mai 1987<br>* das ganze Dokument *<br>--- | 1-8 | C07D311/58<br>C07D311/18 |
| X | DE-A-36 01 397 (BAYER) 27. Juli 1987<br>* Anspruch 1 *<br>--- | 1-8 | **RECHERCHIERTE SACHGEBIETE (Int.Cl.5)** |
| A | EP-A-0 123 112 (BAYER) 31. Oktober 1984<br>* das ganze Dokument *<br>---<br>-/-- | 1-8 | C07D |

## UNVOLLSTÄNDIGE RECHERCHE

Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.
Vollständig recherchierte Patentansprüche:
Unvollständig recherchierte Patentansprüche:
Nicht recherchierte Patentansprüche:
Grund für die Beschränkung der Recherche:

Siehe Ergänzungsblatt C

| Recherchenort | Abschlußdatum der Recherche | Prüfer |
|---|---|---|
| DEN HAAG | 11. Oktober 1994 | Kissler, B |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPO FORM 1503 03.82 (P04C09)

Europäisches Patentamt

# EUROPÄISCHER TEILRECHERCHENBERICHT

Nummer der Anmeldung
EP 94 10 9019

| EINSCHLÄGIGE DOKUMENTE | | | KLASSIFIKATION DER ANMELDUNG (Int.Cl.5) |
|---|---|---|---|
| Kategorie | Kennzeichnung des Dokuments mit Angabe, soweit erforderlich der maßgeblichen Teile | Betrifft Anspruch | |
| A | CHEMICAL ABSTRACTS, vol. 107, no. 13, 28. September 1987, Columbus, Ohio, US; abstract no. 115496, 'Preparation of Dihydropyridine derivatives as vasodilators.' * Zusammenfassung * & JP-A-62 045 586 (ELI LILLY) 27. Februar 1987 --- | 1-8 | C07D335/06<br>C07D311/32<br>C07D311/76<br>C07D241/42 |
| A | CHEMICAL ABSTRACTS, vol. 113, no. 5, 30. Juli 1990, Columbus, Ohio, US; abstract no. 40468, 'Preparation of 4-(7-benzofuryl)-2,6-dimethyl-1,4-dihydropyridines with one or more chiral substituents useful as antiarrytmics and calcium antagonists.' * Zusammenfassung * & ES-A-2 005 543 (LAB. MENARINI) 16. März 1989 --- | 1-8 | RECHERCHIERTE SACHGEBIETE (Int.Cl.5) |
| X | JOURNAL OF MEDICINAL CHEMISTRY., Bd.35, Nr.25, 1992, WASHINGTON US Seiten 4665 - 4675 Tanaka, Masayuki; Chiba, Kenichi; Okita, Makoto; Kaneko, Toshihiko; Tagami, Katsuya; Hibi, Shigeki; Okamoto, Yasushi; Shirota, Hir 'A novel orally active inhibitor of IL-1 generation: synthesis and structure-activity relationships of 3-(4-hydroxy-1-naphthalenyl)-2- propenoic acid derivatives' siehe RN 144272-89-9, 1-Naphthalenecarboxaldehyde, 7-ethyl-2-methoxy- --- | 7 | |

-/--

Europäisches Patentamt

# EUROPÄISCHER TEILRECHERCHENBERICHT

Nummer der Anmeldung
EP 94 10 9019

| EINSCHLÄGIGE DOKUMENTE | | | KLASSIFIKATION DER ANMELDUNG (Int.Cl.5) |
|---|---|---|---|
| Kategorie | Kennzeichnung des Dokuments mit Angabe, soweit erforderlich der maßgeblichen Teile | Betrifft Anspruch | |
| X | CHEMICAL ABSTRACTS, vol. 106, no. 3, 19. Januar 1987, Columbus, Ohio, US; abstract no. 18852, OXYGENATION OF CADALENE AND EUDALENE IN POLAR APROTIC SOLVENTS AU TAKEKUMA, SHINICHI; MATSUBARA, YOSHIHARU; YAMAMOTO, HIROSHI; NOZ 'Oxygenation of cadalene and eudalene in polar aprotic solvents' * Zusammenfassung * siehe RN 105986-11-6, 1-Naphthalenecarboxaldehyde, 7-(1-methylethyl)- & YUKAGAKU (1985), 34(12), 1026-8 --- | 7 | |
| X | CHEMICAL ABSTRACTS, vol. 116, no. 14, 6. April 1992, Columbus, Ohio, US; abstract no. 130100, OHLEMACHER, ANGELA; SCHENK, RAINER; WEITZEL, HANS PETER; TYUTYULKOV, N.; TASSEVA, M.; MUELLEN, KLAUS 'Energy spectra of poly(arylenevinylenes) containing different aromatic units' * Zusammenfassung * siehe RN 139594-64-2, 1-Naphthalenecarboxaldehyde, 3,7-bis(1,1-dimethylethyl)- & MAKROMOL. CHEM. (1992), 193(1), 81-93 --- | 7 | RECHERCHIERTE SACHGEBIETE (Int.Cl.5) |
| X | WO-A-91 17149 (SMITHKLINE BEECHAM) 14. November 1991 siehe RN 139160-01-3, 1-Naphthalenecarboxaldehyde, 7-(5-phenylpentyl)- ----- | 7 | |

EPO FORM 1503 03.82 (P04C12)

Mangelnde Genauigkeit

Die Definition des/der folgenden Substituenten ist zu allgemein und/oder umfasst einen zu grossen Bereich von chemisch grundverschiedenen Resten und ist nur teilweise durch Beispiele in der Beschreibung gestützt: R5

Eine vollständige Recherche der in den Ansprüchen 1-8 beanspruchten Verbindungen und Zwischenprodukte sprengt jedoch, wenn auch technisch/logistisch durchführbar, den Rahmen einer auch dem Gebote der Wirtschaftlichkeit zu folgenden Recherche.

(Vgl. Art. 83,84 EPC, Regel 45 EPC und Richtlinien zur Durchführung Teil B, Kapitel III, 3.6, 3.7).

Anspruch 7 wurde vollständig recherchiert für
R5 = Naphthyl-1, 4-Chinolinyl.

------------------------